(19) Europäisches Patentamt
European Patent Office
Office européen des brevets

(11) EP 4 183 418 A1

(12) **EUROPEAN PATENT APPLICATION**
published in accordance with Art. 153(4) EPC

(43) Date of publication:
**24.05.2023 Bulletin 2023/21**

(21) Application number: **21841752.5**

(22) Date of filing: **16.07.2021**

(51) International Patent Classification (IPC):
***A61K 47/68*** *(2017.01)* ***A61K 47/54*** *(2017.01)*
***A61P 35/00*** *(2006.01)* ***C07F 7/18*** *(2006.01)*

(52) Cooperative Patent Classification (CPC):
**A61K 47/54; A61K 47/68; A61P 35/00; C07F 7/18**

(86) International application number:
**PCT/KR2021/009204**

(87) International publication number:
**WO 2022/015110 (20.01.2022 Gazette 2022/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.07.2020 KR 20200088005**
**11.05.2021 KR 20210060721**

(71) Applicant: **Pinotbio, Inc.**
**Gyeonggi-do 16506 (KR)**

(72) Inventors:
- **JUNG, Doo Young**
**Suwon-si, Gyeonggi-do 16506 (KR)**
- **LEE, Jin Soo**
**Suwon-si, Gyeonggi-do 16506 (KR)**
- **CHO, Hyun Yong**
**Suwon-si, Gyeonggi-do 16506 (KR)**
- **CHOI, Shin Hei**
**Suwon-si, Gyeonggi-do 16506 (KR)**
- **LEE, Byeong Sung**
**Suwon-si, Gyeonggi-do 16506 (KR)**

(74) Representative: **Mewburn Ellis LLP**
**Aurora Building**
**Counterslip**
**Bristol BS1 6BX (GB)**

# (54) CONJUGATE IN WHICH FL118 DRUG IS LINKED TO ACID-SENSITIVE LINKER, AND IMMUNOCONJUGATE USING SAME

(57) The present invention relates to a conjugate in which FL118 is linked to an acid-sensitive linker, and an immunoconjugate using the same.

The present invention is characterized in that at least one FL118 drug of Formula 1 is linked to an antibody or antigen-binding site-containing fragment thereof through an acid-sensitive linker; wherein after being targeted to cancer cells by an antigen-binding site that targets an antigen of cancer cells, the acid-sensitive linker is degraded in acidic atmosphere around cancer (pH ≤ 7) to free at least a part of the FL118 drug of Formula 1 and the free FL118 drug of Formula 1 penetrates a cell membrane and moves into the cells; and wherein FL118 drug of Formula 1 inhibits the action an efflux pump to enrich the intracellular free FL118 drug of Formula 1.

[FIG. 2]

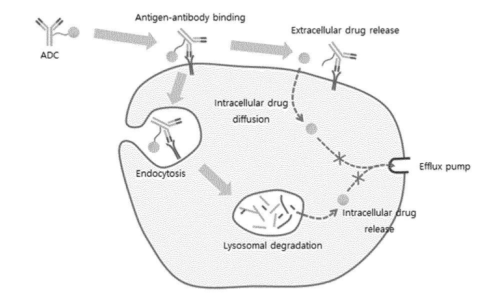


EP 4 183 418 A1

## Description

### TECHNICAL FIELD

The present invention relates to a conjugate in which an FL118 drug is linked to an acid-sensitive linker, and an immunoconjugate using the same.

### BACKGROUND ART

The antibody-drug conjugate (ADC) is a new drug platform that selectively delivers a payload with strong anticancer efficacy only to cancer tissues by taking advantage of the high tissue selectivity of an antibody. ADC selectively delivers a strong payload that kills cancer cells even at a low concentration of a pM level to cancer tissues, and minimizes systemic drug exposure, thereby securing both anticancer efficacy and safety at the same time.

The conventional ADC development has largely relied on the use of ultra-toxic payloads with an $IC_{50}$ value of pM level at the cellular level, but since the types of drugs with these properties are limited to microtubulin inhibitors, DNA damaging agents, and Top-1 inhibitors, there are several emerging problems. In many cases, a sufficient therapeutic window was not secured even when using these drugs in ADC. The side effects experienced by these patients are the same as the side effects of the payload itself, and an excellent ADC is difficult to develop with a payload of which safety is not secured. In addition, when these drugs are used, cancer resistance occurs through various mechanisms, and when such resistance occurs, there is currently no ADC that uses a new payload for overcoming the resistance. In short, there is a very high demand for a new ADC development platform that has an action mechanism that is differentiated from existing anticancer drugs/payloads to provide sufficient efficacy and potentials for overcoming resistance, and has sufficient safety even when used alone to secure a wide therapeutic window.

ADC development started in the 1970s, and many efforts are underway, such as discovery of new antigens/antibodies, development of payloads for ADC, development of linkers for antibody-payload conjugation, improvement of antibodies for uniform product quality, and pioneering of CMC routes. As a result, most of the ADCs are currently being developed by using the second or third generation ADC platform.

Many efforts have been made to develop ADCs that can be effectively used for the treatment of various cancers, especially solid cancers, centering on companies such as Genetech and Seattle Genetics in the US and LegoChem Bioscience in Korea, but still there are very important unmet requirements, such as insufficient therapeutic window and/or the generation of resistance.

In the early stage of the ADC development, identification of the conditions of payloads that can be used, identification of linkers having various characteristics, and development of various ADC candidate materials by simple conjugation method were carried out. Through these efforts, payloads suitable for ADC (supertoxins having a picomolar $IC_{50}$ value, such as Calicheamicin, MMAE and PBD) were identified, and first-generation ADC products such as Mylotarg and Kadcyla were developed.

After that, various second-generation ADCs have been developed by improving CMC (Chemical, Manufacturing and Control) through site-specific conjugation and developing stable linkers that release drugs only at cancer cells, but many of them have not been successfully developed due to the lack of a therapeutic window. These failures established the fact that a payload that has secured a level of safety that allows the payload to be used alone is needed in addition to a strong anticancer effect, rather than simply using a powerful payload.

Since then, centered on Immunomedics and Daiichi-Sankyo, third-generation ADCs using a payload that have secured a sufficient therapeutic window as a single-administered anticancer agent in the human body were developed, and Trodelvy and Enhertu acquired the FDA approval as an ADC for solid cancer.

[Limitations of Conventional ADCs (Second-generation ADCs): Powerful toxin]

Many of the previously developed payloads have a problem that they cannot secure a therapeutic window when applied to a solid cancer. To overcome this problem, powerful toxins have been used as a payload and a ultrastable linker system in which chemical bonds are selectively broken and the drug is released by Cathepsin B, an enzyme that is present in cells, has been introduced.

As a representative ultrastable linker system, a Valine-Citrulllin (Val-Cit) linker developed by Seattle Genetics in the US is used. Representative ADCs using the same include Adcetris, Polivy, Padcev, and the like (FIG. 13).

Payloads paired with the Val-Cit linker are Vedotin and Vedotin-based drugs, which are highly toxic. Although a very stable linker system such as Val-Cit was introduced to solve the high toxicity problem, there is still a limitation in securing a therapeutic window due to the strong toxicity of the payloads.

[Limitations of Third-Generation ADCs]

A new ADC anticancer agent has been developed by introducing as an ADC payload, instead of a powerful toxin which used to be mainly used in second-generation anticancer drugs, a target anticancer agent that has high potentials being developed as therapeutic agent alone, and based on the excellent safety of the target anticancer agent-based payloads, ADC anticancer agents have been successfully developed as solid cancer therapeutics (FIG. 14).

FIG. 14 shows examples of ADCs (Enhertu (top) and Trodelvy (bottom)) using a topoisomerase I inhibitor Camptothecin-based compound as a payload as a target anticancer agent.

However, these third-generation ADCs have several limitations such that the anticancer efficacy is limited due to the use of payloads, which have relatively limited efficacy compared to the payloads used by the conventional second-generation ADCs, or the third-generation ADCs are inconvenient to use due to the large doses required, and resistance develops in patients with solid cancer.

In particular, many of ADCs that are currently in use or being developed are focused on the use of payloads with only a limited action mechanism and so their treatment options are limited, and they are vulnerable to the problem of resistance development. Therefore, there is a need for developing a new ADC having a new action mechanism and potential for overcoming resistant cancers.

## DETAILED DESCRIPTION OF THE INVENTION

## TECHNICAL PROBLEM

These second-generation and third-generation ADC platforms have several problems mainly in relation to the payloads, such as toxicity, lack of efficacy and generation of resistance.

For the development of a novel ADC, there is an urgent need for developing a new payloads with characteristics such as (1) that has an excellent anticancer efficacy comparable to or higher than that of the existing payloads, (2) that has secured a safety level to be safe in the human body even when the payload itself is used alone as a drug, thereby securing a wide therapeutic window for ADC, and (3) that has overcome the resistance acquisition mechanism of the existing payloads.

## TECHNICAL SOLUTION

The present invention provides a new payload-linker conjugate that has an action mechanism that is differentiated from existing anticancer drugs/payloads, thereby having sufficient anticancer efficacy and the potential to overcome resistance and having sufficient safety even when used alone, in order to secure a wide therapeutic window, and a new ADC development platform using the same.

## ADVANTAGEOUS EFFECTS

The immunoconjugate of the present invention is a hydrophobic small molecule drug, and due to the combined use of a cell membrane-permeable FL118 drug and an acid-sensitive linker, it can solve the problem of the drug not entering the cell and the problem of the antibody that does not easily penetrate into the deep part of a cancerous tissue that are caused by the inefficient internalization process by the antigen-antibody complex. In addition, it can maximize the by-stander effect of entering into not only the targeted cells but also the surrounding cells.

Among the problems identified in the process of developing first to third- generation ADCs, the immunoconjugate of the present invention can overcome the problem of generating resistance to ADCs and the problem of a narrow therapeutic window that appears due to the relatively weak efficacy of the payloads used in the third-generation ADCs. In other words, due to the combined use of the FL118 drug with various resistance overcoming mechanisms and an acid-sensitive linker, the immunoconjugate of the present invention can exhibit an optimized therapeutic window by balancing strong anticancer efficacy and *in vivo* safety compared to the existing ADC payload. In particular, the immunoconjugate of the present invention can overcome the insufficient efficacy and resistance problems accompanied by the existing SN-38-based ADCs through the unique strong efficacy of FL118, and accelerate the development through the use of biomarkers that are capable of identifying patient groups that can best respond to FL118.

## DESCRIPTION OF THE DRAWINGS

FIG. 1 shows a camptothecin-based anticancer drugs: SN-38 (left), Topotecane (middle), and Exatecan (right).

FIG. 2 is a schematic diagram showing the mechanism of efficient drug release and resistance overcoming in the immunoconjugate of the present invention.

FIG. 3 shows the results of comparative evaluation of in vitro cell viability between FL118 and SN-38.

FIG. 4 is a schematic diagram of an example of a carrier-drug conjugate comprising an FL118 drug-acid-sensitive linker.

FIG. 5 shows (5a) the results of verifying the preparation of DAR 4 ADC and (5b) the results of verifying the preparation of DAR 8 ADC by using SEC, HIC, and LC-MS.

FIG. 6 shows the evaluation results of trastuzumab-CL2A-FL118 (DAR 8) of Example 5 in the JIMT-1 cell line.

FIG. 7 shows the evaluation results of trastuzumab-CL2A-FL118 (DAR 4) of Example 6 in the JIMT-1 cell line.

FIG. 8 shows the evaluation results of trastuzumab-CL2A-FL118 (DAR 8) of Example 5 in the JIMT-1 xenograft model. Trastuzumab-FL118 ADC exhibits excellent efficacy in a Kadcyla-resistant JIMT-1 (Her2 over-expression) in vivo xenograft model.

FIG. 9 shows the evaluation results of cetumximab-CL2A-FL118 (DAR 8) of Example 8 in the MDA-MB-468 cell line.

FIG. 10 shows the evaluation results of cextuximab-CL2A-FL118 (DAR 8) of Example 8 in the MDA-MB-468 xenograft model. Cetuximab-FL118 ADC exhibits excellent efficacy in an MDA-MB-468 (EGFR over-expression) in vivo xenograft model.

FIG. 11 shows the evaluation results of sacituzumab-CL2A-FL118 (DAR 8) of Example 9 in the MDA-MB-468 cell line.

FIG. 12 shows the comparative evaluation results of sacituzumab-CL2A-FL118 (DAR 8) of Example 9 with Trodelvy in the MDA-MB-468 xenograft model. Sacituzumab-FL118 ADC exhibits excellent efficacy in an MDA-MB-468 (TROP2 over-expression) in vivo xenograft model compared to Trodelvy, which is an approved TROP2-targeted ADC.

FIG. 13 shows ADCs developed by using a Val-Cit linker: Adcetris (top), Polivy (middle), and Padcev (bottom).

FIG. 14 is a schematic diagram of ADCs using as a payload a Camptothecin-based compound, which is a topoisomerase I inhibitor, as a target anticancer agent: Enhertu (top) and Trodelvy (bottom).

## Best mode for carrying out the invention

A first aspect of the present invention is an immunoconjugate comprising: [an FL118 drug of Formula 1]-[an acid-sensitive linker]-[an antibody or antigen-binding site-containing fragment thereof]; or a pharmaceutically acceptable salt thereof, wherein at least one FL118 drug of Formula 1 is linked to an antibody or antigen-binding site-containing fragment thereof through an acid-sensitive linker, wherein after being targeted to cancer cells by an antigen-binding site that targets an antigen of cancer cells, the acid-sensitive linker is degraded in acidic atmosphere around cancer (pH ≤ 7) to free at least a part of the FL118 drug of Formula 1 and the free FL118 drug of Formula 1 penetrates a cell membrane and moves into the cells, wherein FL118 drug of Formula 1 inhibits the action an efflux pump to enrich the intracellular free FL118 drug of Formula 1, and optionally, wherein the immunoconjugate in which the FL118 drug of Formula 1 is linked is internalized into the cell to free the FL118 drug of Formula 1 at lysosomes.

[Formula 1]

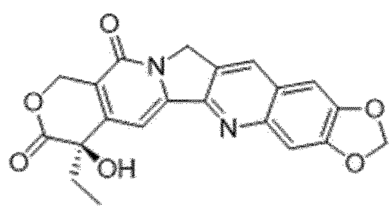

A second aspect of the present invention provides a pharmaceutical composition for preventing or treating cancer comprising the immunoconjugate or a pharmaceutically acceptable salt as an active ingredient of the first aspect.

A third aspect of the present invention provides a drug-linker conjugate or a pharmaceutically acceptable salt thereof, wherein FL118 drug of Formula 1 is linked to an acid-sensitive linker of Formula 2 below in the conjugate.

A fourth aspect of the present invention provides an immunoconjugate or a pharmaceutically acceptable salt thereof, the immunoconjugate comprising: (a) the drug-linker conjugate of the third aspect or a pharmaceutically acceptable salt thereof; and (b) an antibody or antigen-binding site-containing fragment thereof, wherein at least one FL118 drug of Formula 1 is linked to the antibody or antigen-binding site-containing fragment through an acid-sensitive linker of Formula 2 in the immunoconjugate.

A fifth aspect of the present invention provides a method for preparing a carrier-drug conjugate, wherein the

drug-linker conjugate of the first aspect or a pharmaceutically acceptable salt thereof is used to link at least one FL118 drug of Formula 1 to a carrier through an acid-sensitive linker of Formula 2.

Hereinafter, the present invention will be described.

The present invention relates to an immunoconjugate comprising: [an FL118 drug of Formula 1]-[an acid-sensitive linker]-[an antibody or antigen-binding site-containing fragment thereof];

(i) wherein at least one FL118 drug of Formula 1 is linked to an antibody or antigen-binding site-containing fragment thereof through an acid-sensitive linker,
(ii) wherein after being targeted to cancer cells by an antigen-binding site that targets an antigen of cancer cells, the acid-sensitive linker is degraded in acidic atmosphere around cancer (pH ≤ 7) to free at least a part of the FL118 drug of Formula 1 and the free FL118 drug of Formula 1 penetrates a cell membrane and moves into the cells,
(iii) wherein FL118 drug of Formula 1 inhibits the action an efflux pump to enrich the intracellular free FL118 drug of Formula 1, and
(iv) optionally, wherein the immunoconjugate in which the FL118 drug of Formula 1 is linked is internalized into the cell to free the FL118 drug of Formula 1 at lysosomes (FIG. 2).

As illustrated in FIG. 2, the immunoconjugate of the present invention uses an acid-sensitive linker to efficiently release a drug not only from within the cancer cells but also from the surrounding cancer tissue after antigen binding, and so is capable of overcoming the resistance mechanism related to ADC processing.

In addition, the FL118 drug of Formula 1 freed from the immunoconjugate of the present invention is not released out of the cell by ABCG2, which is an efflux pump, and is capable of blocking resistance by various anti-apoptic proteins.

The FL118 drug of Formula 1 is a triple-targeted anticancer drug which, in its free state, directly targets topoisomerase I, a well-established anticancer target, but it simultaneously inhibits the Bcl family such as Survivin, which is a resistance protein involved in the resistance mechanism, and inhibits the action of the efflux pump.

For an ADC to work efficiently, it is not enough to simply maximize strong antigen-selective cytotoxicity to cells *in vitro,* and an ADC must be able to effectively penetrate cancer tissues *in vivo* or in actual patients to efficiently deliver drugs. After the immunoconjugate of the present invention is targeted to a cancer cell by an antigen-binding site that targets an antigen of the cancer cell, the acid-sensitive linker is degraded in an acidic atmosphere (pH ≤ 7) around the cancer to free at least a part of the FL118 drug of Formula 1 and the free FL118 drug of Formula 1, which is a hydrophobic small molecule, can penetrate a cell membrane and move into the cells while penetrating deep into the cancer tissue.

Therefore, the immunoconjugate of the present invention can rapidly release a drug from the tumor microenvironment surrounding cancer by using an acid-sensitive linker that is degraded in acidic atmosphere (pH ≤ 7) surrounding the cancer, and the free FL118 drug, having a low molecular weight, has high cancer tissue penetration power, in contrast to an antibody, and thus is capable of solving the problem of the existing ADCs, which is that the antibodies fail to penetrate well into the deep parts of a cancer tissue.

Therefore, the cells to which the free FL118 drug of Formula 1 moves may be targeted cancer cells and/or surrounding cells thereof.

In addition, the FL118 drug of Formula 1 is (a) a hydrophobic small molecule that is capable of penetrating a cell membrane, and (b) is not released out of the cell through an efflux pump. Therefore, the FL118 drug freed from the immunoconjugate of the present invention by the linker degradation outside the cells surrounding the cancer can be rapidly accumulated in the cancer tissue to maintain a high concentration for a long time. In addition, the free FL118 drug can penetrate the cell membrane and exerts cytotoxicity inside the cell to cause apoptosis, and then be released to continuously penetrate the cell membrane of also the surrounding cells and move into the cells for action.

In short, the drug-linker conjugate of the present invention is characterized in that:

(i) an acid-sensitive linker such as CL2A linker of Formula 2 is used in order to free a hydrophobic small molecule drug that can penetrate the cell membrane and play its proper role inside the cell in a tumor microenvironment surrounding cancer of acidic atmosphere (pH ≤ 7), and then have a large amount of the free hydrophobic small molecule drug be introduced to the cell and/or penetrate deep into the tissue; and
(ii) FL118 drug of Formula 1, which is a hydrophobic small molecule drug that can penetrate the cell membrane and inhibit the action of the efflux pump, is used in order that the acid-sensitive linker may be degraded in a tumor microenvironment surrounding cancer of acidic atmosphere (pH ≤ 7) so that a large amount of free drug may penetrate the cell membrane and move into the cell to be highly enriched in the cell;

and another characteristic is that the organic action mechanism of the FL118 drug-acid sensitive linker is utilized through the combined use thereof.

The FL118 drug of Formula 1 and the acid-sensitive linker are preferably linked by a carbonate or ester bond so that they may be degraded in acidic atmosphere (pH ≤ 7) and the free FL118 drug of Formula 1 may be released

upon the degradation of the acid-sensitive linker (FIG. 4).

In the present invention, the [acid-sensitive linker]-[antibody or antigen-binding site-containing fragment thereof] link may be formed as a thiol group contained in the antibody or antigen-binding fragment thereof is bonded to a maleimide group or a maleic hydrazide group of the acid-sensitive linker through the "click" reaction of Scheme 1.

[Scheme 1]

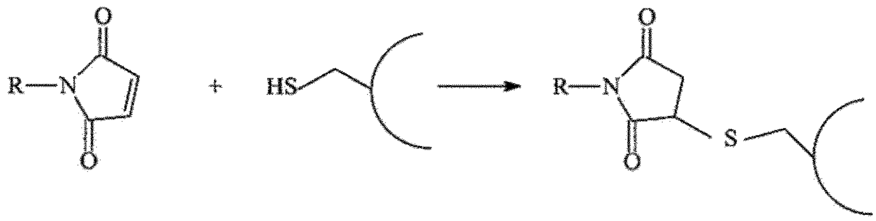

maleimide cysteine residue in protein modified cysteine

In addition, the present invention provides a drug-linker conjugate in which the FL118 drug is linked with various acid-sensitive linkers, and a carrier-drug conjugate in which the FL118 drug is linked by the same with various carriers through various acid-sensitive linkers.

Furthermore, the present invention also provides a method for preparing a carrier-drug conjugate, wherein the drug-linker conjugate of the present invention or a pharmaceutically acceptable salt thereof described above is used to link to a carrier at least one of the FL118 drugs of Formula 1 through the acid-sensitive linker of Formula 2.

FIG. 4 shows an example of a structure of a carrier-drug conjugate in which linked to a carrier by using the drug-linker conjugate of the present invention.

[Competitive advantage of FL118 drug linked through an acid-sensitive linker]

The immunoconjugate of the present invention is targeted to a cancer cell by an antigen-binding site targeted to antigen of the cancer cell, and then is degraded mainly in acidic atmosphere ($pH \leq 7$) surrounding the cancer and/or some acid-sensitive linkers are degraded in the blood. Therefore, in terms of anticancer efficacy and stability issues according to the action mechanism of the free FL118 drug of Formula 1, which begins outside the cell, the competitive advantage of the free FL118 drug of Formula 1 is described as below.

FL118 drug of Formula 1 is a strong Topoisomerase I inhibitor and has the same camptothecin moiety structure such as SN-38, topotecan, exatecan, etc., which are the existing commercially available Top 1 inhibitors (FIG. 1), but even when administered alone in various cancer cell and animal models, it exhibits anticancer efficacy and excellent safety that are differentiated from SN-38, and thus has secured a wide therapeutic window. Specifically, FL118 drug has a Top1 inhibitory efficacy that is equivalent to or higher than that of SN-38 in cancer cells, and exhibits 5 to 20 times more powerful cytotoxicity than that of SN-38 in various cancer cell lines, as represented by a low numerical value of the $IC_{50}$ level (FIG. 3). In addition, the results of the evaluation performed with 140 cell lines originating from various cancers showed that FL118 drug has very strong anticancer efficacy with an $IC_{50}$ of <100 nM against most cancer cells.

FL118 drug can overcome various resistance action mechanisms of SN-38/exatecan. Camptothecin-based anticancer drugs such as SN-38 show resistance in the way that the drug is discharged out of the cell due to the overexpression of ABCG2 transporter, but FL118 drug is not affected by ABCG2 Transporter, so it can overcome resistance by ABCG2 Transporter.

On the other hand, camptothecin-based anticancer drugs show excellent anticancer response in the early stage when used in patients, but strong resistance to these drugs is shown through epigenetic silencing of Top1 gene and Top2 dependence of cancer cells. In contrast, FL118 drug showed strong efficacy in the xenograft model of the cancer cell lines in which Top1 is not expressed through epigenetic silencing or knock-out.

FL118 drug can block the expression of resistance by strongly inhibiting at low concentrations the expression of anti-apoptic protein (Survivin, cIAP2, XIAP, etc.), which is another major cause of resistance to anticancer drugs.

FL118 drug has a PK/safety profile that is optimal for targeted drug delivery (e.g., carrier-drug conjugate) applications. When FL118 drug alone is administered systemically, it is rapidly metabolized/released from the blood and shows only a low concentration, but it is rapidly accumulated in cancer tissues immediately after the administration and maintains a high concentration for a long time. For example, in the ADC application, FL118 drug ensures maximum selectivity between tumor tissue and normal tissue.

Excellent safety of FL118 drug has been secured through a GLP-toxicity test in rats and beagle dogs, and so

FL118 drug is suitable for ADC payload having a secured therapeutic window. FL118 drug showed superior efficacy compared to SN-38 in various cancer cell line xenograft models.

When administered in vivo in the same amount as SN-38, FL118 drug showed strong tumor regression efficacy compared to SN-38 in colorectal cancer, head and neck cancer, and pancreatic cancer. FL118 drug had strong anticancer efficacy even when FL118 was administered after inducing SN-38 resistance in a tumor xenograft. The possibility of FL118 drug for overcoming various mechanisms of the resistance to SN-38/exatecan was confirmed in vivo.

In short, in the conjugate of [FL118 drug of Formula 1]-[acid-sensitive linker], the FL118 drug has competitive advantages as a payload as described below:

(i) FL118 drug creates a strong anticancer effect by selectively inhibiting/ degrading other anticancer targets, DDX5, UBE2T and USP2a, in addition to Top 1, which is a well-proven anticancer target;
(ii) FL118 drug exhibits strong cytotoxicity that is 5 to 10 time stronger than that of SN-38 and cytotoxicity equal to or higher than that of exatecan so that it can secure strong drug efficacy to exhibit a sufficient efficacy as ADC and/or other drug complexes;
(iii) FL118 drug is not a substrate of ABCG2 and P-GP, unlike SN-38 and exatecan and so it can avoid the resistance mechanism by overexpression of these transporter proteins;
(iv) FL118 drug exhibits excellent efficacy in an animal cancer model in which Top 1 epigenetic silencing/Top 2 overexpression, which is a major mechanism of generating Top 1 inhibitor resistance, has occurred;
(v) FL118 drug can avoid resistance by fundamentally blocking the overexpression of anti-apoptic protein, which is a common mechanism for generating resistance in most cancers; and
(vi) biomarkers (DDX5, K-ras, p53) and companion diagnostic techniques that can predict anticancer response in patients have already been established, customized biomarkers have been secured to provide customized treatment to individuals through companion diagnosis, and in particular, FL118 drug exhibits strong efficacy in p53/K-ras mutant cancer cells with poor prognosis.

In particular, the FL118 drug of Formula 1 can maximize the efficacy of the main drug target modulation by additional actions for (i) simultaneously inhibiting the Bcl family such as resistance protein survivin and/or (ii) inhibiting the action of an efflux pump.

In other words, deviating from the existing drug development method in which therapeutic efficacy is limited as a single target is targeted to a single drug, the FL118 drug that is linked to a carrier through an acid-sensitive linker target according to the present invention was selected by a well-designed polypharmacology-based approach for obtaining excellent therapeutic efficacy by simultaneously targeting multiple drug targets that need to be controlled together, and thus it can treat intractable diseases in which existing therapeutics have not shown a clear therapeutic effect.

In addition, in a recurrent/resistant cancer, a sufficient efficacy cannot be obtained only by controlling the activity of a single drug target, and safety cannot be guaranteed when a plurality of unspecified drug targets are simultaneously targeted. However, the FL118 drug that is linked to a carrier through an acid-sensitive linker target according to the present invention is capable of utilizing two or more well-selected drug targets to show a synergic effect in terms of drug efficacy and ensure sufficient safety at the same time.

[Competitive advantage of immunoconjugates in which FL118 drug is linked through an acid-sensitive linker]

The immunoconjugate according to the present invention uses FL118 drug that inhibits the action of an efflux pump by linking the same through an acid-sensitive linker so that the abovementioned competitive advantages of the FL118 drug compared to camptothecin-based anticancer drugs with a similar structure used in Enhertu and Trodelvy may be reflected. Therefore, the immunoconjugate according to the present invention can have resistance-overcoming capabilities that are differentiated from the currently available third-generation ADCs (Enhertu, Trodelvy) and secure a wider therapeutic window.

In particular, in addition to the inhibition of the proven tumor target Topoisomerase I, the immunoconjugate according to the present invention can exhibit, through the FL118 drug, strong efficacy for overcoming resistance by further inhibiting cancer resistance-inducing proteins DDX5, UBE2T, and USP2a. Therefore, the immunoconjugate according to the present invention can be used as an ADC for overcoming resistance in the fields where conventional SN-38-based ADCs, such as Trop-2/CEACAM-5, are successfully developed.

In short, Trop-2-SN-38 ADC is currently developed successfully in many cancers including TNBC, SN-38bladder cancer, and gastric cancer, but there still remain the resistance problems of SN-38 (overexpression of drug efflux transporters, epigenetic silencing of Top1, anti-apoptic protein increase, etc.). The problems can be overcome by utilizing the immunoconjugate comprising [FL118 drug of Formula 1]-[acid-sensitive linker]-[antibody or antigen-binding site-containing fragment] according to the present invention.

The immunoconjugate according to the present invention utilizes related biomarkers such as DDX5, K-ras, and

p53 to identify the optimal response patient group among the antigen-expressing patient groups such as Trop-2 and CEACAM-5 to secure the optimal therapeutic response.

[Payload for various carrier-drug conjugates]

Since FL118 drug satisfies all of the characteristics (1) to (5) described below, FL118 drug was selected in the present invention as a payload for various carrier-drug conjugates.

(1) Exatecan, SN-38 and others have actually been developed as payloads for ADCs among drugs of the same class;
(2) Topoisomerase I inhibitors (Irinotecan, Topotecan, etc.), which are an anticancer mechanism of which efficacy/safety has been verified in clinical practice, are used, wherein the excellent anticancer efficacy of Topoisomerase I inhibitors have been verified in clinical practice with various intractable solid cancers, such as colorectal cancer, lung cancer, breast cancer, and ovarian cancer;
(3) FL118 drug has secured efficacy in resistant/recurrent cancers by overcoming many of the payload-targeted resistance mechanisms among existing camptothecin-based drugs;
(4) FL118 drug is a drug with a wide therapeutic window that can secure both anticancer efficacy and safety even when administered alone; and
(5) FL118 drug has a PK profile in which the drug stays in the cancer cells of a cancer tissue for a long time but is rapidly removed once discharged to the blood, and thus, when used as a payload of ADC, FL118 drug may secure a wider therapeutic window and remain stable when it is freed from ADC near cancer cells.

[Linker technology for efficient drug delivery]

Linkers that are commonly used in ADC include acid-labile hydrazones, protease-labile peptides, and reducing agent-sensitive disulfides. So-called "non-cleavable" thioether linkers that are not cleaved are also used in ADC.

In the case of ADCs using super toxins such as MMAE, calicheamicin, and PBD, the Stable Linker system, which aimed to minimize the separation of the drug in the blood before reaching the cancer tissue (characteristic of the second-generation ADC), was used.

In the operating principle of most of the second-generation ADCs in cancer cells, the first step is the antibody portion constituting the ADC binds to the antigen overexpressed in the cancer cell; the second step is that the ADC bound to the antigen on the surface of the cancer cell by an antigen-antibody reaction is transported into the cancer cell through endosomes and lysosomes; the third step is that the antigen-antibody portion is degraded in the lysosome and an enzymatic (cathepsin B) drug is released; and the final step is that the cancer cell is killed by the drug released from the inside of the cancer cell.

In order to develop a new ADC that can be selectively used for various solid cancers, it is necessary to utilize a linker system that exceeds the drug delivery efficiency of existing ADCs. In pursuit of developing ADCs targeting new antigens beyond the established drug targets such as Trop-2, Her2, and folate receptor ADCs, ADCs should be developed with respect to slow internalizing antigens such as CEACAM-5 and/or cancer-specific antigens such as NY-ESO-1/HLA complex. However, it is difficult to deliver a sufficient amount of drug with the limited drug delivery efficiency of the existing Val-Cit or MAC-glucuronide linker system.

In particular, for the efficient drug delivery to antigens such as CEACAM-5, it is necessary that an antibody-drug conjugate should maintain stability in the blood and/or the surrounding environment of normal tissues but rapidly release the drug in the environment surrounding cancer cells, such as the tumor microenvironment.

If the drug can be rapidly released from the tumor microenvironment surrounding the cancer, the antibody-drug conjugate can also be advantageously used for targeting various antigens (typically CEACAM-5, various cancer specific antigen-HLA complex, etc.) that have limitations in the ADC uptake rate. Moreover, since the FL118 drug has an excellent therapeutic window even when it is used alone, in the immunoconjugate using the FL118 drug, a release profile in which the drug is rapidly and efficiently delivered after reaching a cancer tissue is required of the linker more strongly than the stability for minimizing the separation of the FL118 drug in the blood. Therefore, the acid-sensitive linker used in the present invention, which is degraded in acidic atmosphere ($pH \leq 7$) surrounding a cancer, was selected by taking the advantage of the free FL118 drug of Formula 1 that is a hydrophobic low-molecular weight drug capable of penetrating deep into the cancer tissue and moving into the cells through the cell membrane.

The antibody to which the FL118 drug of Formula 1 is linked through an acid-sensitive linker according to the present invention is bound to an antigen overexpressed on the surface of cancer cells in the same way as the first step in which the second-generation ADC operates in cancer cells, but some of the antibodies can undergo an intracellular processing step, the same as the second-generation ADC, and a considerable other portion of the antibodies can release the drug due to the low pH around the cancer cells. Afterwards, the drug released from the cancer tissue moves into the cancer cells by diffusion without going through endosomes or lysosomes, and it acts directly on the cancer cells to

induce apoptosis without an enzyme (cathepsin B) reaction. Therefore, when compared with the second-generation ADCs in which drug release is possible only by an enzyme reaction, the antigen selectivity of cancer cells is the same, but the main characteristic of the immunoconjugate of the present invention is that a pH-sensitive linker is used to maximize the efficiency of releasing the drug and transferring the same into cancer cells.

In the [FL118 drug of Formula 1]-[acid-sensitive linker] of the present invention, for free FL118 drug of Formula 1 to be released when the acid-sensitive linker is degraded, the FL118 drug of Formula 1 is preferably linked with the acid-sensitive linker by a carbonate or ester bond.

Generally, in relation to hydrolysis, a carbamate bond provides better drug linker stability compared to ester and carbonate bonds. However, the present invention is characterized in that an unstable ester or carbonate bond is employed instead of a carbamate bond so that the linker can be designed such that the FL118 drug is separable from the drug linker in both outside and inside the cells surrounding the cancer cells in acidic atmosphere (pH).

Blood has a constant pH between 7.3 and 7.4. Therefore, the FL118 drug is not cleaved from the acid-sensitive linker in the blood, and even if it is cleaved, the release rate of the FL118 drug from the ADC at the neutral pH of the serum is much lower than the release rate in the tumor tissue in acidic atmosphere. Unlike humans, mouse plasma has a high level of esterase activity and thus easily cleaves ester and carbonate functional groups, which may be a problem in the mouse model experiment of Example 12. Nevertheless, it was confirmed that the FL118 drug-containing ADC showed no side effects such weight loss due to normal cell attack upon the separation and release of the FL118 in the blood (Example 12).

CL2A, a linker used in Trodelvy, which is an existing FDA-approved ADC, is a linker that satisfies all the properties of

(i) the storage stability after manufacture, (ii) the stability in the blood upon administration (almost no free payload exposure in the plasma), and (iii) the rapid release of the payload in cancer tissue.

As the acid-sensitive linker used in the present invention, the CL2A linker may be used, because it has already proved that the CL2A linker can be successfully applied to the ADC development of camptothecin-based compounds through the successful development of Trodelvy and IMMU-130 and the like, and the linker may be designed as shown in Formula 2 below to deliver the FL118 drug selectively and efficiently to cancer tissues. In other words, the acid-sensitive linker of the present invention may be derived from a compound of Formula 2 below.

[Formula 2]

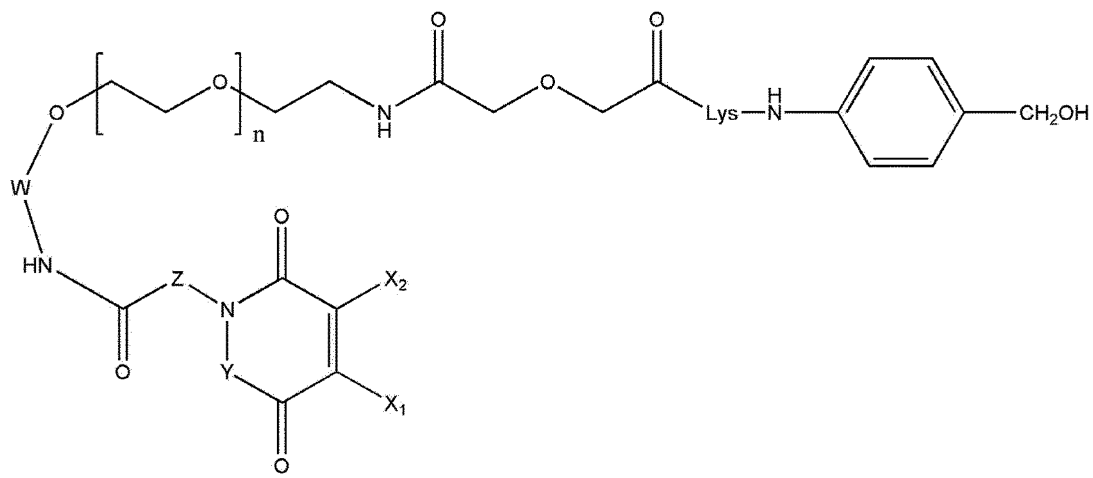

wherein, $X_1$ and $X_2$ are each independently -H or -halogen;

Y is -NH-, -$NR^A$ -, or null;

Z is -$C_1$-$C_4$ alkyl-, -$C_3$-$C_6$ cycloalkyl-, - ($C_1$-$C_2$ alkyl)-($C_3$-$C_6$ cycloalkyl)-, -($C_3$-$C_6$ cycloalkyl)-($C_1$-$C_2$ alkyl)-, or - ($C_1$-$C_2$ alkyl)-($C_3$-$C_6$ cycloalkyl)-($C_1$-$C_2$ alkyl)-;

W is -$R^B$-, -M- -$R^B$-M-, -M-$R^B$ - or -$R^B$-M-$R^C$-;

$R^A$ to $R^C$ are each independently $C_1$-$C_4$ alkyl;

M is

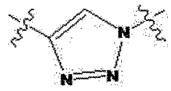
;

and
n is an integer from 5 to 9.

In the present invention, the length of the linker, that is, n in Formula 2 may be an integer from 5 to 9, specifically, n may be an integer from 6 to 8, and more specifically, n may be 7, but is not limited thereto. Even when out of the range described above, if there is no significant difference in the effect according to the change in the linker length, all are naturally included within the equivalent scope of the present invention. Since the solubility of the drug can be improved by placing a polyethylene glycol (PEG) spacer between the drug and the carrier, the linker of Formula 2 contains a low-molecular weight PEG moiety comprising a finite number (n=5 to 9) of PEG monomers. The acid-sensitive linker of Formula 2 is a customized linker that can be optimized according to the characteristics of the target, payload, and carrier.
The FL118 drug of Formula 1 has various linkers and sites for easy attachment for the preparation of carrier-drug conjugates. For example, an alcohol group site of the drug of Formula 1 may be used as an attachment site with a linker.
Therefore, in the present invention, [FL118 drug of Formula 1]-[acid-sensitive linker] may be one in which the alcohol group site of the FL118 drug of Formula 1 and the alcohol group site of the acid-sensitive linker of Formula 2 are linked.
It was confirmed through experiments that various conjugates of the FL118 drug-acid-sensitive linker are stable at pH 7.4 and/or plasma conditions, but they readily release the drug under tumor lysosome conditions (pH 4.5 and proteolytic enzyme). In addition, it was confirmed that FL118 can be attached to various antibodies by using the CL2A linker, a linker system that has been used for FDA-approved ADCs.
In addition, a method for easily preparing FL118-ADC, which is DAR 2, through site-specific conjugation was secured. A method for preparing ADCs of DAR 4 and 8 through the partial reduction of the interchain S-S bond of the antibody and conjugation was secured (FIG. 5).
The stability of the acquired ADC was evaluated, and it was confirmed that the ADC can be stored stably under low temperature conditions when stored in the form of freeze-dried powder or solution that can subject to re-constitution.
It was confirmed that each of the acquired DAR 2, 4, and 8 ADCs maintained antigen-binding affinity at a level similar to that of the original antibody.
In the present invention, the [FL118 drug of Formula 1]-[acid-sensitive linker] conjugate may be any one selected from the group consisting of compounds represented by Formulas 3 to 5 below.

[Formula 3]

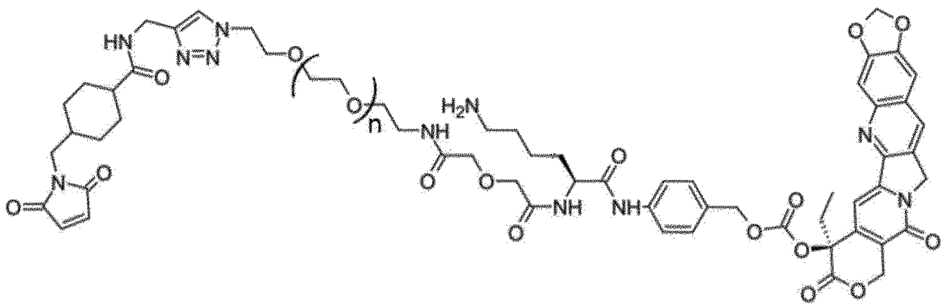
;

[Formula 4]

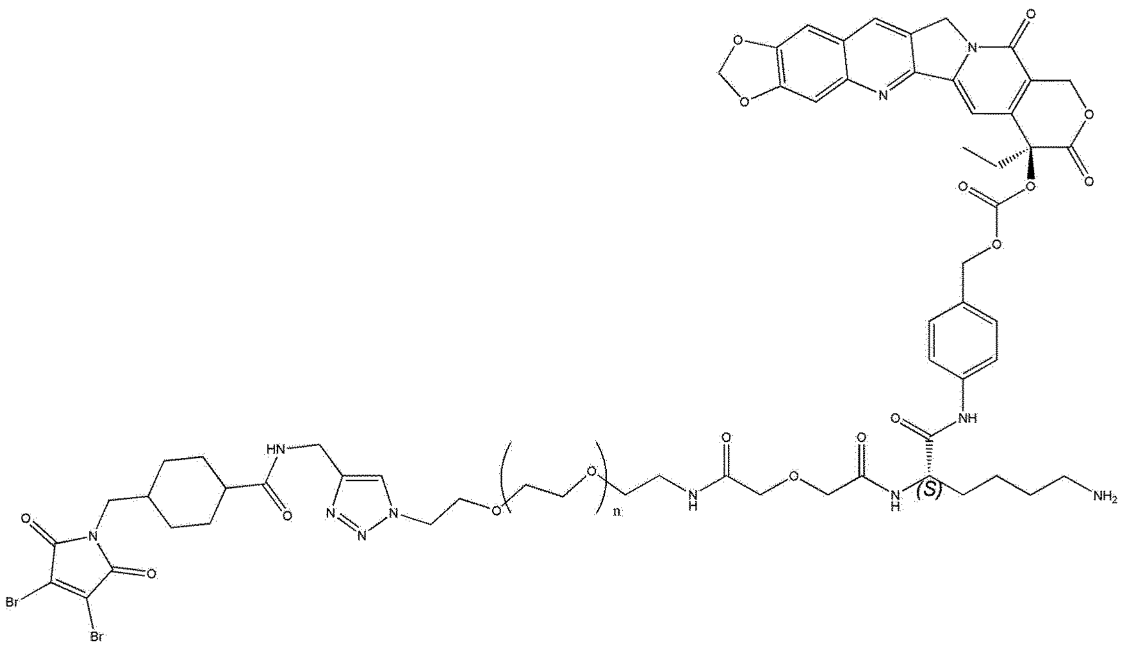

;

and

[Formula 5]

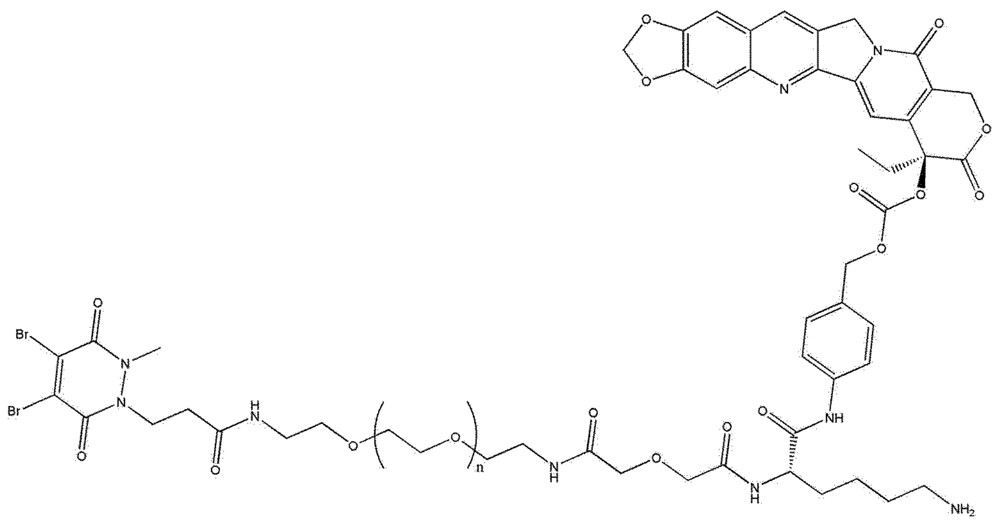

(wherein n is each independently an integer from 5 to 9).

In order to improve the drug delivery efficiency of the acid-sensitive linker of the present invention, a multivalent linker system that can overcome the problem of low DAR, which is considered a disadvantage of site-specific conjugation, may be developed and applied so that a method for preparing a high DAR ADC having a DAR from 4 to 12 even at site-specific conjugation may be secured by attaching two or three payloads to one attachment site

To this end, in the present invention, a bridgeable linker system exemplified in Formulas 4 and 5 may be used as a linker.

Such a bridgeable linker system can increase the efficiency of CMC and simplify the process so that the ADC of DAR 4 can be easily prepared, while maintaining the fast release characteristic of the payload in cancer tissue, which is the greatest advantage of the CL2A linker system. A site-specific antibody-drug complex of DAR 4 can be prepared

without performing separate antibody engineering. When a disulfide (-S-S-) that is present in the antibody is reduced, two thiol groups (-SH) are formed, and the generated thiols form a 2:1 conjugate with a new bridgeable linker-FL118 compound. Therefore, by reacting all the four disulfide groups in general antibodies, an antibody-drug complex of DAR 4 can be easily prepared as a single product.

[Immunoconjugate]

As used in the present invention, the term "immunoconjugate" refers to a complex in which a cytotoxic drug-linker conjugate is linked to an antibody or antigen-binding fragment thereof. Since an antibody-drug conjugate (ADC) is an example of an immunoconjugate, in the present invention, the description of ADC and the description of immunoconjugate may be used interchangeably.

In the present invention, the term "drug-linker conjugate" refers to a material for the preparation of an immunoconjugate or carrier-drug conjugate, wherein an antibody, antigen-binding site-containing fragment or carrier is not linked. In addition, according to the purpose, a drug-linker conjugate may be conjugated with any antibody, antigen-binding site-containing fragment, or carrier to be used as an immunoconjugate or carrier-drug conjugate.

When the immunoconjugate is administered in vivo, an antibody or antigen-binding site-containing fragment thereof, which is a component thereof, binds to a target antigen and then releases the drug so that the drug can act on the target cells and/or the surrounding cells. Therefore, excellent drug efficacy and reduced side effects can be expected.

Factors that have significantly impacts on the effectiveness of an immunoconjugate include, in particular, (1) drug potency, (2) drug linker stability, and (3) efficient on-target drug release. Since several factors have a complex effect on the effect, it is very difficult to predict the effect of an immunoconjugate, which is a combination of the factors, based only on the facts known about the individual factors.

Immunoconjugates designed to release a drug after internalization have a problem that a sufficient concentration of active drug cannot be delivered into the cell when the internalization process is inefficient, and there is the disadvantage that even if a hydrophobic drug is adopted as a cytotoxic drug, a by-stander cell-killing is hardly expected.

In order to solve this problem, the immunoconjugate of the present invention employs:

(i) an acid-sensitive linker to free a hydrophobic drug that can penetrate the cell membrane in the tumor microenvironment ($pH \leq 7$) around the cancer and play a proper role inside the cell, and then to introduce a large amount of the free hydrophobic drug into the cell; and

(ii) the FL118 drug of Formula 1, which is a hydrophobic drug that can penetrate the cell membrane and inhibit the action of an efflux pump so that when the acid-sensitive linker is degraded in acidic atmosphere ($pH \leq 7$) around the cancer cells and the drug is freed, a large amount of the free drug can penetrate the cell membrane and can be highly enriched in the cells (FIG. 2) .

FL118 drug may exhibit cytotoxicity to cancer cells as a Topoisomerase I inhibitor, but no example of preparing an immunoconjugate by using the drug has been reported at all until now.

The FL118 drug of Formula 1 is (a) a hydrophobic small molcule that can penetrate the cell membrane and (b) that is not discharged out of the cells through an efflux pump. Therefore, it can be rapidly accumulated in a cancer tissue and can maintain a high concentration for a long time. In addition, it exhibits a high concentration and high cytotoxicity in the cells to cause apoptosis, and then it is released to continuously move into the surrounding cells and act in the cells by penetrating the cell membrane.

The immunoconjugate of the present invention is targeted to a cancer cell by an antigen-binding site targeting an antigen of the cancer cell, and then acid-sensitive linkers are degraded mainly in acidic atmosphere ($pH \leq 7$) around the cancer and/or degraded partially in blood so that the action mechanism of the FL118 drug of Formula 1 is mainly initiated outside of the cells. Therefore, exhibiting the excellent anticancer efficacy and stability described above is a surprising result that could not be predicted until the results were confirmed by experiments. FL118 drug of Formula 1 is named as (4S)-4-ethyl-4-hydroxy-8,9-methylenedioxy-1H-pyrano[3',4':6,7]indolizino[1,2-b]quinoline-3,14(4H,12H)-dione (PubChem CID 72403).

Although not limited thereto, even if a simple function group, such as $C_{1-3}$ alkyl, hydroxy, halogen, and amine groups, is added or an existing hydroxy, ethyl and oxo functional group is substituted with another functional group or removed in the compound of Formula 1 above, it is obvious that an immunoconjugate, as long as it exhibits an efficacy equal to that of the immunoconjugate of the present invention, is included within the equivalent scope of the present invention.

As described above, since the immunoconjugate of the present invention has technical characteristics in using FL118 drug and an acid-sensitive linker, the immunoconjugate can be designed by binding any antibody or antigen-binding site-containing fragment thereof according to a desired purpose. All of these are included within the scope of the present invention. In the present invention, as a specific example, an immunoconjugate was prepared by binding

trastuzumab, cetuximab and sacituzumab to the FL118-linker conjugate of the present invention, and their excellent anticancer effects were confirmed.

In the present invention, the immunoconjugate may have an average drug-to-antibody ratio (DAR) of 2 to 12, preferably, DAR 4 to 12.

[ADC platform design]

The ADC platform according to the present invention may be designed under the basic design concepts described below.

(1) targeting an antigen that is distributed more than 10 times in cancer cells compared to normal cells;
(2) an acid-sensitive linker that is capable of efficiently releasing a drug in cancer cells; and
(3) FL118 drug that has a strong efficacy to exhibit tumor regression even when used alone and that secures safety at the same time.

By applying the ADC platform according to the present invention as described above, various antibodies can be utilized, and the tolerance and resistance to Trodelvy and Enhertu, the existing third-generation ADCs, can be overcome.

[FL118 drug - CL2A linker - antibody platform preparation process] <FL118 drug - CL2A linker synthesis>

An FL118 drug - CL2A linker conjugate may be synthesized in two major steps. In Step 1, the intermediate, $N_3$-$PEG_8$-Lys(MMT)-PABOH, is synthesized, and the synthesized intermediate is conjugated with FL118 in Step 2 to synthesize FL118-CL2A.

More specifically, in Step 1, Fmoc-Lys(MMT)-OH and 4-Aminobenzyl alcohol are synthesized by amide coupling using EEDQ, and after removing the protecting group, which is Fmoc, $N_3$-$PEG_8$-COOH and EEDQ are used to perform a reaction. After the reaction, the product is separated and purified to synthesize $N_3$-$PEG_8$-Lys(MMT)-PABOH, which is an intermediate.

In Step 2, the previously synthesized $N_3$-$PEG_8$-Lys(MMT)-PABOH and FL118 are subjected to a reaction by using triphosgene and DMAP to synthesize $N_3$-$PEG_8$-Lys(MMT)-PABC-FL118. MCC-alkyne, which is a portion needed for the conjugation with an antibody, is synthesized by performing a click reaction of the azide of $N_3$-$PEG_8$-Lys(MMT)-PABOH by using CuBr, $PPh_3$, and DIPEA. After that, MMT, an amine protecting group of lysine, is removed to synthesize the FL118 drug-CL2A linker conjugate as a final product.

<Antibody and FL118-CL2A conjugation>

To conjugate to the FL118 drug-CL2A linker conjugate an antibody (e.g., trastuzumab, which is an Her2 target monoclonal antibody; cetumximab, which is an EGFR target monoclonal antibody, and sacituzumab, which is a Trop-2 target antibody), conjugation is performed by forming a bond between a cysteine residue generated by reducing a disulfide bond of the antibody and a maleimide functional group of the FL118 drug-CL2A linker conjugate.

At that time, in order to produce ADC of DAR 4 or 8, in a reaction buffer (20mM Histidine, 150mM NaCl, pH 6.0), TCEP (tris(2-carboxyethyl)phosphine hydrochloride) of 2.8 or 7.5 times (DAR 4 and 8 ADC, respectively) compared to the antibody was used to reduce the disulfide bond of the antibody, and then the TCEP remaining after the reaction was removed by using a desalting column.

In the reaction buffer, 10 or 12 times (DAR 4, 8 ADC, respectively) amount of FL118-CL2A compared to the antibody is reacted with reduced antibody. The FL118-CL2A remaining after the reaction was removed by using a desalting column, and the buffer was replaced with 18 mM MES (pH 6.5).

The prepared ADC was subject to sterile filtration by using a 0.2 $\mu$m PVDF membrane filter, and added with 0.07 mM Polysorbate 80 (PS80) and 20 mM Trehalose dehydrate. The resulting mixture was freeze-dried and stored.

[Target antigen_drug target]

In the design of the ADC or immunoconjugate of the present invention, the targeted targets may be extended to not only cancer cells but also infectious disease organisms and/or cells associated with autoimmune diseases.

Therefore, the cells targeted by the antibody or antigen-binding site-containing fragment thereof may be a cancer cell, an infectious disease organism and/or a cell associated with an autoimmune disease.

Non-limiting examples of target antigens include antigens selectively distributed on the surface of cancer, such as Her2, FolR and PSMA, and cancer cell overexpression antigens, which are also distributed in a small number in

normal tissues, such as Trop2.

Cancer cell specific antigens include, for example, 5T4, ABL, ABCF1, ACVR1, ACVR1B, ACVR2, ACVR2B, ACVRL1, ADORA2A, AFP, Aggrecan, AGR2, AICDA, AIF1, AIGI, AKAP1, AKAP2, ALCAM, ALK, AMH, AMHR2, ANGPT1, ANGPT2, ANGPTL3, ANGPTL4, ANPEP, APC, APOCI, AR, aromatase, ASPH, ATX, AX1, AXL, AZGP1 (zinc-a-glycoprotein), B4GALNT1, B7, B7.1, B7.2, B7-H1, B7-H3, B7-H4, B7-H6, BAD, BAFF, BAG1, BAI1, BCR, BCL2, BCL6, BCMA, BDNF, BLNK, BLR1 (MDR15), BIyS, BMP1, BMP2, BMP3B (GDFIO), BMP4, BMP6, BMP8, BMP10, BMPR1A, BMPR1B, BMPR2, BPAG1 (plectin), BRCA1, C19orflO (IL27w), C3, C4A, C5, C5R1, CA6, CA9, CANT1, CAPRIN-1, CASP1, CASP4, CAV1, CCBP2 (D6/JAB61), CCL1 (1-309), CCLI1 (eotaxin), CCL13 (MCP-4), CCL15 (MIP-Id), CCL16 (HCC-4), CCL17 (TARC), CCL18 (PARC), CCL19 (MIP-3b), CCL2 (MCP-1), MCAF, CCL20 (MIP-3a), CCL21 (MEP-2), SLC, exodus-2, CCL22 (MDC/STC-I), CCL23 (MPIF-I), CCL24 (MPIF-2/eotaxin-2), CCL25 (TECK), CCL26 (eotaxin -3), CCL27 (CTACK/ILC), CCL28, CCL3 (MIP-Ia), CCL4 (MIPIb), CCL5 (RANTES), CCL7 (MCP-3), CCL8 (mcp-2), CCNA1, CCNA2, CCND1, CCNE1, CCNE2, CCR1 (CKR1/HM145), CCR2 (mcp-IRB/RA), CCR3 (CKR3/CMKBR3), CCR4, CCR5(CMKBR5/ChemR13), CCR6 (CMKBR6/CKR-L3/STRL22/DRY6), CCR7 (CKR7/EBI1), CCR8 or CDw198 (CMKBR8/TERI/CKR-L1), CCR9 (GPR-9-6), CCRL1 (VSHK1), CCRL2 (L-CCR), CD13, CD164, CD19, CDH6, CDIC, CD2, CD20, CD21, CD200, CD22, CD23, CD24, CD27, CD28, CD29, CD3, CD33, CD35, CD37, CD38, CD3E, CD3G, CD3Z, CD4, CD40, CD40L, CD44, CD45RB, CD47, CD52, CD56, CD69, CD70, CD72, CD74, CD79A, CD79B, CD8, CD80, CD81, CD83, CD86, CD97, CD99, CD117, CD125, CD137, CD147, CD179b, CD223, CD279, CD152, CD274, CDH1 (E- cadherin), CDH1O, CDH12, CDH13, CDH18, CDH19, CDH2O, CDH3, CDH5, CDH7, CDH8, CDH9, CDH17, CDK2, CDK3, CDK4, CDK5, CDK6, CDK7, CDK9, CDKN1A (p21Wap1/Cip1), CDKN1B (p27Kip1), CDKN1C, CDKN2A (p16INK4a), CDKN2B, CDKN2C, CDKN3, CEA, CEACAM5, CEACAM6, CEBPB, CERI, CFC1B, CHGA, CHGB, chitinase, CHST1O, CIK, CKLFSF2, CKLFSF3, CKLFSF4, CKLFSF5, CKLFSF6, CKLFSF7, CKLFSF8, CLDN3, CLDN6, CLDN7 (claudin -7), CLDN18, CLEC5A, CLEC6A, CLEC11A, CLEC14A, CLN3, CLU (clusterin), CMKLR1, CMKOR1 (RDC1), CNR1, C-MET, COL18A1, COLIA1, COL4A3, COL6A1, CR2, Cripto, CRP, CSF1 (M-CSF), CSF2 (GM-CSF), CSF3 (GCSF), CTAG1B (NY-ESO-1), CTLA4, CTL8, CTNNB1 (b-catenin), CTSB (cathepsin B), CX3CL1 (SCYD1), CX3CR1 (V28), CXCL1 (GRO1), CXCL1O (IP-IO), CXCLI1 (1-TAC/IP-9), CXCL12 (SDF1), CXCL13, CXCL14, CXCL16, CXCL2 (GRO2), CXCL3 (GRO3), CXCL5 (ENA-78/LIX), CXCL6 (GCP-2), CXCL9 (MIG), CXCR3 (GPR9/CKR-L2), CXCR4, CXCR6 (TYMSTR/STRL33/Bonzo), CYB5, CYC1, CYSLTR1, DAB2IP, DES, DKFZp451J0118, DLK1, DNCL1, DPP4, E2F1, Engel, Edge, Fennel, EFNA3, EFNB2, EGF, EGFR, ELAC2, ENG, Enola, ENO2, ENO3, EpCAM, EPHA1, EPHA2, EPHA3, EPHA4, EPHA5, EPHA6, EPHA7, EPHA8, EPHA9, EPHA10, EPHB1, EPHB2, EPHB3, EPHB4, EPHB5, EPHB6, EPHRIN-A1, EPHRIN-A2, EPHRINA3, EPHRIN-A4, EPHRIN-A5, EPHRIN-A6, EPHRIN-B1, EPHRIN-B2, EPHRIN-B3, EPHB4, EPG, ERBB2 (HER-2), ERBB3, ERBB4, EREG, ERK8, estrogen receptor, Earl, ESR2, F3 (TF), FADD, FAP, farnesyltransferase, FasL, FASNf, FCER1A, FCER2, FCGR3A, FGF, FGF1 (aFGF), FGF10, FGF1 1, FGF12, FGF12B, FGF13, FGF14, FGF16, FGF17, FGF18, FGF19, FGF2 (bFGF), FGF20, FGF21, FGF22, FGF23, FGF3 (int-2), FGF4 (HST), FGF5, FGF6 (HST-2), FGF7 (KGF), FGF8, FGF9, FGFR1, FGFR2, FGFR3, FGFR4, FIGF (VEGFD), FIL1(EPSILON), FBL1 (ZETA), FLJ12584, FLJ25530, FLRT1 (fibronectin), FLT1, FLT-3, FOLR1, FOS, FOSL1(FRA-1), FR-alpha, FY (DARC), GABRP (GABAa), GAGEB1, GAGEC1, GALNAC4S-6ST, GATA3, GD2, GD3, GDF5, GFI1, GFRA1, GGT1, GM-CSF, GNAS1, GNRH1, GPC1, GPC3, GPNB, GPR2 (CCR10), GPR31, GPR44, GPR81 (FKSG80), GRCC1O (C1O), GRP, GSN (Gelsolin), GSTP1, GUCY2C, HAVCR1, HAVCR2, HDAC, HDAC4, HDAC5, HDAC7A, HDAC9, Hedgehog, HER3, HGF, HIF1A, HIP1, histamine and histamine receptor, HLA-A, HLA-DR, HLA-DRA, HLA-E, HM74, HMOXI, HSP90, HUMCYT2A, ICEBERG, ICOSL, ID2, IFN-a, IFNA1, IFNA2, IFNA4, IFNA5, EFNA6, BFNA7, IFNB1, IFN gamma, IFNW1, IGBP1, IGF1, IGFIR, IGF2, IGFBP2, IGFBP3, IGFBP6, DL-1, ILIO, ILIORA, ILIORB, IL-1, IL1R1 (CD121a), IL1R2(CD121b), IL-IRA, IL-2, IL2RA (CD25), IL2RB(CD122), IL2RG(CD132), IL-4, IL-4R(CD123), IL-5, IL5RA(CD125), IL3RB(CD131), IL-6, IL6RA, (CD126), IR6RB(CD130), IL-7, IL7RA(CD127), IL-8, CXCR1 (IL8RA), CXCR2, (IL8RB/CD128), IL-9, IL9R(CD129), IL-10, IL10RA(CD210), IL10RB(CDW210B), IL-11, IL11RA, IL-12, IL-12A, IL-12B, IL-12RB1, IL-12RB2, IL-13, IL13RA1, IL13RA2, IL14, IL15, IL15RA, IL16, IL17, IL17A, IL17B, IL17C, IL17R, IL18, IL18BP, IL18R1, IL18RAP, IL19, ILIA, ILIB, ILIF10, ILIF5, IL1F6, ILIF7, IL1F8, DL1F9, ILIHYI, ILIR1, IL1R2, ILIRAP, ILIRAPLI, ILIRAPL2, ILIRL1, IL1RL2, ILIRN, IL2, IL20, IL20RA, IL21R, IL22, IL22R, IL22RA2, IL23, DL24, IL25, IL26, IL27, IL28A, IL28B, IL29, IL2RA, IL2RB, IL2RG, IL3, IL30, IL3RA, IL4, 1L4, IL6ST (glycoprotein 130), ILK, INHA, INHBA, INSL3, INSL4, IRAK1, IRAK2, ITGA1, ITGA2, ITGA3, ITGA6 (a6 integrin), ITGAV, ITGB3, ITGB4 (β4 integrin), JAG1, JAK1, JAK3, JTB, JUN, K6HF, KAI1, KDR, KIT, KITLG, KLF5 (GC Box BP), KLF6, KLK10, KLK12, KLK13, KLK14, KLK15, KLK3, KLK4, KLK5, KLK6, KLK9, KRT1, KRT19 (keratin 19), KRT2A, KRTHB6 (hair-specific type II keratin), L1CAM, LAG3, LAMA5, LAMP1, LEP (leptin), Lewis Y antigen ("LeY"), LILRB1, Lingo-p75, Lingo-Troy, LGALS3BP, LRRC15, LPS, LTA (TNF-b), LTB, LTB4R (GPR16), LTB4R2, LTBR, LY75, LYPD3, MACMARCKS, MAG or OMgp, MAGEA3, MAGEA6, MAP2K7 (c-Jun), MCP-1, MDK, MIB1, midkine, MIF, MISRII, MJP-2, MLSN, MK, MKI67 (Ki-67), MMP2, MMP9, MS4A1, MSMB, MT3 (metallothionectin-UI), mTOR, MTSS1, MUC1 (mucin), MUC16, MYC, MYD88, NCK2, NCR3LG1, neurocan, NFKBI, NFKB2, NGFB (NGF), NGFR, NgR-Lingo, NgRNogo66, (Nogo), NgR-p75, NgR-Troy, NMEI (NM23A), NOTCH, NOTCH1, NOTCH3, NOX5, NPPB, NROB1, NROB2, NRID1, NR1D2, NR1H2, NR1H3, NR1H4, NR112, NR113, NR2C1, NR2C2, NR2E1, NR2E3, NR2F1, NR2F2, NR2F6, NR3C1,

NR3C2, NR4A1, NR4A2, NR4A3, NR5A1, NR5A2, NR6A1, NRP1, NRP2, NT5E, NTN4, NY-ESO1, ODZI, OPRDI, P2RX7, PAP, PART1, PATE, PAWR, P- cadherin, PCA3, PCD1, PD-L1, PCDGF, PCNA, PDGFA, PDGFB, PDGFRA, PDGFRB, PECAMI, L1-CAM, peg-asparaginase, PF4 (CXCL4), PGF, PGR, phosphacan, PIAS2, PI3 kinase, PIK3CG, PLAU (uPA), PLG, PLXDCI, PKC, PKC-beta, PPBP (CXCL7), PPID, PR1, FRAME, PRKCQ, PRKD1, PRL, PROC, PROK2, PSAP, PSCA, PSMA, PTAFR, PTEN, PTHR2, PTGS2 (COX-2), PTN, PVRIG, RAC2 (P21Rac2), RANK, RANK ligand, RARB, RGS1, RGS13, RGS3, RNFI1O (ZNF144), Ron, ROBO2, ROR1, RXR, S100A2, SCGB 1D2 (lipophilin B), SCGB2A1 (mammaglobin 2), SCGB2A2 (mammaglobin 1), SCYE1 (endothelial monocyte-activation cytokine), SDF2, SERPENA1, SERPINA3, SERPINB5 (maspin), SERPINEI (PAI-I), SERPINFI, SHIP-1, SHIP-2, SHB1, SHB2, SHBG, SfcAZ, SLAMF7, SLC2A2, SLC33A1, SLC43A1, SLC44A4, SLC34A2, SLIT2, SPP1, SPRR1B (Spr1), ST6GAL1, ST8SIA1, STAB1, STATE, STEAP, STEAP2, TB4R2, TBX21, TCP1O, TDGF1, TEK, TGFA, TGFB1, TGFB1I1, TGFB2, TGFB3, TGFBI, TGFBR1, TGFBR2, TGFBR3, THIL, THBS1 (thrombospondin-1), THBS2, THBS4, THPO, TIE (Tie-1), TIMP3, tissue factor, TLR1, TLR2, TLR3, TLR4, TLR5, TLR6, TLR7, TLR8, TLR9, TLR10, TLR11, TNF, TNF-a, TNFAIP2 (B94), TNFAIP3, TNFRSFI1A, TNFRSF1A, TNFRSF1B, TNFRSF21, TNFRSF5, TNFRSF6 (Fas), TNFRSF7, TNFRSF8, TNFRSF9, TNFSF1O (TRAIL), TNFRSF10A, TNFRSF10B, TNFRSF12A, TNFRSF17, TNFSF1 1 (TRANCE), TNFSF12 (APO3L), TNFSF13 (April), TNFSF13B, TNFSF14 (HVEM-L), TNFRSF14 (HVEM), TNFSF15 (VEGI), TNFSF18, TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TOLLIP, Toll-like receptor, TOP2A (topoisomerase IIa), TP53, TPM1, TPM2, TRADD, TRAF1, TRAF2, TRAF3, TRAF4, TRAF5, TRAF6, TRKA, TREM1, TREM2, TROP2, TRPC6, TSLP, TWEAK, tyrosinase, uPAR, VEGF, VEGFB, VEGFC, versican, VHL C5, VLA-4, WT1, Wnt-1, XCL1 (lymphotactin), XCL2 (SCM-Ib), XCRI (GPR5/CCXCR1), YY1, ZFPM2, CLEC4C (BDCA-2, DLEC, CD303, CDH6, CLECSF7), CLEC4D (MCL, CLECSF8), CLEC4E (Mincle), CLEC6A (dectin-2), CLEC5A (MDL-1, CLECSF5), CLEC1B (CLEC-2), CLEC9A (DNGR-1), CLEC7A (dectin-1), CLEC11A, PDGFRa, SLAMF7, GP6 (GPVI), LILRA1 (CD85I), LILRA2 (CD85H, ILT1), LILRA4 (CD85G, ILT7), LILRA5 (CD85F, ILT11), LILRA6 (CD85b, ILT8), LILRB1, NCR1 (CD335, LY94, NKp46), NCR3 (CD335, LY94, NKp46), NCR3 (CD337, NKp30), OSCAR, TARM1, CD30, CD300C, CD300E, CD300LB (CD300B), CD300LD (CD300D), KIR2DL4 (CD158D), KIR2DS, KLRC2 (CD159C, NKG2C), KLRK1 (CD314, NKG2D), NCR2 (CD336, NKp44), PILRB, SIGLEC1 (CD169, SN), SIGLEC5, SIGLEC6, SIGLEC7, SIGLEC8, SIGLEC9, SIGLEC10, SIGLEC11, SIGLEC12, SIGLEC14, SIGLEC15 (CD33L3), SIGLEC16, SIRPA, SIRPB1 (CD172B), TREM1 (CD354), TREM2, KLRF1 (NKp80), 17-1A, SLAM7, MSLN, CTAG1B/NY-ESO-1, MAGEA3/A6, ATP5I (Q06185), OAT (P29758), AIFM1 (Q9Z0X1), AGFA (Q64133), MTDC (P18155), CMC1 (Q8BH59), PREP (Q8K411), YMEL1 (O88967), LPPRC (Q6PB66), LONM (Q8CGK3), ACON (Q99KI0), ODO1 (Q60597), IDHP (P54071), ALDH2 (P47738), ATPB (P56480), AATM (P05202), TMM93 (Q9CQW0), ERGI3 (Q9CQE7), RTN4 (Q99P72), CL041 (Q8BQR4), ERLN2 (Q8BFZ9), TERA (Q01853), DAD1 (P61804), CALX (P35564), CALU (O35887), VAPA (Q9WV55), MOGS (Q80UM7), GANAB (Q8BHN3), ERO1A (Q8R180), UGGG1 (Q6P5E4), P4HA1 (Q60715), HYEP (Q9D379), CALR (P14211), AT2A2 (055143), PDIA4 (P08003), PDIA1 (P09103), PDIA3 (P27773), PDIA6 (Q922R8), CLH (Q68FD5), PPIB (P24369), TCPG (P80318), MOT4 (P57787), NICA (P57716), BASI (P18572), VAPA (Q9WV55), ENV2 (P11370), VAT1 (Q62465), 4F2 (P10852), ENOA (P17182), ILK (O55222), GPNMB (Q99P91), ENV1 (P10404), ERO1A (Q8R180), CLH (Q68FD5), DSG1A (Q61495), AT1A1 (Q8VDN2), HYOU1 (Q9JKR6), TRAP1 (Q9CQN1), GRP75 (P38647), ENPL (P08113), CH60 (P63038), or CH10 (Q64433), but not limited thereto.

The target antigen may be an antigen distributed 10 times or more in cancer cells than in normal cells.

In particular, by utilizing a next-generation ADC platform focusing on the FL118 drug of Formula 1, which is a new payload that can overcome the shortcomings of existing ADCs, the present invention can provide a best-in-class ADC targeted to Trop-2 as a drug target, which is an antigen selectively overexpressed on the surface of solid cancer; and a first-in-class ADC targeted to EGFR as a drug target, which is a well-known cancer-related antigen for which an associated ADC has not been developed due to the issues in safety and efficacy.

[Antibody or antigen-binding site-containing fragment thereof]

According to the present invention, the immunoconjugate comprising [the FL118 drug of Formula 1]-[acid-sensitive linker]-[antibody or antigen-binding site-containing fragment thereof] can exhibit the organic action mechanism according to the combined use of [the FL118 drug of Formula 1]-[acid-sensitive linker] described above, regardless of the type, as long as the antibody or antigen-binding site-containing fragment thereof targets an antigen of a cancer cell. In particular, it is because free FL118 drug exhibits 5 to 20 times more powerful cytotoxicity than that of SN-38 in various cancer cell lines, as represented by a low numerical value of the $IC_{50}$ level, and the results of the evaluation performed with 140 cell lines originating from various cancers showed that FL118 drug shares very strong anticancer efficacy with an $IC_{50}$ of <100 nM against most cancer cells. In addition, the FL118 drug exhibited excellent efficacy in the xenograft models of various cancer cell lines compared to SN-38, and the safety has been secured through a GLP-toxicity test in rats and beagle dogs. Therefore, the FL118 drug shares the advantage that a therapeutic window can be secured also in an immunoconjugate using the drug as a payload. Furthermore, the FL118 drug utilizes an inhibitor of Topoisomerase

I, an anticancer mechanism of which efficacy/safety has been verified in clinical practice, and the excellent anticancer efficacy of Topoisomerase I inhibitors have been verified in clinical practice with various intractable solid cancers, such as colorectal cancer, lung cancer, breast cancer, and ovarian cancer; Furthermore, in Examples 11 and 12, the anticancer efficacy of the antibody-FL118 conjugate was verified by using antibodies targeting Her2, EGFR, and TROP2, respectively.

As used in the present Specification, the term "antibody" refers to a protein molecule serving as a ligand that specifically recognizes an antigen, including an immunoglobulin molecule having immunological reactivity with a specific antigen, and the term includes all of polyclonal antibody, monoclonal antibody, and whole antibody. In addition, the term also includes chimeric antibodies and bivalent or bispecific molecules, diabodies, triabodies and tetrabodies. The term further includes singlechain antibodies having a binding function to FcRn, scaps, derivatives of antibody constant regions, and artificial antibodies based on protein scaffolds. A whole antibody has a structure having two full-length light chains and two full-length heavy chains, and each light chain is connected to the heavy chain by a disulfide bond. The whole antibody includes IgA, IgD, IgE, IgM and IgG, and IgG is a subtype, including IgG1, IgG2, IgG3 and IgG4.

As used in the present Specification, the term "antigen-binding site-containing fragment" may be any fragment of an antibody that has antigen-binding activity of the antibody. Exemplary antibody fragments include single chain antibodies, Fd, Fab, Fab', $F(ab')_2$ , dsFv or scFv, but are not limited thereto. The Fd refers to the heavy chain portion included in the Fab fragment. The Fab has a structure having light chain and heavy chain variable regions and a first heavy chain constant region (CH1 domain) with one antigen-binding site. The Fab' differs from Fab in that the Fab' has a hinge region including at least one cysteine residue at the C terminal of the heavy chain CH1 domain. The $F(ab')_2$ antibody is generated as the cysteine residue in the hinge region forms a disulfide bond. The Fv (variable fragment) refers to a minimal antibody fragment having only a heavy chain variable region and a light chain variable region. In the double disulfide Fv (dsFv), a heavy chain variable region and a light chain variable region are linked by a disulfide bond, and in the single chain Fv (scFv), a heavy chain variable region and a light chain variable region are covalently linked generally through a peptide linker. These antibody fragments can be obtained by using a proteolytic enzyme (For example, a Fab can be obtained by the restriction digestion of a whole antibody with papain, and a $F(ab')_2$ fragment can be obtained by the restriction digestion of a whole antibody with pepsin), and preferably, it can be prepared through genetic recombination technology.

[Pharmaceutically acceptable salt]

In the present Specification, a pharmaceutically acceptable salt refers to a salt that is commonly used in pharmaceutical industry, and examples include salts of inorganic ions such as sodium, potassium, calcium, magnesium, lithium, copper, manganese, zinc, iron, etc., salts of inorganic acids such as hydrochloric acid, phosphoric acid, and sulfuric acid. Other examples include salts of organic acids such as ascorbic acid, citric acid, tartaric acid, lactic acid, maleic acid, malonic acid, fumaric acid, glycolic acid, succinic acid, propionic acid, acetic acid, orotate acid, and acetylsalicylic acid, and amino acid salts of lysine, arginine, guanidine, etc. Examples also include salts of organic ions such as tetramethyl ammonium, tetraethyl ammonium, tetrapropyl ammonium, tetrabutyl ammonium, benzyl trimethyl ammonium, and benzethonium, which can be used in pharmaceutical reactions and purification and separation processes. However, the types of salts in the present invention are not limited by these listed salts.

[Various carrier-drug conjugate]

The [FL118 drug of Formula 1]-[acid-sensitive linker] conjugate according to the present invention is applicable to various types of drug carriers other than antibodies, and the conjugate uses a carrier that can well penetrate deep into the cancer tissue and that can be easily used for CMC, unlike antibodies, and so it can be well utilized for various applications.

Therefore, the method for preparing the carrier-drug conjugate of the present invention is characterized in that the [FL118 drug of Formula 1]-[acid sensitive linker of Formula 2] conjugate or a pharmaceutically acceptable salt thereof according to the present invention is used and at least one of the drug of Formula 1 is linked to a carrier through a linker of Formula 2.

In this case, the carrier may be an antibody, a repebody, and/or an aptamer.

Descriptions of antibodies and non-limiting examples thereof are as described above.

Aptamers are DNA/RNA-based biopolymers and, like antibodies, have an antigen binding affinity of several nM levels, so they can be used as cancer tissue selective carriers for drugs. Repebody is a new class of immune protein discovered in fish and it can recognize antigens, like antibodies and nanobodies (single chain antibodies derived from mammals such as llama), and has an antigen binding affinity of several nM levels to antigens. Therefore, repebody can be used as a cancer tissue-selective carrier of drugs. In particular, since repebody has no immunogenicity, it can be used as a drug carrier, and at the same time, it can enhance the penetration into cancer cells due to the low molecular

weight. In addition, since it is easy to continuously produce a repebody of uniform quality, repebody is suitable for the preparation of a drug complex with enhanced penetration into cancer tissues.

[Roles of ADC as therapeutic antibody]

The ADC of the present invention can also play the role of a basic therapeutic antibody, which has been playing an important role in the treatment of diseases such as cancer and autoimmunity for the past 10 years.

Antibodies have the ability to differentiate between antigens and cells in our body, and so they have an excellent selection function to selectively act on antigens. Antibody therapeutics are therapeutics that utilize these antigen-selective binding properties of antibodies. Antibodies have a Y-shape, and antigen binding sites are present on each arm, and complementarity determining regions (CDRs) enable the antibody to selectively recognize a target antigen. In addition, there are five main types of antibodies (IgM, IgD, IgG, IgA, and IgE; Immunoglobulin (Ig)), and IgG is mainly used for antibody therapeutics. Antibody therapeutics can remove antigens while selectively responding to antigens. Various mechanisms are used for that purpose, and the characteristics of the representative mechanisms used in antibody therapeutics are described below. The first mechanism is the blocking function. An antibody selectively binds to the target receptor of the cell and blocks the physiological function of the target cell. Antibodies bind to ligands or receptors expressed on the cell surface, block the target signal transduction pathway, and thereby cause cell activity loss, proliferation inhibition, and apoptosis due to reduced signal transduction.

When an antibody binds to a receptor by targeting the receptor on the cell surface, the antibody changes the structure of the receptor or prevents the binding of factors that should bind to the receptor, thereby inhibiting intracellular signal transduction and, through this, controlling cell growth and differentiation. In addition, when an antibody binds to a receptor, the receptor can enter the cell by the intracellular influx mechanism, and the number of receptors on the cell surface decreases, thereby regulating the physiological mechanism of the cell. Trastuzumab (Herceptin®), which targets HER2-positive breast cancer, one of the human epidermal growth factor receptors (HER/EGFR/ERBB), is a representative antibody therapeutic that shows excellent effects through this mechanism.

The second mechanism is antibody-dependent cell-mediated cytotoxicity (ADCC) mechanism. Cancer cells have specific receptors or overexpressed receptors that are different from normal cells, and the immune system recognizes them and produces antibodies, which bind to the cell surface. When NK cells are activated after recognizing the Fc receptor (Fc$\gamma$RIII), apoptosis is induced.

The third mechanism is complement-dependent cytotoxicity (CDC), where an antibody binds to the surface of cancer cells and activates the complement protein (C1q), triggering a classical pathway to attack the cell membrane and thus creating pores and causing target cell lysis. In addition, there are mechanisms such as opsonization, neutralization, and inflammatory response. Therapeutic antibodies are generally made in an optimized form by re-engineering the antibody from various perspectives for a specific therapeutic purpose. The antibody engineering technology that used to be applied to the development of such therapeutic monoclonal antibodies has been extended and applied to the development of optimized ADCs. For example, most of the current therapeutic antibodies or ADCs in clinical development are $IgG_1$, and $IgG_2$ and $IgG_4$ are used in only a few cases. Antibody isotype selection is equally important for therapeutic antibodies and ADCs. The reason is that the antibody isotype affects the functions of antibody-dependent cellular cytotoxicity (ADCC), antibody-dependent cellular phagocytosis (ADCP) and complement-dependent cytotoxicity (CDC). In addition, $IgG_4$ becomes functionally monovalent by Fab arm exchange in vivo. In addition, the efficacy of the ADC by isotype and the Fc-domain engineering of the antibody may be applied to improve the in vivo efficacy of the ADC.

[Pharmaceutical composition for preventing or treating cancer]

The present invention provides a pharmaceutical composition for preventing or treating cancer, comprising the above-described immunoconjugate according to the present invention or a pharmaceutically acceptable salt thereof as an active ingredient. The immunoconjugate of the present invention specifically binds to the antigen of cancer cells and exhibits cytotoxicity by releasing drugs inside and outside the cancer cells, and thus can be usefully applied to the treatment or prevention of cancer. The anticancer activity of the immunoconjugate of the present invention is as described above.

In the present invention, the cancer includes all cancers that can be treated by the inhibition of topoisomerase I and/or by the inhibition of at least one cancer-associated survival gene selected from the group consisting of survivin, Mcl-1, XIAP, and cIAP2, and the cancer may be solid cancers or blood cancers. For examples, the cancer may be at least one selected from the group consisting of pseudomyxoma, intrahepatic biliary tract cancer, hepatoblastoma, liver cancer, thyroid cancer, colon cancer, testicular cancer, myelodysplastic syndrome, glioblastoma, oral cancer, labial cancer, mycosis fungoides, acute myeloid leukemia, acute lymphoblastic leukemia, basal cell cancer, ovarian epithelial cancer, ovarian germ cell cancer, male breast cancer, brain cancer, pituitary adenoma, multiple myeloma, gallbladder cancer, biliary tract cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, nasal sinus cancer, non-small cell lung cancer, tongue cancer, astrocytoma, small cell lung cancer, juvenile brain cancer, juvenile lymphoma, juvenile leukemia, small intestine cancer, meningioma, esophageal cancer, glioma, renal pelvic cancer, renal cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, cancer of unknown primary site, gastric lymphoma, gastric cancer, gastric carcinoma, gastrointestinal stromal cancer, Wilms cancer , breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoma, vaginal cancer, spinal cord cancer, acoustic schwannoma, pancreatic cancer, salivary gland cancer, Kaposi's sarcoma, Paget's disease, tonsil cancer, squamous cell carcinoma, lung adenocarcinoma, lung cancer, lung squamous cell carcinoma, skin cancer, anal cancer, rhabdomyosarcoma, laryngeal cancer, pleural cancer, blood cancer, and thymus cancer, but is not limited thereto. In addition, the cancer includes not only primary cancers but also metastatic cancers.

In addition, according to one embodiment of the present invention, there is provided a method for treating or preventing cancer, comprising administering a therapeutically effective amount of the immunoconjugate to a subject in need thereof. The subject may be a mammal including a human.

As used in the present invention, the term "therapeutically effective amount" refers to the amount of the immunoconjugate effective for the treatment or prevention of cancer. Specifically, "therapeutically effective amount" means an amount that is sufficient to treat a disease with a reasonable benefit/risk ratio applicable to medical treatment, and an effective dose level may be determined by factors including the subject type, severity, age, sex, type of disease, activity of the drug, sensitivity to the drug, duration of administration, route of administration, rate of excretion, duration of treatment, and concurrently used drugs, and other factors that are well known in the medical field. The pharmaceutical composition of the present invention may be administered as an individual therapeutic agent or may be administered in combination with other therapeutic agents, and may be administered sequentially or simultaneously with commercially available therapeutic agents. In addition, it may be administered by single administration or multiple administration. It is important to administer an amount that can obtain the maximum effect with a minimum amount without side effects in consideration of all of the factors mentioned above. Since the immunoconjugate of the present invention exhibits a dose-dependent effect, the dose may be easily determined by one of ordinary skill in the art according to various factors such as conditions, age, sex and complications of the patient. Since the active ingredient of the pharmaceutical composition of the present invention has excellent safety, it can be used even more than the determined dose.

In addition, according to one embodiment of the present invention, the present invention provides a use of the immunoconjugate for use in the preparation of a medicament to be used in the treatment or prevention of cancer. The immunoconjugate for the preparation of a medicament may be mixed with an acceptable adjuvant, diluent, carrier and so on, and may be prepared as a complex formulation together with other active agents to have a synergic action of the active ingredients.

Matters mentioned in the uses, compositions, and treatment methods of the present invention are equally applicable as long as they do not contradict with each other.

Mode for carrying out the invention.

Hereinafter, the present invention will be described in more details through Examples. However, the following Examples are only for clearly illustrating the technical features of the present invention, and do not limit the protection scope of the present invention.

Example 1: Preparation of FL118-linker 1 conjugate

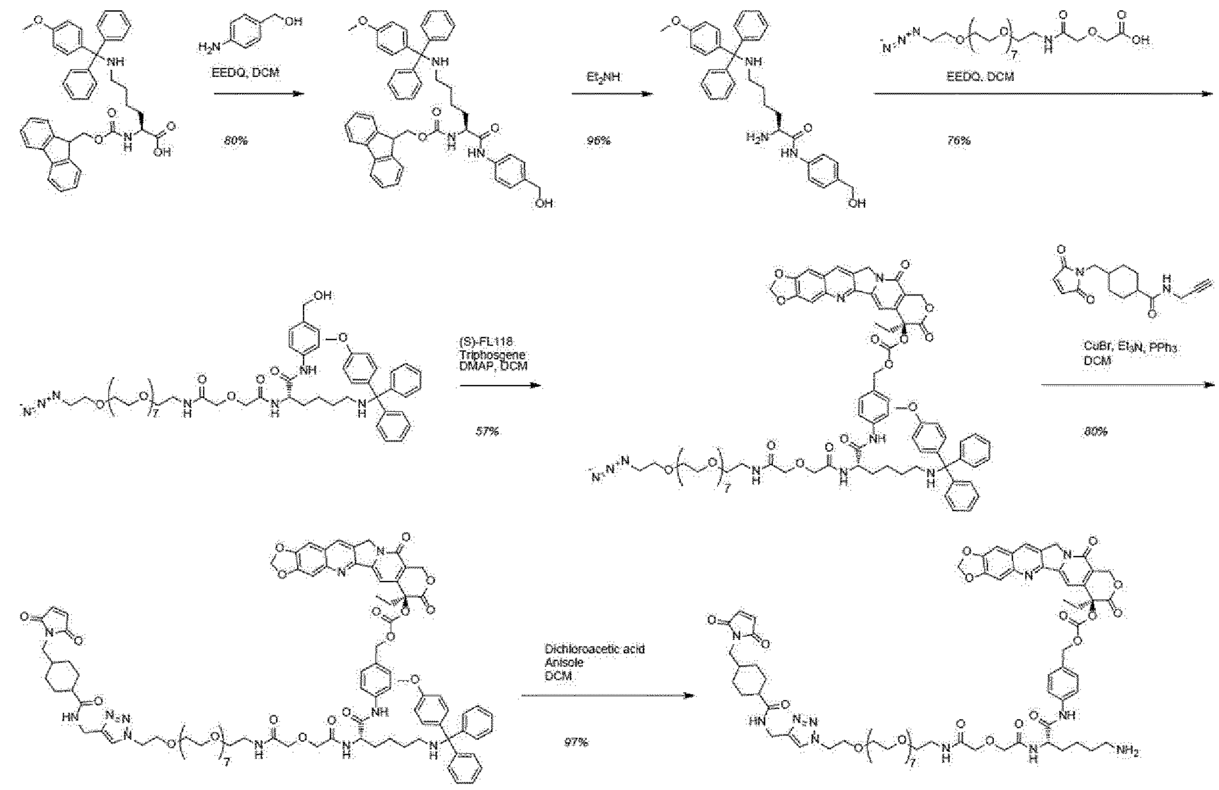


Step 1: Synthesis of (9H-fluoren-9-yl)methyl(S)-(1-((4-(hydroxy methyl)phenyl)amino)-6-(((4-methoxyphenyl)diphenylmethyl)amino)-1-oxohexan-2-yl)carbamate

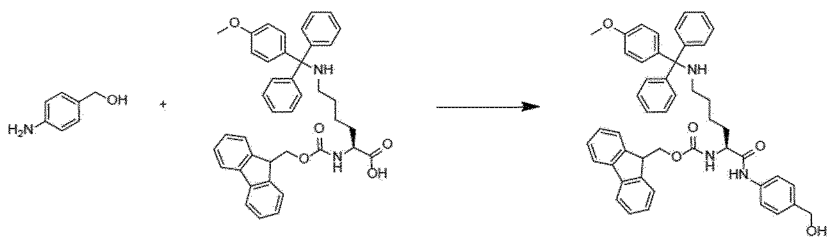

EEDQ (0.405 g, 1.623 mmol) was added to a suspension of 4-aminobenzyl alcohol (0.202 g, 1.639 mmol) and Fmoc-Lys(Mmt)-OH (1.00 g, 1.561 mmol) in dichloromethane (10 mL). The reaction mixture was stirred at room temperature for 4 hours. The reaction mixture was diluted with dichloromethane (10 mL) and washed with water (2×5 mL). The organic layer was washed with brine, dried over anhydrous sodium sulfate, and concentrated under reduced pressure to obtain a yellowish foam, which was purified by flash column chromatography (24 g Si, heptane / ethyl acetate 0 - 100 %, dichloromethane loading). The product fractions were combined and concentrated under reduced pressure to obtain a title compound (928 mg, 80 %) of a white foam.

LCMS (sc_basic): 522 $[M-Fmoc-H]^-$

Step 2: Synthesis of (S)-2-amino-N-(4-(hydroxymethyl)phenyl)-6-(((4-methoxyphenyl)diphenylmethyl)amino)hexanamide

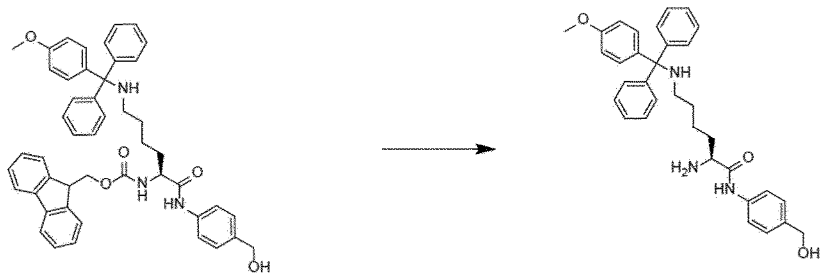

(9H-fluoren-9-yl)methyl(S)-(1-((4-(hydroxymethyl)phenyl)amino)-6-(((4-methoxyphenyl)diphenylmethyl)amino)-1-oxohexan-2-yl)carbamate (928 mg, 1.244 mmol) was dissolved in diethylamine (10 mL, 97 mmol) and the resulting mixture was stirred at room temperature for 2 hours. After 2 hours, the solvent was concentrated under reduced pressure and the sticky residue was washed twice with heptane. Then, the residue was dissolved in dichloromethane and precipitated with heptane. The solvent was drained off, and the procedure was repeated. As a result of evaporation, a product of a white foam was obtained (Yd: 677 mg, purity < 90%).

The product was dissolved in 5 mL dichloromethane and precipitated with 20 mL heptane, and the solvent was drained off. Through evaporation and drying, a 627 mg, 96 % title compound of a white foam was obtained.

LCMS (sc_acid): 522 $[M-H]^-$

Step 3: Synthesis of (S)-2-(32-azido-5-oxo-3,9,12,15,18,21,24,27,30-nonaoxa-6-azadotriacontanamido)-N-(4-(hydroxymethyl)phenyl)-6-(((4-methoxyphenyl)diphenylmethyl)amino)hexanamide

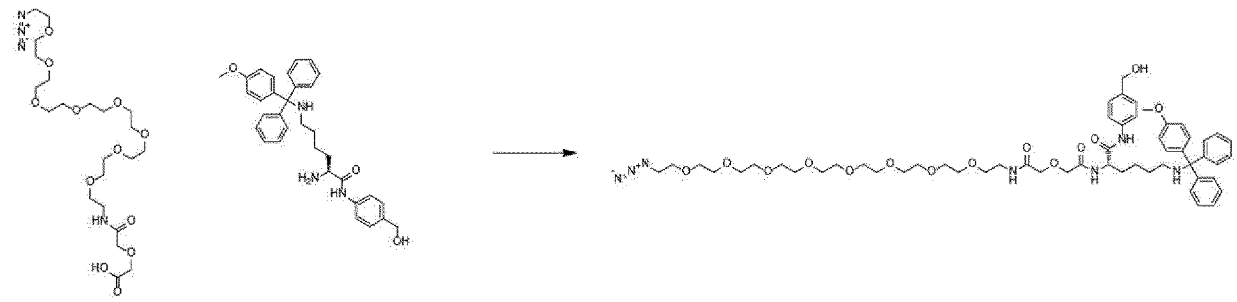

EEDQ (300 mg, 1.203 mmol) was added to a light yellow solution of O-(2-azidoethyl)-O'-(N-diglycolyl-2-aminoethyl)-heptaethyleneglycol (662 mg, 1.193 mmol) and (S)-2-amino-N-(4-(hydroxymethyl)phenyl)-6-(((4-methoxyphenyl)diphenylmethyl)amino)hexanamide (625 mg, 1.193 mmol) in dichloromethane (10 mL). The reaction mixture was stirred at room temperature for 3 hours. The reactants were concentrated under reduced pressure. The crude product (1.68 g) was purified by column chromatography (silica gel, ethyl acetate/methanol 0 - 10 %) to obtain a title compound as a dark colorless oil.

Yd: 1.02 g

LCMS: purity > 95 %. m/z 1082 M+Na, 1058 M-H

$^1$H NMR (d6-DMSO): Consistent with the structure. Containing residual ethyl acetate.

$^1$H NMR (400 MHz, DMSO) $\delta$ 10.06 (s, 1H), 8.15 (d, *J* = 8.0 Hz, 1H), 8.08 (t, *J* = 5.8 Hz, 1H), 7.58 - 7.49 (m, 2H), 7.37 (m, 4H), 7.25 (m, 8H), 7.15 (m, 2H), 6.86 - 6.76 (m, 2H), 5.11 (t, *J* = 5.7 Hz, 1H), 4.43 (m, 3H), 4.00 (s, 2H), 3.96 (d, *J* = 4.4 Hz, 2H), 3.70 (s, 3H), 3.59 (m, 2H), 3.57 - 3.44 (m, 30H), 3.40 (m, 5H), 3.25 (q, J = 6.1 Hz, 2H), 2.43 (m, 1H), 1.93 (m, 2H), 1.64 (m, 2H), 1.54 - 1.22 (m, 4H)

Colorless oil of 0.968 g was generated by further drying under vacuum.

Yield: 76 %. Used without further analysis.

Step 4: Synthesis of 4-((2,5-dioxo-2,5-dihydro-1H-pyrrol-1-yl)methyl)-N-(prop-2-yn-1-yl)cyclohexane-1-carboxamide

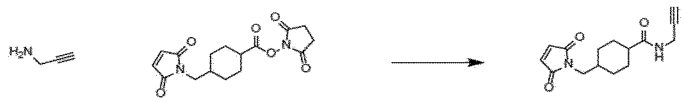

Succinimidyl 4-(N-maleimidomethyl)cyclohexanecarboxylate (1.01 g, 3.02 mmol) was dissolved in 20 mL of dichloromethane, and propargylamine (0.213 mL, 3.32 mmol) and N,N-diisopropyl Ethylamine (0.528 mL, 3.02 mmol) were added. The reaction mixture was stirred at room temperature for 1 hour. The reaction mixture was diluted with 100 mL dichloromethane, washed with 100 mL 1N HCl and then washed with 100 mL brine. The organic layer was dried over anhydrous sodium sulfate and evaporated under vacuum. The crude product (white solid, 950 mg) was purified through column chromatography (silica gel, 24 g, dichloromethane/methanol 0 - 2 %). The product fractions were combined and concentrated under vacuum to generate a white solid, which was then triturated in cold dichloromethane. Filtration was performed to obtain a title compound as a white solid. Yield: 577 mg, 73%.

LCMS (sc_acid): 275 $[M+H]^+$

$^1H$ NMR (400 MHz, DMSO) δ 8.47 (s, 1H), 8.16 (d, *J* = 7.9 Hz, 1H), 8.09 (t, *J* = 5.8 Hz, 1H), 7.58 (d, *J* = 8.4 Hz, 2H), 7.52 (d, *J* = 2.9 Hz, 2H), 7.41 - 7.20 (m, 12H), 7.14 (t, *J* = 7.3 Hz, 2H), 6.94 (s, 1H), 6.81 (d, *J* = 8.9 Hz, 2H), 6.28 (d, *J* = 2.0 Hz, 2H), 5.50 (s, 2H), 5.24 (s, 2H), 5.17 - 5.01 (m, 2H), 4.49 - 4.40 (m, 1H), 4.01 (s, 2H), 3.99 - 3.91 (m, 2H), 3.69 (s, 3H), 3.62 - 3.45 (m, 30H), 3.45 - 3.37 (m, 4H), 3.28 - 3.21 (m, 2H), 2.46 - 2.40 (m, 1H), 2.25 - 2.02 (m, 2H), 2.01 - 1.81 (m, 2H), 1.74 - 1.54 (m, 2H), 1.53 - 1.41 (m, 2H), 1.40 - 1.19 (m, 2H), 0.89 (t, *J* = 7.4 Hz, 3H).

Step 5: Synthesis of 4-((S)-35-azido-2-(4-(((4-methoxyphenyl)diphenylmethyl)amino)butyl)-4,8-dioxo-6,12,15,18,21,24,27,30,33-nonaoxa-3,9-diazapentatriacontanamidobenzyl ((S)-7-ethyl-8,11-dioxo-7,8,11,13-tetrahydro-10H-[1,3]dioxolo[4,5-g]pyrano[3',4':6,7]indolizino[1,2-b]quinolin-7-yl)carbonate

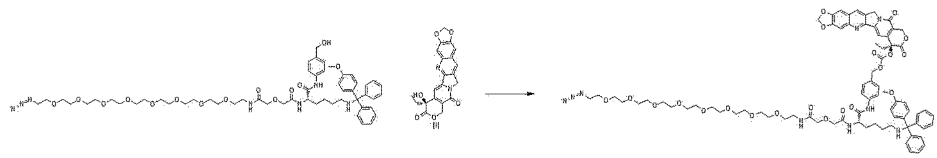

FL118 (50 mg, 0.127 mmol) and 4-dimethylaminopyridine (DAMP) (58.4 mg, 0.478 mmol) were dissolved in 8 mL of dichloromethane, and triphosgene (11.4 mg, 0.038 mmol) was dissolved in 2 mL of dichloromethane and then quickly added. The yellow reaction mixture was stirred for 30 minutes. After 1 hour, (S)-2-(32-azido-5-oxo-3,9,12,15,18,21,24,27,30-nonaoxa-6-zadotriacontanamido)-N-(4-(hydroxymethyl)phenyl)-6-(((4-methoxyphenyl)diphenylmethyl)amino)hexanamide (81 mg, 0.076 mmol) was dissolved in 2 mL of dichloromethane and added, and the resulting mixture was stirred for 30 minutes. Subsequently, the reaction mixture was concentrated under vacuum. The crude product was taken up in DMSO and purified by basic preparative HPLC. The product fractions were combined and freeze-dried to obtain 64 mg, 57% of a title compound of a yellow solid.

LCMS (sc_base): 1206 $[M-MMT+H]^+$

$^1H$ NMR (400 MHz, DMSO) δ 8.47 (s, 1H), 8.16 (d, *J* = 7.9 Hz, 1H), 8.09 (t, *J* = 5.8 Hz, 1H), 7.58 (d, *J* = 8.4 Hz, 2H), 7.52 (d, *J* = 2.9 Hz, 2H), 7.41 - 7.20 (m, 12H), 7.14 (t, *J* = 7.3 Hz, 2H), 6.94 (s, 1H), 6.81 (d, *J* = 8.9 Hz, 2H), 6.28 (d, *J* = 2.0 Hz, 2H), 5.50 (s, 2H), 5.24 (s, 2H), 5.17 - 5.01 (m, 2H), 4.49 - 4.40 (m, 1H), 4.01 (s, 2H), 3.99 - 3.91 (m, 2H), 3.69 (s, 3H), 3.62 - 3.45 (m, 30H), 3.45 - 3.37 (m, 4H), 3.28 - 3.21 (m, 2H), 2.46 - 2.40 (m, 1H), 2.25 - 2.02 (m, 2H), 2.01 - 1.81 (m, 2H), 1.74 - 1.54 (m, 2H), 1.53 - 1.41 (m, 2H), 1.40 - 1.19 (m, 2H), 0.89 (t, *J* = 7.4 Hz, 3H).

Step 6: Synthesis of 4-((S)-35-(4-((4-((2,5-dioxo-2,5-dihydro-1H-pyrrol-1-yl)methyl)cyclohexane-1-carboxamido)methyl)-1H-1,2,3-triazol-1-yl)-2-(4-(((4-methoxyphenyl)diphenylmethyl)amino)butyl)-4,8-dioxo-6,12,15,18,21,24,27,30,33-nonaoxa-3,9-diazapentatriacontanamido)benzyl ((S)-7-ethyl-8,11-dioxo-7,8,11,13-tetrahydro-10H-[1,3]dioxolo[4,5-g]pyrano[3',4':6,7]indolizino[1,2-b]quinolin-7-yl)carbonate

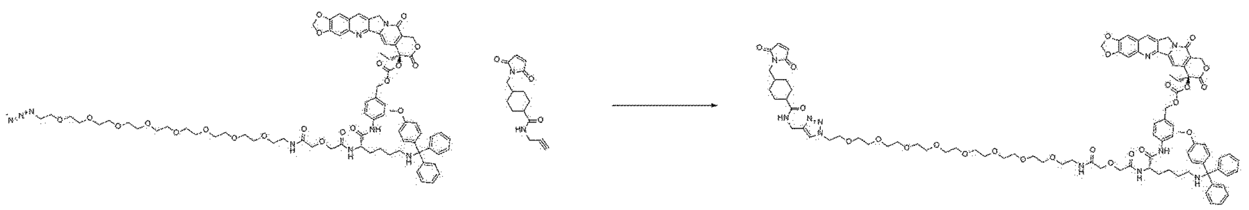

4-((2,5-dioxo-2,5-dihydro-1H-pyrrol-1-yl)methyl)-N-(prop-2-yn-1-yl)cyclohexane-1-carboxamide (25 mg, 91 μmol) and 4-((S)-35-azido-2-(4-(((4-methoxyphenyl)diphenylmethyl)amino)butyl)-4,8-dioxo-6,12,15,18,21,24,27,30,33-nonaoxa-3,9-diazapentatriacontanamido)benzyl ((S)-7-ethyl-8,11-dioxo-7,8,11,13-tetrahydro-10H-[1,3]dioxolo[4,5-g]pyrano[3',4':6,7]indolizino[1,2-b]quinolin-7-yl)carbonate (65 mg, 44.0 μmol), copper(I) bromide (2.5 mg, 17.43 μmol), and triphenylphosphine (2.3 mg, 8.77 μmol) were dissolved in dichloromethane (2.0 mL), and N,N-diisopropylmethylamine (0.023 mL, 132 μmol) was added.

A rapid change of the reaction mixture to a light yellow suspension was confirmed, and the suspension was stirred for 28 hours. Then, the reaction mixture was concentrated under vacuum. The crude product was taken up in DMSO and purified by baic preparative HPLC. The product fractions were combined and freeze-dried to obtain 62 mg, 80% of a title compound of a white solid.

LCMS (sc_base): 1480 [M-MMT+H]$^+$

Step 7: Synthesis of 4-((S)-2-(4-aminobutyl)-35-(4-((4-((2,5-dioxo-2,5-dihydro-1H-pyrrol-1-yl)methyl)cyclohexane-1-carboxamido)methyl)-1H-1,2,3-triazol-1-yl)-4,8-dioxo-6,12,15,18,21,24,27,30,33-nonaoxa-3,9-diazapentatriacontanamido)benzyl ((S)-7-ethyl-8,11-dioxo-7,8,11,13-tetrahydro-10H-[1,3]dioxolo[4,5-g]pyrano[3',4':6,7]indolizino[1,2-b]quinolin-7-yl)carbonate

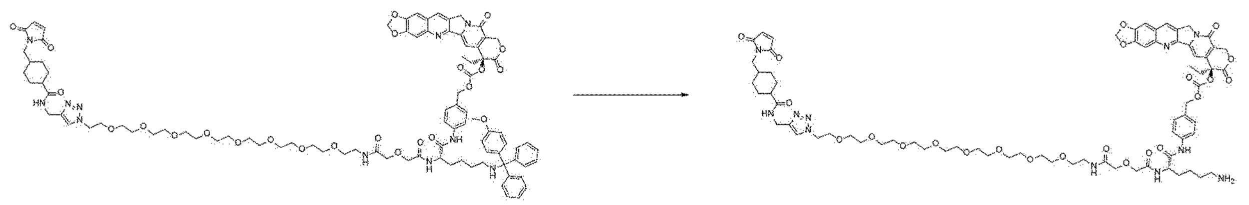

4-((S)-35-(4-((4-((2,5-dioxo-2,5-dihydro-1H-pyrrol-1-yl)methyl)cyclohexane-1-carboxamido)methyl)-1H-1,2,3-triazol-1-yl)-2-(4-(((4-methoxyphenyl)diphenylmethyl)amino)butyl)-4,8-dioxo-6,12,15,18,21,24,27,30,33-nonaoxa-3,9-diazapentatriacontanamido)benzyl ((S)-7-ethyl-8,11-dioxo-7,8,11,13-tetrahydro-10H-[1,3]dioxolo[4,5-g]pyrano[3',4':6,7]indolizino[1,2-b]quinolin-7-yl)carbonate (65 mg, 0.037 mmol) was dissolved in 0.7 mL dichloromethane. Anisole (15 ul, 0.137 mmol) and dichloroacetic acid (32 μl, 0.387 mmol) were added dropwise. The reaction mixture was stirred for 30 minutes. MTBE (5 mL) was added, and the resulting mixture was stirred vigorously. The solid was allowed to settle and the solvent was drained off. The residue was washed twice with 5% dichloromethane in MTBE and once with MTBE. The solid was then dried at room temperature under reduced pressure. A title compound (FL118-linker compound) of a pale yellow solid was obtained and stored at -20 °C.

Yd: 53 mg, 97 %

LCMS (an_acid): 1480 [M+H]$^+$

$^1$H NMR (400 MHz, DMSO) δ 10.18 (s, 1H), 8.48 (s, 1H), 8.24 - 8.17 (m, 2H), 8.09 (t, *J* = 5.7 Hz, 1H), 7.81 (s, 1H), 7.68 (s, 3H), 7.62 - 7.57 (m, 2H), 7.53 (s, 2H), 7.31 (d, *J* = 8.6 Hz, 2H), 7.01 (s, 2H), 6.94 (s, 1H), 6.29 (s, 2H), 6.01 (s, 1H), 5.50 (s, 2H), 5.25 (s, 2H), 5.17 - 5.01 (m, 2H), 4.47 (m, 3H), 4.25 (d, *J* = 5.6 Hz, 2H), 4.08 - 3.95 (m, 4H), 3.77 (t, *J* = 5.2 Hz, 2H), 3.54 - 3.41 (m, 34H), 3.28 - 3.20 (m, 4H), 2.78 (s, 2H), 2.18 - 1.99 (m, 3H), 1.83 - 1.67 (m, 4H), 1.65 - 1.45 (m, 6H), 1.42 - 1.19 (m, 5H), 0.94 - 0.84 (m, 5H).

Example 2: FL118-liker 2 conjugate

Step 1: Synthesis of tert-butyl ((4-(prop-2-yn-1-ylcarbamoyl)cyclohexyl)methyl)carbamate

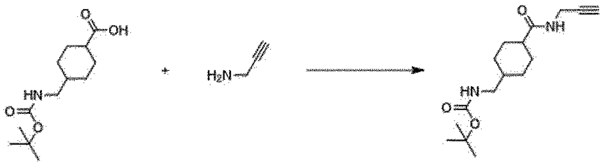

HATU (1.77 g, 4.66 mmol) was added to a solution of Boctranexamic acid (trans) (1.00 g, 3.89 mmol) in N,N-dimethylformamide (10 mL), and the resulting mixture was stirred for 10 minutes. Propargylamine (0.25 mL, 3.90 mmol) was added, and then diisopropylamine (0.71 mL, 4.07 mmol) was added. The mixture, which turned into a yellow solution, was stirred at room temperature overnight, and then most DMF was evaporated under reduced pressure. Subsequently, the mixture was diluted with ethyl acetate and water. The aqueous layer was separated, and the organic layer was washed with water and brine. Then, the organic layer was dried over sodium sulfate, and filtered and concentrated to obtain a beige solid (1.4 g).

The solid was softened in dichloromethane and then filtered to obtain a title compound (493 mg, 43 %) of an light gray solid. The filtrate was purified by column chromatography (silica gel, DCM/MeOH 0-5%) to obtain another batch of the title compound (315 mg, 27 %).

SC_ACID: 239 $[M\text{-}tBu+H]^+$

1H NMR (400 MHz, $CDCl_3$) δ 5.57 (s, 1H), 4.55 (s, 1H), 4.04 (dd, *J* = 5.2, 2.6 Hz, 2H), 2.98 (t, *J* = 6.5 Hz, 2H), 2.23 (t, *J* = 5.1 Hz, 1H), 2.09 - 1.99 (m, 1H), 1.98 - 1.89 (m, 2H), 1.89 - 1.80 (m, 2H), 1.52 - 1.39 (m, 12H), 1.02 - 0.90 (m, 2H).

Step 2: Synthesis of 4-(aminomethyl)-N-ethynylcyclohexane-1-carboxamide

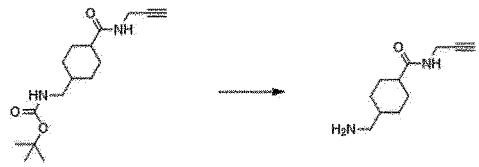

Tert-Butyl ((4-(prop-2-yn-1-ylcarbamoyl)cyclohexyl)methyl)carbamate (800 mg, 2.72 mmol) was suspended in 5 mL dioxane, and HCl (4 N in dioxane) (10 mL, 40.0 mmol) was added to obtain a clear solution. The reaction mixture was stirred at room temperature for 30 minutes, and rapid precipitation of a solid was confirmed. The reaction mixture was evaporated and dried, and then co-evaporated twice with DCM. The crude product was dissolved in 1 mL methanol and loaded into SCX-2 (2 × 5 g). The column was flushed with methanol to be neutral, and then the product was eluted with $NH_3$ (7 N) in methanol. The product fractions were evaporated and dried to obtain a title compound (520 mg, 98%) of a white solid.

SC_ACID: 195 $[M+H]^+$

$^1H$ NMR (400 MHz, $D_2O$) (crude product, HCl salt) δ 3.86 (d, *J* = 2.5 Hz, 2H), 2.79 (d, *J* = 7.1 Hz, 2H), 2.51 (t, *J* = 2.5 Hz, 1H), 2.22 - 2.11 (m, 1H), 1.87 - 1.74 (m, 4H), 1.65 - 1.54 (m, 1H), 1.42 - 1.28 (m, 2H), 1.07 - 0.94 (m, 2H).

**Step 3: Synthesis of 4-((3,4-dibromo-2,5-dioxo-2,5-dihydro-1H-pyrrol-1-yl)methyl)-N-(prop-2-yn-1-yl)cyclohexane-1-carboxamide**

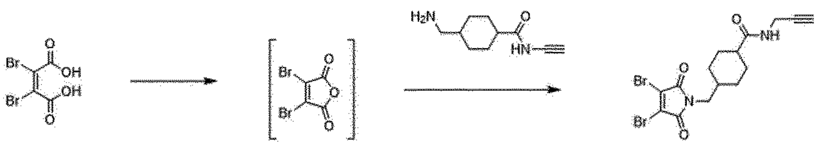

A solution of 2,3-dibromomaleic acid (200 mg, 0.730 mmol) in acetic acid (2.5 mL) was stirred under reflux for 45 minutes, and then 4-(aminomethyl)-N-ethynylcyclohexane-1-carboxamide (135 mg, 0.749 mmol) was added, and the resulting mixture was stirred for 2 hours. The reaction mixture was evaporated and dried, pulverized in dichloromethane, and filtered to obtain a slight amount of the product. The filtrate was purified by column chromatography (silica gel,

heptane/ethyl acetate 0-100%) to obtain another product in a slight amount. The two batches were combined to obtain a title compound (153 mg, 48%) of a yellow solid.

SC_ACID: 433 $[M+H]^+$, double Br pattern

$^1H$ NMR (400 MHz, DMSO) δ 8.14 (t, *J* = 5.5 Hz, 1H), 3.81 (dd, *J* = 5.5, 2.5 Hz, 2H), 3.29 (s, 2H), 3.05 (t, *J* = 2.5 Hz, 1H), 2.08 - 1.99 (m, 1H), 1.69 (d, *J* = 12.3 Hz, 4H), 1.52 (s, 1H), 1.32 - 1.20 (m, 2H), 0.96 - 0.83 (m, 2H).

Step 4: Synthesis of (S)-2-(32-azido-5-oxo-3,9,12,15,18,21,24,27,30-nonaoxa-6-azadotriacontanamido)-N-(4-(hydroxymethyl)phenyl)-6-(((4-methoxyphenyl)diphenylmethyl)amino)hexanamide

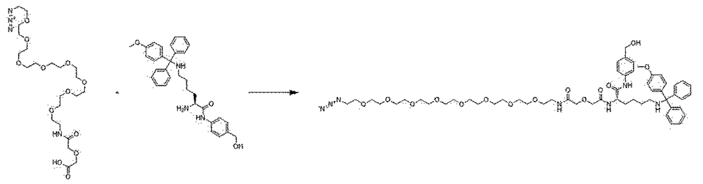

EEDQ (0.476 g, 1.910 mmol) was added to a light yellow solution of O-(2-azidoethyl)-O'-(N-diglycolyl-2-aminoethyl)-heptaethyleneglycol (1.06 g, 1.910 mmol) and (S)-2-amino-N-(4-(hydroxymethyl)phenyl)-6-(((4-methoxyphenyl)diphenylmethyl)amino)hexanamide (1.0 g, 1.910 mmol) in dichloromethane (10 mL). The reaction mixture was stirred at room temperature for 7.5 hours and concentrated under reduced pressure. The crude product (1.68 g) was purified by column chromatography (silica gel, ethyl acetate/methanol 0-10%) to obtain a dark colorless title compound (1.53 g, 76 % yield).

SC_ACID_M1200: 1082 $[M+Na]^+$, 1058 [M-H]~

Step 5: 4-((S)-35-azido-2-(4-(((4-methoxyphenyl)diphenylmethyl)amino)butyl)-4,8-dioxo-6,12,15,18,21,24,27,30,33-nonaoxa-3,9-diazapentatriacontanamido)benzyl ((S)-7-ethyl-8,11-dioxo-7,8,11,13-tetrahydro-10H-[1,3]dioxolo[4,5-g]pyrano[3',4':6,7]indolizino[1,2-b]quinoline-7-1)carbonate

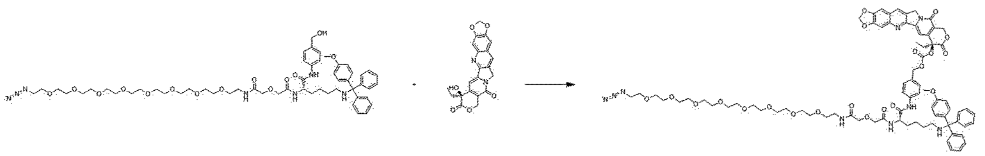

FL118 (60 mg, 0.153 mmol) and DMAP (70.1 mg, 0.573 mmol) were mixed in dry dichloromethane (8 mL). Triphosgene (20.4 mg, 0.069 mmol) dissolved in dry dichloromethane (1.75 mL) was added all at once, and then the brownish suspension was stirred at room temperature for 1 hour. (S)-2-(32-azido-5-oxo-3,9,12,15,18,21,24,27,30-nonaoxa-6-azadotriacontanamido)-N-(4-(hydroxymethyl)phenyl)-6-(((4-methoxyphenyl)diphenylmethyl)amino)hexanamide (162 mg, 0.153 mmol) in dry dichloromethane (1.75 mL) was added all at once. The reaction mixture was evaporated under vacuum, and the residue was dissoloved in DMSO, filtered, and purified by basic preparative MPLC. The product fractions were combined and freeze-dried to obtain a title compound of a white fluffy solid (81 mg, 35 %).

SC_BASE_M700-1900: 1206 $[M-MMT+H]^+$

Step 6: Synthesis of 4-((S)-35-(4-((4-((3,4-dibromo-2,5-dioxo-2,5-dihydro-1H-pyrrol-1-yl))methyl)cyclohexane-1-carboxamido)-methyl)-1H-1,2,3-triazol-1-yl)-2-(4-(((4-methoxyphenyl)diphenylmethyl)amino)butyl)-4,8-dioxo-6,12,15,18,21,24,27,30,33-nonaoxa-3,9-diazapentatriacontanamido)benzyl((S)-7-ethyl-8,11-dioxo-7,8,11,13-tetrahydro-10H-[1,3]dioxolo[4,5-g]pyrano[3',4':6,7]indolizino[1,2-b]quinolin-7-yl)carbamate

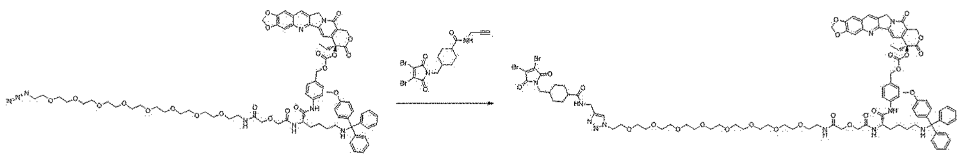

4-((3,4-dibromo-2,5-dioxo-2,5-dihydro-1H-pyrrol-1-yl)methyl)-N-(prop-2-yn-1-yl)cyclohexane-1-carboxamide (105 mg, 0.243 mmol), 4-((S)-35-azido-2-(4-(((4-methoxyphenyl)diphenylmethyl)amino)butyl)-4,8-dioxo-6,12,15,18,21,24,27,30,33-nonaoxa-3,9-diazapentatriacontanamido)benzyl ((S)-7-ethyl-8,11-dioxo-7,8,11,13-tetrahydro-10H-[1,3]dioxolo[4,5-g]pyrano[3',4':6,7]indolizino[1,2-b]quinolin-7-yl)carbonate (120 mg, 0.081 mmol), copper(I) bromide (4.7 mg, 0.032 mmol) and triphenylphosphine (4.3 mg, 0.016 mmol) were dissolved in 1.5 mL dichloromethane, and then diisopropylethylamine (0.043 mL, 0.243 mmol) was added. Rapid formation of a yellow suspension was confirmed. The reaction mixture was stirred at room temperature for 6 hours and purified by column chromatography (silica gel 12g, dichloromethane/methanol 0-10%) to obtain a title compound (94 mg, 60%) of a yellow solid.

SC_ACID: 820 $[M+2H-MMT]^{2+}/2$, doubly charged ion

1H NMR (400 MHz, DMSO) δ 10.12 (s, 1H), 8.47 (s, 1H), 8.22 - 8.01 (m, 4H), 7.81 (s, 1H), 7.57 (d, *J* = 8.4 Hz, 2H), 7.52 (d, *J* = 2.5 Hz, 2H), 7.37 (d, *J* = 7.9 Hz, 4H), 7.32 - 7.21 (m, 9H), 7.19 - 7.10 (m, 2H), 6.94 (s, 1H), 6.81 (d, *J* = 8.7 Hz, 2H), 6.28 (s, 2H), 5.49 (s, 2H), 5.24 (s, 2H), 5.16 - 5.01 (m, 2H), 4.47 (t, *J* = 5.2 Hz, 3H), 4.25 (d, *J* = 5.8 Hz, 2H), 4.07 - 3.94 (m, 7H), 3.77 (t, *J* = 5.3 Hz, 2H), 3.69 (s, 3H), 3.62 (s, 3H), 3.52 - 3.44 (m, 30H), 3.42 (t, *J* = 6.0 Hz, 3H), 3.26 - 3.22 (m, 3H), 3.17 - 3.11 (m, 2H), 2.18 - 2.04 (m, 3H), 1.96 - 1.88 (m, 2H), 1.73 - 1.64 (m, 5H), 1.51 - 1.44 (m, 2H), 0.90 (t, *J* = 7.3 Hz, 3H).

Step 7: Synthesis of FL118-linker 2 conjugate

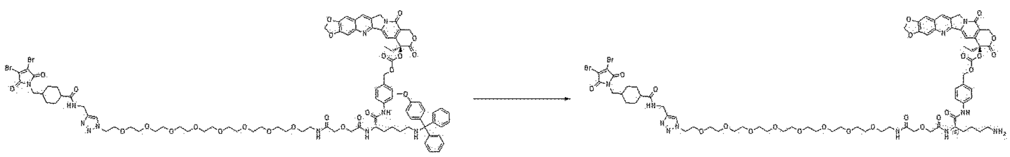

Anisole (0.064 mL, 0.590 mmol) and a dichloroacetic acid solution (0.049 mL, 0.590 mmol) in dichloromethane (2.5 mL) were added to 4-((S)-35-(4-((4-((3,4-dibromo-2,5-dioxo-2,5-dihydro-1H-pyrrol-1-yl)methyl)cyclohexane-1-carboxamido)-methyl)-1H-1,2,3-triazol-1-yl)-2-(4-(((4-methoxyphenyl)diphenylmethyl)amino)butyl)-4,8-dioxo-6,12,15,18,21,24,27,30,33-nonaoxa-3,9-diazapentatriacontanamido)benzyl ((S)-7-Ethyl-8,11-dioxo-7,8,11,13-tetrahydro-10H-[1,3]dioxolo[4,5-g]pyrano[3',4':6,7]indolizino[1,2-b]quinolin-7-yl)carbonate (94 mg, 0.049 mmol), and the yellow solution was stirred at room temperature for 45 minutes. Then, MTBE (8 mL) was added, and the resulting mixture was stirred until a light brown solid was precipitated. After the solvent was mostly removed, the solid was softened 5 times with 8 mL of MTBE and dried under reduced pressure to obtain a title compound (73 mg, 91 %) of a light yellow solid.

UPLC_AN_ACID: 820 $[M+2H]^{2+}/2$, doubly charged ion

SC_ACID_M700-2000: 1640 $[M+H]^{+}$, double Br pattern

1H NMR (400 MHz, DMSO) δ 10.17 (s, 1H), 8.48 (s, 1H), 8.25 - 8.16 (m, 2H), 8.09 (t, *J* = 5.9 Hz, 1H), 7.82 (s, 1H), 7.67 - 7.56 (m, 5H), 7.53 (s, 2H), 7.32 (d, *J* = 8.3 Hz, 2H), 6.94 (s, 1H), 6.29 (s, 2H), 5.50 (s, 2H), 5.25 (s, 2H), 5.17 - 5.02 (m, 2H), 4.51 - 4.44 (m, 3H), 4.25 (d, *J* = 5.6 Hz, 2H), 4.08 - 3.95 (m, 4H), 3.78 (t, *J* = 5.2 Hz, 2H), 3.54 - 3.40 (m, 30H), 3.28 (t, *J* = 6.0 Hz, 4H), 2.77 (d, *J* = 6.0 Hz, 2H), 2.21 - 2.01 (m, 3H), 1.81 - 1.63 (m, 6H), 1.60 - 1.46 (m, 3H), 1.41 - 1.21 (m, 6H), 0.89 (t, *J* = 7.3 Hz, 3H).

Example 3: Preparation of FL118-linker 3 conjugate

Step 1: Synthesis of (9H-fluoren-9-yl)methyl (S)-(1-((4-(hydroxymethyl)phenyl)amino)-6-(((4-methoxyphenyl)diphenylmethyl)amino)-1-oxohexan-2-yl)carbamate

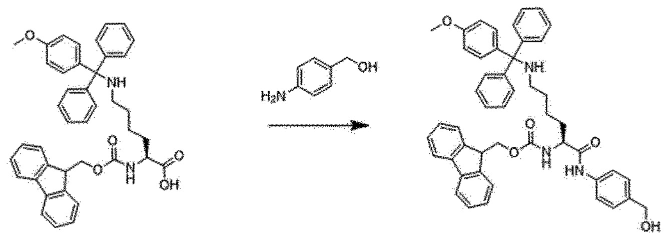

EEDQ (0.809 g, 3.25 mmol) was added to a solution of 4-aminobelzyl alcohol (0.404 g, 3.28 mmol) and Fmoc-Lys(Mmt)-OH (2g, 3.12 mmol) in dichloromethane (20 mL), and the reaction mixture was stirred at room temperature for 4 hours. The reaction mixture was diluted with dichloromethane (20 mL) and washed with water (20 mL). The organic layer was washed with brine, dried over $Na_2SO_4$ and concentrated under reduced pressure to obtain a yellowish foam. Purification was performed by flash column chromatography (40 g Si, heptane/EtOAc 0-100%) to obtain a title compound (1.77 g, 76 %) of a white foam.

It was confirmed that the analytical results were consistent with the data from the literature.

SC_ACID: 768 $[M+Na]^+$

Step 2: Synthesis of (S)-2-amino-N-(4-(hydroxymethyl)phenyl)-6-(((4-methoxyphenyl)diphenylmethyl)amino)hexanamide

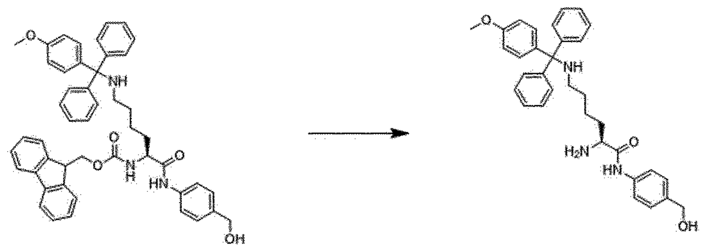

(9H-fluoren-9-yl)methyl (S)-(1-((4-(hydroxymethyl)phenyl)amino)-6-(((4-methoxyphenyl)diphenylmethyl)amino)-1-oxohexan-2-yl)carbamate (1.77 g, 2.373 mmol) was dissolved in diethylamine (19.5 mL, 189 mmol), and the resulting mixture was stirred at room temperature for one hour. Then, the solvent was evaporated under reduced pressure. The residue was dissolved in DCM (5 mL) and precipitated with heptane (25 mL), and then the solvent was poured. The procedure described above was repeated and then evaporation was performed to obtain a title compound of a white foam (1.3 g).

SC_ACID: 522 $[M-H]^-$

It was confirmed that the analytical results were consistent with the data from the literature.

Step 3: Synthesis of (S)-2-(32-azido-5-oxo-3,9,12,15,18,21,24,27,30-nonaoxa-6-azadotriacontanamido)-N-(4-(hydroxymethyl)phenyl)-6-(((4-methoxyphenyl)diphenylmethyl)amino)hexanamide

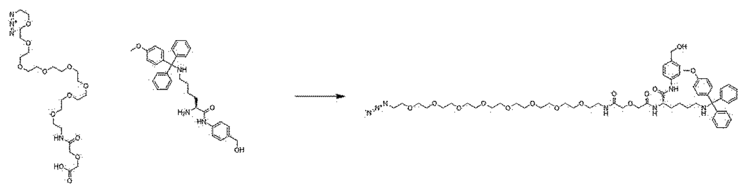

EEDQ (0.476 g, 1.910 mmol) was added to a light yellow solution of O-(2-azidoethyl)-O'-(N-diglycolyl-2-aminoethyl)-heptaethyleneglycol (1.059 g, 1.910 mmol) and (S)-2-amino-N-(4-(hydroxymethyl)phenyl)-6-(((4-methoxyphenyl)diphenylmethyl)amino)hexanamide (1.0 g, 1.910 mmol) in dichloromethane (10 mL), and the reaction mixture was stirred at room temperature for 7.5 hours and then concentrated under reduced pressure. Subsequently, the crude product was purified by column chromatography (silica gel, EtOAc/MeOH 0-10%) to obtain a title compound as a dark

colorless oil (1.53 g, 76 %).

SC_ACID_M1200: 1082 $[M+Na]^+$, 1058 $[M-H]^-$

Step 4: Synthesis of 4-((S)-35-azido-2-(4-(((4-methoxyphenyl)diphenylmethyl)amino)butyl)-4,8-dioxo-6,12,15,18,21,24,27,30,33-nonaoxa-3,9-diazapentatriacontanamido)benzyl ((S)-7-ethyl-8,11-dioxo-7,8,11,13-tetrahydro-10H-[1,3]dioxolo[4,5-g]pyrano[3',4':6,7]indolizino[1,2-b]quinoline-7-yl)carbonate

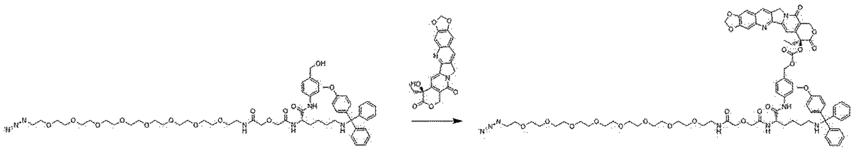

FL118 (70 mg, 0.178 mmol) and DMAP (82 mg, 0.669 mmol) were mixed in dry dichloromethane (10 mL), and then triphosgene dissolved in dry dichloromethane (2 mL) was added all at once. The brownish suspension was stirred for 1 hour. Almost complete conversion of FL118 to the corresponding methyl carbonate was confirmed by LCMS (sample in MeOH). Subsequently, (S)-2-(32-azido-5-oxo-3,9,12,15,18,21,24,27,30-nonaoxa-6-azadotriacontanamido)-N-(4-(hydroxymethyl)phenyl)-6-(((4-methoxyphenyl)diphenylmethyl)amino)hexanamide (189 mg, 0.178 mmol) in dry dichloromethane (2 mL) was added all at once. The reaction mixture was stirred for 30 minutes, and then evaporated under vacuum. The residue was taken up with DMSO, filtered, and purified by basic preparative MPLC to obtain a title compound (178 mg, 67 %) of a white solid.

SC_BASE_M1800: 1500 $[M+Na]^+$, 1206 $[M-MTT+H]^+$

Step 5: Synthesis of 4-((S)-35-amino-2-(4-(((4-methoxyphenyl)diphenylmethyl)amino)butyl)-4,8-dioxo-6,12,15,18,21,24,27,30,33-nonaoxa-3,9-diazapentatriacontanamido)benzyl ((S)-7-ethyl-8,11-dioxo-7,8,11,13-tetrahydro-10H-[1,3]dioxolo[4,5-g]pyrano[3',4':6,7]indolizino[1,2-b]quinolin-7-yl)carbonate

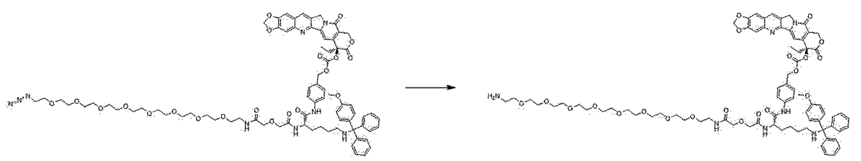

Water (2.25 mL) was added to a solution of 4-((S)-35-azido-2-(4-(((4-methoxyphenyl)diphenylmethyl)amino)butyl)-4,8-dioxo-6,12,15,18,21,24,27,30,33-nonaoxa-3,9-diazapentatriacontanamido)benzyl ((S)-7-ethyl-8,11-dioxo-7,8,11,13-tetrahydro-10H-[1,3]dioxolo[4,5-g]pyrano[3',4':6,7]indolizino[1,2-b]quinolin-7-yl)carbonate (177 mg, 0.120 mmol) in tetrahydrofuran (4.5 mL), and then a solution of trimethylphosphine (1.0 M) in THF (0.239 mL, 0.239 mmol) was added. The mixture was stirred at room temperature for 1 hour to obtain a yellow-pink solution. The mixture was dried, concentrated, and used immediately in the next step.

SC_BASE_M700-1900: 1452 $[M +H]^+$

Step 6: Synthesis of 3-(4,5-dibromo-2-methyl-3,6-dioxo-3,6-dihydropyridazin-1(2H)-yl)propanoic acid

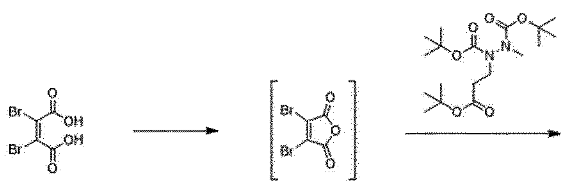

2,3-dibromomaleic acid (500 mg, 1.826 mmol) was dissolved in acetic acid (15 mL) and heated under reflux for 30 minutes. Di-tert-butyl 1-(3-(tert-butoxy)-3-oxopropyl)-2-methylhydrazine-1,2-dicarboxylate (615 mg, 1.643 mmol)

in acetic acid (2 mL) was added to the resulting solution, and the mixture was stirred under reflux for another 3 hours. Then, the mixture was cooled to room temperature and concentrated and co-evaporated with toluene (2x) and dichloromethane (2x) to obtain an orange-colored oil. Then, purification was performed by flash column chromatography (24 g; 50-100% EtOAc in n-heptane + 1% AcOH) to obtain a title compound (408 mg) as a light yellow oil.

SC_ACID: 357 $[M+H]^+$, 2 Br isotope pattern

1H NMR (400 MHz, DMSO) δ 12.48 (s, 1H), 4.27 (d, *J* = 7.4 Hz, 3H), 3.56 (s, 5H), 2.62 (t, *J* = 7.4 Hz, 3H).

Step 7: Synthesis of 4-((S)-39-(4,5-dibromo-2-methyl-3,6-dioxo-3,6-dihydropyridazin-1(2H)-yl)-2-(4-(((4-methoxyphenyl)diphenylmethyl)amino)butyl)-4,8,37-trioxo-6,12,15,18,21,24,27,30,33-nonaoxa-3,9,36-triazanonacontanamido)benzyl ((S)-7-ethyl-8,11-dioxo-7,8,11,13-tetrahydro-10H-[1, 3]dioxolo [4,5-g] pyrano [3 ’, 4’: 6, 7]indolizino[1,2-b]quinolin-7-yl)carbonate

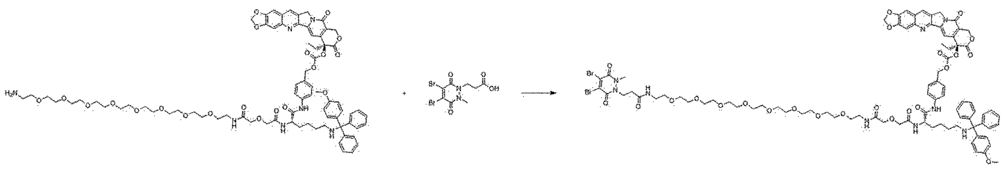

HATU (137 mg, 0.360 mmol) was added to a solution of 3-(4,5-dibromo-2-methyl-3,6-dioxo-3,6-dihydropyridazine-1(2H)-yl)propanoic acid (128 mg, 0.360 mmol) in peptide grade N,N-dimethylformamide (4 mL) was added, and 4-((S)-35-amino-2-(4-(((4-methoxyphenyl)diphenylmethyl)amino)butyl)-4,8-dioxo-6,12,15,18,21,24,27,30,33-nonaoxa-3,9-diazapentatriacontane amido)benzyl ((S)-7-ethyl-8,11-dioxo-7,8,11,13-tetrahydro-10H-[1,3]dioxolo[4,5-g]pyrano[3’,4’:6,7]indolizino[1,2-b]quinolin-7-yl)carbonate [crude] (174 mg, 0.120 mmol) was added to the resulting solution, and then the resulting mixture was stirred for 15 minutes. Then, DIPEA (0.063 mL, 0.360 mmol) was added, and the resulting mixture was stirred at room temperature for 1 hour.

The reaction mixture was directly purified by acidic preparative MPLC, and the product fractions were combined and freeze-dried to obtain a product of a white solid (120 mg). The analytical results identified that the product is a 4/1 mixture of the title compound and MMT-deprotected compound.

SC_ACID_M1000-1800: 1790 $[M+H]^+$, 2 Br-isotope pattern

Step 8: Synthesis of FL118-linker 3 conjugate

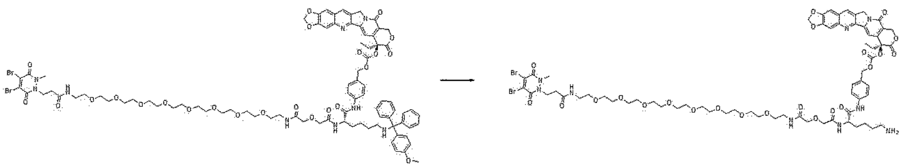

Anisole (0.028 mL, 0.257 mmol) was added to a solution of 4-((S)-39-(4,5-dibromo-2-methyl-3,6-dioxo-3,6-dihydropyridazine-1(2H)-yl)-2-(4-(((4-methoxyphenyl)diphenylmethyl)amino)butyl)-4,8,37-trioxo-6,12,15,18,21,24,27,30,33-nonaoxa-3,9,36-triazanonathriacontanamido)benzyl ((S)-7-ethyl-8,11-dioxo-7,8,11,13-tetrahydro-10H-[1,3]dioxolo[4,5-g]pyrano[3’,4’:6,7]indolizino[1,2-b]quinolin-7-yl)carbonate (115 mg, 0.064 mmol) in dichloromethane (3 mL), and then dichloroacetic acid (0.064 mL, 0.771 mmol) was added. After confirming that the reaction mixture turned into bright orange, the reaction mixture was stirred at room temperature for 45 minutes. MTBE (1.5 mL) was added to the reaction mixture, and then the suspension was stirred vigorously for 2-3 minutes. After stopping the stirring, formation of a precipitate on the bottom of the vial was confirmed. After removing the solvent with a pipette, MTBE (10 mL) was added, and the softening process was repeated 6 times. The residual suspension was dried at 40 °C under reduced pressure to obtain a title compound (90 mg, 92 %) of a white solid.

UPLC_AN_ACID_M1600: 1518 $[M+H]^+$

1H NMR (400 MHz, DMSO) δ 10.18 (s, 1H), 8.48 (s, 1H), 8.21 (d, *J* = 8.0 Hz, 1H), 8.10 (dd, *J* = 10. 0, 5. 0 Hz,

2H), 7.81 - 7.56 (m, 5H), 7.53 (s, 2H), 7.32 (d, *J* = 8.4 Hz, 2H), 6.94 (s, 1H), 6.29 (s, 2H), 5.82 (s, 1H), 5.50 (s, 2H), 5.25 (s, 2H), 5.19 - 5.01 (m, 2H), 4.47 (q, *J* = 7.9 Hz, 1H), 4.26 (t, *J* = 7.0 Hz, 2H), 4.11 - 3.91 (m, 4H), 3.54 (s, 3H), 3.51 - 3.40 (m, 30H), 3.30 - 3.24 (m, 2H), 3.15 (q, *J* = 5.8 Hz, 2H), 2.83 - 2.72 (m, 2H), 2.44 (t, *J* = 7.0 Hz, 2H), 2.22 - 2.08 (m, 2H), 1.83 - 1.62 (m, 2H), 1.61 - 1.48 (m, 2H), 1.44 - 1.20 (m, 3H), 0.89 (t, *J* = 7.4 Hz, 3H).

Example 4: Preparation of FL118-linker 4 conjugate

Step 1: (9H-fluoren-9-yl)methyl (S)-(1-((4-(hydroxymethyl)phenyl)amino)-6-(((4-methoxyphenyl)diphenylmethyl)amino)-1-oxohexan-2-yl)carbamate

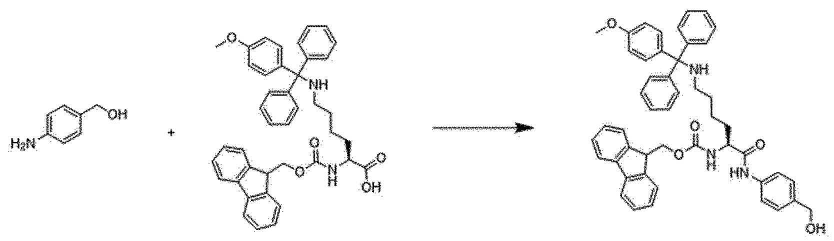

EEDQ (0.809 g, 3.25 mmol) was added to a solution of 4-aminobenzyl alcohol (0.404 g, 3.28 mmol) and Fmoc-Lys(Mmt)-OH (2 g, 3.12 mmol) in dichloromethane (20 mL), and the resulting mixture was stirred overnight. The reaction mixture was diluted with dichloromethane (40 mL) and washed with water (40 mL). The organic layer was dried over $Na_2SO_4$ and concentrated under reduced pressure to obtain a yellowish foam. Purification was performed by flash column chromatography (40 g silica, Hept/EtOAc 0-100%) to obtain a title compound (1.54 g, 66 %) of a white foam.

SC_ACID: 273 $[MMT]^+$, 522 $[M\text{-}Fmoc\text{-}H]^-$

Step 2: Synthesis of (9H-fluoren-9-yl)methyl ((S)-1-((4-((((((S)-7-ethyl-8,11-dioxo-7,8,11,13-tetrahydro-10H-[1,3]dioxolo[4,5-g]pyrano[3',4':6,7]indolizino[1,2-b]quinolin-7-yl)oxy)carbonyl)oxy)methyl)phenyl)amino)-6-(((4-methoxyphenyl)diphenylmethyl)amino)-1-oxohexan-2-yl)carbamate

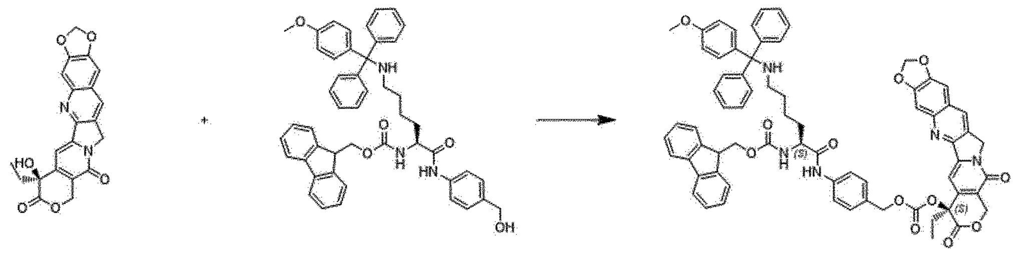

FL118 (50 mg, 0.127 mmol) and DMAP (58.4 mg, 0.478 mmol) were mixed with dry dichloromethane (7 mL), and dry dichloromethane (1.5 mL) in which triphosgene (17.02 mg, 0.057 mmol) was dissolved was added all at once. After that, the brown suspension was stirred for 30 minutes. Then, (9H-fluoren-9-yl)methyl (S)-(1-((4-(hydroxymethyl)phenyl)amino)-6-(((4-methoxyphenyl)diphenylmethyl)amino)-1-oxohexan-2-yl)carbamate (95 mg, 0.127 mmol) was added all at once to slowly obtain a brown solution. The reaction mixture was evaporated and dried to obtain a brown/gray foam, which was then purified by column chromatography (12 g; 0-5% methanol in dichloromethane) to obtain a title compound (107 mg, 72 %) of an orange/brown foam.

SC_ACID: 892 $[M\text{-}MMT+H]^+$

1H NMR (400 MHz, $CDCl_3$) δ 8.07 (s, 1H), 7.86 (s, 1H), 7.71 (d, *J* = 7.6 Hz, 2H), 7.56 - 7.27 (m, 16H), 7.25 - 7.11 (m, 10H), 7.09 (s, 1H), 6.78 (d, *J* = 8.8 Hz, 2H), 6.12 (s, 2H), 5.68 (d, *J* = 17.1 Hz, 1H), 5.37 (d, *J* = 17.0 Hz, 1H), 5.28 - 5.04 (m, 5H), 4.46 (d, *J* = 6.6 Hz, 2H), 4.15 (s, 2H), 3.75 (s, 3H), 3.48 - 3.38 (m, 1H), 2.32 - 2.20 (m, 1H), 2.19 - 2.08 (m, 3H), 1.50 (s, 3H), 1.40 - 1.28 (m, 2H), 0.98 (t, *J* = 7.5 Hz, 3H).

Step 3: Synthesis of 4-((S)-2-amino-6-(((4-methoxyphenyl)diphenylmethyl)amino)hexanamido)benzyl ((S)-7-ethyl-8,11-di oxo-7,8,11,13-tetrahydro-10H-[1,3]dioxolo[4,5-g]pyrano[3',4':6,7]indolizino[1,2- b] quinolin-7-yl)carbonate

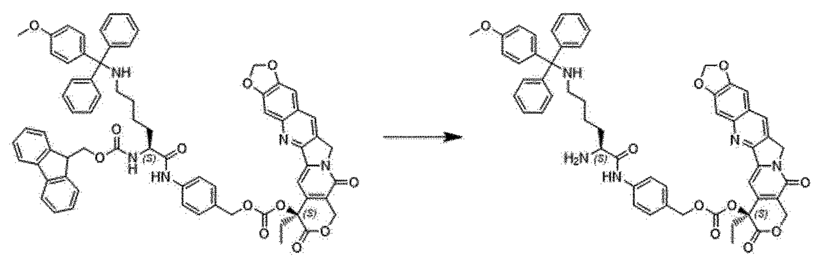

(9H-fluoren-9-yl)methyl ((S)-1-((4-((((((S)-7-ethyl-8,11-dioxo-7,8,11,13- tetrahydro-10H-[1,3]dioxolo[4,5-g]pyrano[3',4':6,7]indolizino[1,2-b]quinolin-7-yl)oxy) carbonyl)oxy)methyl)phenyl)amino)-6-(((4-methoxyphenyl)diphenylmethyl)amino)-1-oxohexan-2-yl)carbamate (162 mg, 0.139 mmol) was dissolved in dry dichloromethane (2.5 mL), and diethylamine (1.3 mL, 12.44 mmol) was added. The resulting mixture was stirred at room temperature for 2 hours, and formation of a suspension was confirmed. The reaction mixture was evaporated and dried to obtain a title compound (154 mg) of a yellow/beige solid, which was used in the next step.

SC_ACID: 964 {M+Na]$^+$, 670 [M-MMT+H]$^+$

Step 4: Synthesis of DBCO-PEG-acid

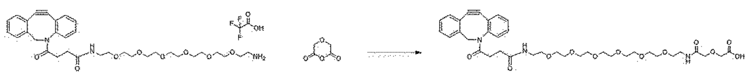

A solution of DBCO-PEG6-amine TFA salt (196 mg, 0.270 mmol) and diisopropylethylamine (0.708 mL, 4.05 mmol) in dichloromethane (2.5 mL) was stirred at room temperature for 10 minutes. Then, diglycolic anhydride (62.7 mg, 0.540 mmol) was added, and the reaction mixture was stirred at room temperature overnight. Then, water (0.125 mL) was added, and the resulting mixture was stirred at room temperature for 4 hours. The mixture was diluted with dichloromethane (2 mL) to obtain a clear solution, which was dried over sodium sulfate, filtered, and concentrated to obtain a yellow turbid oil (340 mg) containing a title compound and residual DIPEA. The yellow turbid oil was used in the next step.

SC_ACID: 728 [M+H]$^+$

Step 5: Synthesis of dibenzocyclooctyne-PEG-Lys(MMT)-PABC-FL118

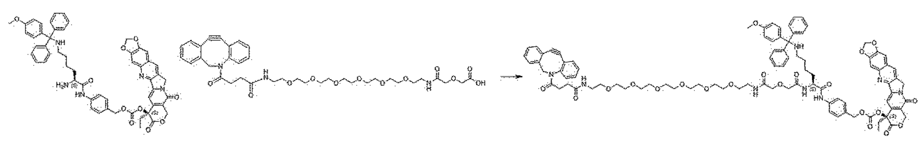

HATU (111 mg, 0.292 mmol) was added to DBCO-PEG-acid (296 mg, 0.244 mmol) in dichloromethane (10 mL), and the mixture was stirred at room temperature for 20 minutes. 4-((S)-2-amino-6-(((4-methoxyphenyl)diphenylmethyl)amino)hexanamido)benzyl ((S)-7-ethyl-8,11-dioxo-7,8,11,13-tetrahydro-10H-[1,3]dioxolo[4,5-g]pyrano[3',4':6,7]indolizino[1,2-b]quinoline -7-yl)carbonate (270 mg, 0.244 mmol) was added, and the mixture was stirred at room temperature. The solution was purified by flash column chromatography (silica 12 g; 0-10% methanol in dichloromethane), and the 11-13 fractions were combined and concentrated to obtain a compound (139 mg) of an off-white solid, which was then purified by basic preparative MPLC to obtain a title compound (86 mg, 21 %) of a white solid.

SC_BASE_M700-2000: 1652 [M+H]$^+$

1H NMR (400 MHz, DMSO) δ 10.15 (s, 1H), 8.47 (s, 1H), 8.15 (d, *J* = 8.0 Hz, 1H), 8.08 (t, *J* = 5.8 Hz, 1H), 7.76 (t, *J* = 5.6 Hz, 1H), 7.70 - 7.65 (m, 1H), 7.64 - 7.49 (m, 6H), 7.49 - 7.40 (m, 3H), 7.39 - 7.10 (m, 20H), 6.93 (s, 1H), 6.81 (d, *J* = 8.9 Hz, 2H), 6.28 (d, *J* = 2.1 Hz, 2H), 5.50 (s, 2H), 5.23 (s, 2H), 5.15 - 4.98 (m, 3H), 4.48 - 4.40 (m, 1H), 4.03 - 3.93 (m, 4H), 3.69 (s, 3H), 3.50 - 3.37 (m, 23H), 3.30 - 3.20 (m, 4H), 3.12 - 3.03 (m, 2H), 2.62 - 2.54 (m, 1H), 2.45 - 2.40 (m, 1H), 2.27 - 2.09 (m, 3H), 2.02 - 1.89 (m, 3H), 1.79 - 1.57 (m, 3H), 1.51 - 1.44 (m, 2H), 1.37 - 1.22 (m, 3H), 0.89 (t, *J* = 7.4 Hz, 3H).

Step 6: Synthesis of dibenzocyclooctyne-FEG-Lys-PABC-FL118 (FL118-linker 4 conjugate)

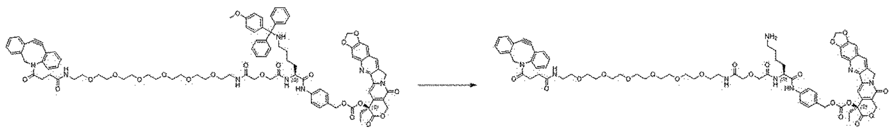

The compound obtained in Step 5 above was dissolved in dichloromethane (0.5 mL) and dried over $Na_2SO_4$ overnight. The solution was then transferred to a reaction tube and evaporated and dried. Dichloromethane (4 mL) in which anisole (0.052 mL, 0.472 mmol) and dichloroacetic acid (0.039 mL, 0.472 mmol) was dissolved was added, and the yellow reaction mixture was stirred at room temperature for 30 minutes. Tert-butyl methyl ether (15 mL) was added to form a suspension, but since the product did not sufficiently precipitate, the solvent was evaporated at room temperature under reduced pressure. Tert-butyl methyl ether (5 mL) was added to obtain a suspension, which was stirred at 0 °C and then the solvent was removed with a pipette. Softening was repeated 4 times with MTBE (5 mL) to obtain a title compound (56 mg) of a light yellow solid.

AN_BASE_M700-2000: 1379 $[M+H]^+$

1H NMR (400 MHz, DMSO) δ 10.19 (s, 1H), 8.48 (s, 1H), 8.21 (d, *J* = 8.1 Hz, 1H), 8.10 (t, *J* = 5.8 Hz, 1H), 7.80 - 7.64 (m, 4H), 7.64 - 7.56 (m, 3H), 7.56 - 7.16 (m, 11H), 6.94 (s, 1H), 6.29 (s, 2H), 6.14 (s, 1H), 5.50 (s, 2H), 5.24 (s, 2H), 5.19 - 4.98 (m, 3H), 4.53 - 4.43 (m, 1H), 4.10 - 3.93 (m, 4H), 3.53 - 3.40 (m, 23H), 3.30 - 3.27 (m, 4H), 2.77 (s, 2H), 2.63 - 2.54 (m, 1H), 2.28 - 2.08 (m, 3H), 2.04 - 1.93 (m, 1H), 1.80 - 1.66 (m, 2H), 1.61 - 1.49 (m, 2H), 1.44 - 1.27 (m, 2H), 0.89 (t, *J* = 7.4 Hz, 3H).

Example 5: Preparation of anti-HER2 antibody-linker 1-FL118 immunoconjugate (DAR 8)

Anti-HER2 antibody (trastuzumab) in the original buffer (20 mM histidine, 150 mM NaCl, pH 6.0) was reduced with 5 mM TCEP, and the reaction vial was placed in an incubator shaker at 22 °C at a rotation speed of 60 rpm for 6 hours. After reduction, TCEP was removed through a spin desalting column (40 K, 2 mL).

A solution of DMSO and FL118-linker 1 compound (10 mg/mL stock in DMSO, 12 eq. for antibody) was added to the reduced antibody solution of which final DMSO concentration was 10%. The reaction mixture was appropriately mixed, and the reaction vial was left at 4°C for 2 hours.

After conjugation, the reaction mixture was placed in an exchange buffer with 18 mM MES (pH 6.5) through a spin desalting column (40 K, 5 mL). The product was then sterile filtered through a 0.2 um PVDF disposable filter. The resulting immunoconjugate was characterized, 0.07 mM PS80 and 20 mM trehalose dehydrate were added, and the produced immunoconjugate was freeze-dried.

The DAR of the immunoconjugate of Example 5 was determined by using HIC and MS, and it was confirmed that the average MS-DAR value was 7.14 and the HIC-DAR value was 8.00 (Table 1 and FIG. 5b).

[Table 1]

| | scale | aggrega te (%) | recover Y (%) | D0 (%) | MS-DAR | HIC-DAR |
|---|---|---|---|---|---|---|
| **Example 5** | 8 mg | 3.44 | 73 | 0.00 | 7.14 | 8.00 |

Example 6: Preparation of anti-HER2 antibody-linker 1-FL118 conjugate (DAR 4)

Anti-HER2 antibody (trastuzumab) in the original buffer (20 mM histidine, 150 mM NaCl, pH 6.0) was reduced with 5 mM TCEP and the reaction vial was placed in an incubator shaker at 22 °C at a rotation speed of 60 rpm for 3 hours. After reduction, TCEP was removed through a spin desalting column (40 K, 2 mL).

A solution of DMSO and FL118-linker compound (10 mg/mL stock in DMSO, 10 eq. for antibody) was added to the reduced antibody solution of which final DMSO concentration was 10%. The reaction mixture was appropriately mixed, and the reaction vial was left at 4°C for 2 hours.

After conjugation, the reaction mixture was placed in an exchange buffer with 18 mM MES (pH 6.5) through a spin desalting column (40 K, 5 mL). The product was then sterile filtered through a 0.2 um PVDF disposable filter. Then, the resulting immunoconjugate was characterized, 0.07 mM PS80 and 20 mM trehalose dehydrate were added, and the produced immunoconjugate was freeze-dried.

The DAR of the immunoconjugate of Example 6 was determined by using HIC and MS, and it was confirmed that the average MS-DAR value was 4.02 (Table 2 and FIG. 5a).

[Table 2]

| | scale | aggrega te (%) | recover y (%) | D0 (%) | MS-DAR | HIC-DAR |
|---|---|---|---|---|---|---|
| **Example 6** | 8 mg | 2.24 | 68 | 7.21 | 4.02 | NA |

Example 7: Preparation of anti-HER2 antibody-linker 2-FL118 conjugate and anti-HER2 antibody-linker 3-FL118 conjugate

To reduce the anti-HER2 antibody (trastuzumab) with 5 mM TCEP in the original buffer (20 mM histidine, 150 mM NaCl, pH 6.0), the reaction vial was placed in a constant-temperature water bath set at 25 °C for 3 hours. After reduction, TCEP was removed through a spin desalting column (100 K, 0.5 mL).

DMSO and either a solution of FL118-Linker 2 compound or a solution of FL118-Linker 3 compound (5 mM stock in DMSO, 20 eq. for antibody, respectively) were added to the reduced antibody solution of which final DMSO concentration was 10%. The reaction mixture was appropriately mixed, and the reaction vial was placed in a constant-temperature water bath set at 25 °C for 4 hours. To terminate the reaction, a cysteine solution (10 mM stock in distilled water, 40 eq. for antibody) was added to the reaction solution, and the resulting mixture was then left for 30 minutes under the same conditions.

The reaction was purified through an SEC column under phosphate buffer conditions, and SDS PAGE, SEC, and HIC were used to confirm that the immunoconjugates of Example 7 were prepared.

Example 8: Preparation of anti-EGFR antibody-linker 1-FL118 conjugate (DAR 8)

Anti-EGFR antibody (cetuximab) in the original buffer (20 mM histidine, 150 mM NaCl, pH 6.0) was reduced with 5 mM TCEP and the reaction vial was placed in an incubator shaker at 22°C at a rotation speed of 60 rpm for 3 hours. After reduction, TCEP was removed through a spin desalting column (40 K, 2 mL × 2). DMSO and a solution of FL118-linker compound (10 mg/mL stock of DMSO, 12 eq. for antibody) were added to the reduced antibody solution of which final DMSO concentration was 10%. The reaction mixture was appropriately mixed, and the reaction vial was left at 4 °C for 2 hours.

After conjugation, the reaction mixture was placed in an exchange buffer with 18 mM MES (pH 6.5) through a spin desalting column (40 K, 10 mL). The product was then sterile filtered through a 0.2 um PVDF disposable filter. The resulting immunoconjugate was characterized, 0.07 mM PS80 and 20 mM trehalose dehydrate were added, and the produced immunoconjugate was freeze-dried.

The DAR of the immunoconjugate of Example 8 was determined by using HIC and MS, and it was confirmed that the average MS-DAR value was 8.00 (Table 3).

[Table 3]

| | scale | aggrega te (%) | recover y (%) | D0 (%) | MS-DAR | HIC-DAR |
|---|---|---|---|---|---|---|
| **Example 8** | 9 mg | 0.95 | 60 | 0.88 | 8.00 | NA |

Example 9: Preparation of anti-Trop-2 antibody-linker 1-FL118 conjugate (DAR 8)

An anti-Trop-2 antibody-linker 1-FL118 conjugate (DAR 8) was prepared by a method similar to Example 8, except that an anti-Trop-2 antibody (sacituzumab) was used instead of an anti-EGFR antibody (cetuximab) (Table 4).

[Table 4]

| Abs | 3 days | | 6 days | |
|---|---|---|---|---|
| | $IC_{50}$ (nM) | Max Cytotoxicit y (%) | $IC_{50}$ (nM) | Max Cytotoxicit y (%) |
| Trastuzumab -linker 1-FL118 (DAR8) | 31.32 | 88.6 | 0.35 | 99.5 |
| DS8201 | NA | 27.2 | 433.60 | 77.2 |

Example 10: Preparation of animal models and materials used to evaluate in vitro cytotoxicity and in vivo drug efficacy All procedures related to animal handling, care, and treatment in this study were carried out in accordance with the guidelines approved by the Institutional Animal Care and Use Committee (IACUC) of Shanghai Model Organisms Center, Inc. according to the guidelines of the Association for Assessment and Accreditation of Laboratory Animal Care (AAALAC). Thirty 9 to 11 weeks old female mice weighing 16 to 18 g were accommodated in cages (300 mm × 180 mm × 150 mm), 3 to 5 mice in each cage, and cared at constant temperature (18 to 26 °C) and humidity (40 to 70%). The mice were provided with free access to radiation-sterilized dry granular feed and sterile drinking water in individual ventilated cages.

The immunoconjugates of Example 5 (molecular weight 159 kDa, purity 97%), Example 6 (molecular weight 154 kDa, purity 98%), Example 8 (molecular weight 160 kDa, purity 99%) and Example 9 (molecular weight 160 kDa, purity 99.6%) were used as freeze-dried powder each stored at -80 °C.

Trastuzumab (molecular weight 148 kDa, purity 97%) supplied from BCD as a solution stored at -80 °C was dissolved in a buffer (20 mM Histidine, 150 mM NaCl, pH 6.0) (concentration 10.09 mg/mL) and then used as a control. Cetuximab (molecular weight 148 kDa, purity 100%) was used after being dissolved in a buffer (20 mM Histidine, 150 mM NaCl, pH 6.0) (concentration 10.47 mg/mL), and sacituzumab (molecular weight 148 kDa, purity 100%) was used after being dissolved in a buffer.

DS8201 (molecular weight 156 kDa, purity 99%) supplied from BCD in a solution state stored at -80°C was dissolved in a buffer (25 mM Histidine, pH 5.5) (concentration 2.87 mg/mL) and then used as a control. DS8201 is an immunoconjugate of trastuzumab and deruxtecan, and the immunoconjugates of Examples 5 and 6 and the anti-HER2 targeting antibody are the same as trastuzumab. Deruxtecan, which is a cytotoxic drug (payload), is a topoisomerase I inhibitor, and has similarities with the FL118 of Examples in terms of structure and function. However, DS8201 is different from the immunoconjugates of Examples 5 and 6 in the structure and properties of the linker because DS8201 uses a cleavable tetrapeptide-based linker. DS8201 has the characteristic that it is internalized and then cleaved to release the drug, and the released drug is known to exhibit cytotoxicity even to the surrounding cancer cells regardless of target expression due to its high cell membrane permeability (Cancer Sci 2016; 107: 1039-1046). In addition, an excellent effect was recently reported in a phase 2 clinical trial performed with patients having HER2-positive metastatic breast cancer (N Engl J Med 2020; 382: 610-621). In addition, DS8201 was designated by the US FDA as a breakthrough therapy for HER2-positive recurrent and metastatic breast cancer and for HER2-positive recurrent and metastatic stomach cancer in 2017 and 2020, respectively.

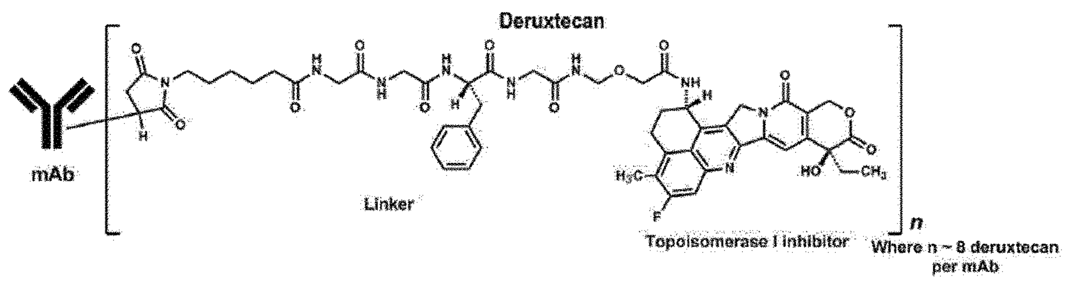


Control DS8201 (*Enhertu*)

Example 11: Evaluation of in vitro cytotoxicity

For the immunoconjugates of Examples 5, 6, 8 and 9, and DS8201 as a control, a cytotoxicity analysis was performed with the HER2-overexpression JIMT-1 breast cancer cell line, the EGFR-overexpression MDA-MB-468 breast cancer cell line or the MDA-MB-468 cell line by a process described below. Immunoconjugate-mediated cytotoxicity to JIMT-1 cells or MDA-MB-468 cells was determined by cell viability measurement. On Day 0, $6\times10^3$ JIMT-1 cells per well (for 3-days culture) or $3\times10^3$ JIMT-1 cells per well (for 6-days culture) were plated in a 96-well plate (Greinier, #

655090) in which 50 μL DMEM (Gibco, # 11995-065) containing 10% FBS (HyClone, SV30087.03) was dispensed. In the case of MDA-MB-468 cells, on Day 0, $6\times10^3$ cells per well (for 3-days culture) or $3\times10^3$ cells per well (for 6-days culture) were plated in a 96-well plate (Greinier, # 655090) in which 50 μL RPMI (Gibco, #11875-093) containing 10% FBS (HyClone, SV30087.03) was dispensed. On the next day, the immunoconjugate samples were serially diluted in 3 folds (2000, 666.667, 222.222, 74.074, 24.6914, 8.2305, 2.7435, 0.9145, 0.3048, 0.1016 and 0 nM) and then each added to the plated cells at 50 μL/well. The cells were cultured for 3 or 6 days in an incubator at 37 °C and 5% $CO_2$.

On Day 4 or Day 7, cell viability was detected by using CellTiter-Glo (Promega, # G7573) according to the manufacturer's instructions. After the plate was equilibrated to room temperature for approximately 15 minutes, the CellTiter-Glo solution was added at 50 μL/well. The plate was gently shaken for about 1 minute and then incubated at room temperature for 10 minutes. After that, luminescence was read by using Envision (PerkinElmer).

The cytotoxic effect obtained from each immunoconjugate was calculated by comparing the luminescence value obtained from the control well (cell only) according to the formula below.

$$\text{Cytotoxicity \%} = 100 \times (V_{\text{cell only}} - V_{\text{sample}}) / V_{\text{cell only}}$$

The GraphPad Prism 6.0 software program was used to perform a four-parameter nonlinear regression analysis to obtain the $IC_{50}$ values for cytotoxicity.

Results

When JIMT-1 cells were cultured for 3 days or 6 days by adding the immunoconjugate of Example 5, a cytotoxic effect was found, and apoptosis was induced in both cases. Surprisingly, the immunoconjugate of Example 5 showed a significantly superior cytotoxic effect compared to DS8201 (positive control). When the culture was performed for 3 days, DS8201 showed only 27.2% cytotoxicity, whereas the immunoconjugate of Example 5 exhibited 88.6% cytotoxicity, showing a three-fold or more excellent effect. In addition, when the culture was performed for 6 days, it was confirmed that the immunoconjugate of Example 5 had a 100-fold or more superior effect in terms of the $IC_{50}$ value compared to the positive control DS8201 (FIG. 6).

When JIMT-1 cells were cultured for 3 days or 6 days by adding the immunoconjugate of Example 6, a cytotoxic effect was also found, and apoptosis was induced in both cases. As in Example 5, the immunoconjugate of Example 6 also showed a significantly superior cytotoxic effect compared to DS8201 (positive control) (FIG. 7).

In addition, when JIMT-1 cells were cultured for 3 days or 6 days by adding the immunoconjugate of Example 8, a cytotoxic effect was also found, and apoptosis was induced in both cases. In other words, as in Examples 5 and 6, it was confirmed that the immunoconjugate of Example 8 also showed a considerably high cytotoxic effect (FIG. 9).

In addition, when MDA-MB-468 cells were cultured for 3 days or 6 days by adding the immunoconjugate of Example 9, a cytotoxic effect was also found, and apoptosis was induced in both cases. In other words, as in Examples 5, 6 and 8, it was confirmed that the immunoconjugate of Example 9 also showed a considerably high cytotoxic effect (FIG. 11).

Example 12: Evaluation of *in vivo* drug efficacy

12-1. Cell culture

The JIMT-1 breast cancer cells were cultured under 5 % $CO_2$ and 37 °C conditions with DMEM containing 10% FBS, 100 U/mL penicillin, and 100 ug/mL streptomycin, and a subculture was performed by a routine method twice per week by trypsin-EDTA treatment. Cells growing in the exponential growth phase were harvested and counted for tumor inoculation. The MDA-MB-468 breast cancer cells were cultured under 5 % $CO_2$ and 37 °C conditions with RPMI containing 10% FBS, 100 U/mL penicillin, and 100 ug/mL streptomycin, and a subculture was performed by a routine method twice per week by trypsin-EDTA treatment. Cells growing in the exponential growth phase were harvested and counted for tumor inoculation.

12-2. Tumor inoculation

JIMT-1 tumor cells ($1\times10^7$) or MDA-MB-468 cells ($1\times10^7$) in 0.2 mL DPBS with Matrigel were inoculated subcutaneously into the right anterior flank of each mouse. The animals were randomly grouped (Tables 5-7) when the mean tumor volume reached about 215 $mm^3$, and then treatment for the drug efficacy study was initiated.

[Table 5]

| Abs | 3 days | | 6 days | |
|---|---|---|---|---|
| | $IC_{50}$ (nM) | Max Cytotoxicit y (%) | $IC_{50}$ (nM) | Max Cytotoxicit y (%) |
| Trastuzumab-linker 1-FL118 (DAR4) | 100.4 | 90 | 0.39 | 99.6 |
| DS8201 | 447.4 | 40 | 335.9 | 77.9 |

[Table 6]

| Abs | 3 days | | 6 days | |
|---|---|---|---|---|
| | $IC_{50}$ (nM) | Max Cytotoxicit y (%) | $IC_{50}$ (nM) | Max Cytotoxicit y (%) |
| Cetuximab-linker 1-FL118 (DAR8) | 0.4546 | 97 | NA | 100 |

[Table 7]

| **JIMT-1** | **Treatment** | **Dose (mg/kg)** | **Administra tion route** | **Number of animals (n)** |
|---|---|---|---|---|
| Group 1 (negative control) | Saline solution | - | I.V. | 3 |
| Group 2 (positive control) | Trastuzumab | 10 | I.V. | 3 |
| Group 3 | Immunoconjuga te of Example 5 | 1 | I.V. | 3 |
| Group 4 | Immunoconjuga te of Example 5 | 5 | I.V. | 3 |
| Group 5 | Immunoconjuga te of Example 5 | 10 | I.V. | 3 |

12-3. Observations

During routine monitoring, the animals were checked daily for normal behavior such as mobility, food and water consumption (observation only), weight change (weighed twice a week), eye/hair matting, tumor growth, and treatment effects. Mortality and observed clinical signs were recorded based on the number of animals in each subset.

12-4. Tumor measurement and endpoints

The main endpoint was determined as the time point when it could be confirmed if tumor growth could de delayed or if the mouse could be treated. The two-dimensional size of the tumor was measured twice a week by using a caliper, and the volume was recorded in the unit of $mm^3$ by using the formula ($V = 0.5\ a \times b^2$, where a and b are the major and minor diameters of the tumor, respectively).

The tumor growth inhibition (TGI) was calculated for each group.

$$TGI\ (\%) = [1 - (T_i / T_0) / (V_i / V_0)] \times 100$$

($T_i$ is the mean tumor volume of the treatment group on a given day; $T_0$ is the mean tumor volume of the treatment group on the first treatment day; $V_i$ is the mean tumor volume of the vehicle control group on the same day as $T_i$; and $V_0$ is the mean tumor volume of the vehicle control group on first treatment day.) Animals with a weight loss of 15% or greater or a tumor volume equal to or greater than 3000 $mm^3$ were euthanized at a humane endpoint.

12-5. Statistical analysis

The results are expressed as mean and standard error (mean ± SEM). The data was analyzed by performing a two-way RM ANOVA Dunnett's multiple comparison test by using the Graphpad Prism 6.0 software program, and $p < 0.05$ was considered statistically significant.

12-6. Body weight of JIMT-1 or MDA-MB-468 tumor model

The body weight of the mice of the JIMT-1 or MDA-MB-468 tumor model was regularly monitored. As shown in Tables 8-10 and FIGS. 8, 10 and 12, no clear weight loss was observed in any of the treatment groups.

[Table 8]

| MDA-MB-468 | Treatment | Dose (mg/kg) | Administra tion route | Number of animals (n) |
|---|---|---|---|---|
| Group 1 (negative control) | Saline solution | - | I.V. | 3 |
| Group 2 (positive control) | Cetuximab | 10 | I.V. | 3 |
| Group 3 | Immunoconjuga te of Example 8 | 1 | I.V. | 3 |
| Group 4 | Immunoconjuga te of Example 8 | 5 | I.V. | 3 |
| Group 5 | Immunoconjuga te of Example 8 | 10 | I.V. | 3 |

[Table 9]

| | Body weight (g) (Day 0) | Body weight (g) (Day 21) | Change of body weight (g) |
|---|---|---|---|
| Group 1 | 16.97 ± 0.37 | 18.94 ± 0.50 | + 1.97 |
| Group 2 | 17.49 ± 0.67 | 19.05 ± 0.84 | + 1.56 |
| Group 3 | 16.97 ± 0.12 | 17.62 ± 0.45 | + 0.65 |
| Group 4 | 16.89 ± 0.12 | 16.65 ± 0.41 | - 0.24 |
| Group 5 | 17.16 ± 0.50 | 16.73 ± 0.52 | - 0.43 |

[Table 10]

| | Body weight (g) (Day 0) | Body weight (g) (Day 21) | Change of body weight (g) |
|---|---|---|---|
| Group 1 | 16.29 ± 0.37 | 16.93 ± 0.33 | + 0.64 |
| Group 2 | 16.52 ± 0.35 | 17.42 ± 0.61 | + 0.9 |
| Group 3 | 16.81 ± 0.41 | 17.90 ± 0.06 | + 1.09 |
| Group 4 | 17.04 ± 0.27 | 18.23 ± 0.22 | + 1.19 |
| Group 5 | 16.31 ± 0.29 | 16.84 ± 0.39 | + 0.53 |

Unlike humans, mouse plasma has a high level of esterase activity and can easily cleave acid-sensitive bonds such as ester or carbonate functional groups, and so it was predicted that side effects such as weight loss would occur when the immunoconjugate of the present invention was administered to tumor model mice. Surprisingly, however, no such side effects were observed. From the results described above, it was found that the immunoconjugate of the present invention did not exhibit toxicity to normal cells even when FL118 was separated and released in the blood.

12-7. Analysis of tumor growth inhibition in JIMT-1 or MDA-MB-468 tumor model mouse.

The average tumor volume of JIMT-1 or MDA-MB-468 tumor model mice (n = 3) in each group is shown in Tables 11 to 13, respectively.

[Table 11]

| Day * | Tumor volume ($mm^3$) | | | | |
|---|---|---|---|---|---|
| | Group 1 | Group 2 | Group 3 | Group 4 | Group 5 |
| 0 | 217.07 ± 2.79 | 216.90 ± 5.63 | 216.88 ± 6.84 | 216.71 ± 10.87 | 216.32 ± 12.67 |
| 4 | 401.98 ± 2.43 | 394.05 ± 24.62 | 355.15 ± 18.97 | 277.37 ± 34.46 | 260.58 ± 13.63 |
| 7 | 689.82 ± 50.65 | 628.63 ± 91.39 | 469.39 ± 93.29 | 315.61 ± 77.97 | 222.38 ± 56.72 |
| 11 | 948.32 ± 134.39 | 828.63 ± 152.73 | 423.47 ± 85.89 | 216.46 ± 56.19 | 154.95 ± 66.67 |
| 14 | 1066.95 ± 183.23 | 857.34 ± 174.86 | 337.93 ± 47.95 | 135.60 ± 31.90 | 112.48 ± 55.46 |
| 18 | 1177.47 ± 197.18 | 775.02 ± 141.84 | 317.85 ± 45.01 | 107.02 ± 23.21 | 70.20 ± 14.39 |
| 21 | 1336.32 ± 361.41 | 671.06 ± 92.85 | 370.14 ± 71.86 | 102.20 ± 18.12 | 73.87 ± 12.01 |
| *: Date after start of treatment | | | | | |

[Table 12]

| Day * | Tumor volume ($mm^3$) | | | | |
|---|---|---|---|---|---|
| | Group 1 | Group 2 | Group 3 | Group 4 | Group 5 |
| 0 | 214.67 ± 37.46 | 215.45 ± 17.45 | 212.05 ± 28.35 | 212.77 ± 16.17 | 212.28 ± 42.78 |
| 4 | 247.97 ± 30.89 | 237.94 ± 11.05 | 226.22 ± 17.07 | 175.37 ± 16.02 | 155.83 ± 26.88 |
| 7 | 282.55 ± 42.21 | 293.12 ± 23.39 | 225.91 ± 17.31 | 94.29 ± 6.31 | 61.23 ± 6.83 |
| 11 | 319.38 ± 39.33 | 318.07 ± 23.97 | 231.65 ± 15.17 | 48.37 ± 1.79 | 38.29 ± 8.03 |
| 14 | 349.00 ± 46.54 | 327.41 ± 30.50 | 252.72 ± 13.96 | 41.37 ± 5.48 | 23.16 ± 13.32 |
| 18 | 367.57 ± 51.24 | 340.95 ± 34.19 | 281.69 ± 20.40 | 24.77 ± 12.4 | 16.21 ± 8.12 |
| 21 | 390.24 ± 45.45 | 363.07 ± 44.38 | 361.79 ± 28.44 | 24.59 ± 12.8 | 7.56 ± 7.56 |
| *: Date after start of treatment | | | | | |

[Table 13]

| | Tumor volume ($mm^3$) (Day 0) | Tumor volume ($mm^3$) (Day 21) | TGI (%) (Day 21) | Significa nce[a] |
|---|---|---|---|---|
| Group 1 | 217.07 ± 2.79 | 1336.32 ± 361.41 | NA | NA |
| Group 2 | 216.90 ± 5.63 | 671.06 ± 92.85 | 59.41 | NS |
| Group 3 | 216.88 ± 6.84 | 370.14 ± 71.86 | 86.31 | ** |
| Group 4 | 216.71 ± 10.87 | 102.20 ± 18.12 | 110.23 | *** |
| Group 5 | 216.32 ± 12.67 | 73.87 ± 12.01 | 112.73 | *** |
| *a*. The significance was calculated by performing two-way ANOVA by comparing the tumor size with that of Group 1. * $p < 0.05$, ** $p < 0.01$, *** $p < 0.001$ | | | | |

Next, TGI values of the JIMT-1 or MDA-MB-468 tumor model mice (n = 3) were calculated based on the tumor size (n = 3) on Day 21 after treatment. The results are shown in Tables 14 to 16, respectively.

[Table 14]

| | Tumor size ($mm^3$) (Day 0) | Tumor size ($mm^3$) (Day 21) | TGI (%) (Day 21) | Signifi cance[a] |
|---|---|---|---|---|
| Group 1 | 214.67 ± 37.46 | 390.24 ±45.45 | NA | NA |
| Group 2 | 215.45 ± 17.45 | 363.07 ± 44.38 | 15.92 | NS |
| Group 3 | 212.05 ± 28.35 | 361.79 ± 28.44 | 14.71 | NS |
| Group 4 | 212.77 ± 16.17 | 24.59 ± 12.8 | 207.18 | *** |
| Group 5 | 212.28 ± 42.78 | 7.56 ± 7.56 | 216.60 | **** |
| *a*. The significance was calculated by performing two-way ANOVA by comparing the tumor size with that of Group 1. $p < 0.05$, ** $p < 0.01$, *** $p < 0.001$ | | | | |

[Table 15]

| | Tumor size ($mm^3$) (Day 0) | Tumor size ($mm^3$) (Day 21) | TGI (%) (Day 21) | Signifi cance[a] |
|---|---|---|---|---|
| Group 1 | 214.67 ± 37.46 | 390.24 ±45.45 | NA | NA |
| Group 2 | 215.45 ± 17.45 | 363.07 ± 44.38 | 15.92 | NS |
| Group 3 | 212.05 ± 28.35 | 361.79 ± 28.44 | 14.71 | NS |
| Group 4 | 212.77 ± 16.17 | 24.59 ± 12.8 | 207.18 | *** |
| Group 5 | 212.28 ± 42.78 | 7.56 ± 7.56 | 216.60 | **** |
| *a*. The significance was calculated by performing two-way ANOVA by comparing the tumor size with that of Group 1. $p < 0.05$, ** $p < 0.01$, *** $p < 0.001$ | | | | |

[Table 16]

| | Tumor size ($mm^3$) (Day 0) | Tumor size ($mm^3$) (Day 21) | TGI (%) (Day 21) | 유의성[a] |
|---|---|---|---|---|
| Group 1 | 200.81 ± 27.71 | 613.76 ± 125.11 | NA | NA |
| Group 2 | 200.20 ± 30.90 | 433.08 ± 126.82 | 43.60 | not signifi cant |
| Group 3 | 200.86 ± 22.49 | 129.35 ± 14.70 | 117.32 | **** |
| Group 4 | 200.66 ± 28.55 | 234.34 ± 64.31 | 91.85 | **** |
| Group 5 | 200.11 ± 26.13 | 77.45 ± 1.46 | 129.70 | **** |
| Group 6 | 200.69 ± 36.42 | 74.61 ± 11.55 | 130.53 | **** |
| *a*. The significance was calculated by performing two-way ANOVA by comparing the tumor size with that of Group 1. $p < 0.05$, ** $p < 0.01$, *** $p < 0.001$ | | | | |

The results of the tumor growth curves of the JIMT-1 or MDA-MB-468 tumor model mice (n = 3) are shown in FIGS. 8, 10 and 12, respectively. The test results showed that the mean tumor size of Group 1, the saline solution control group, was 1336.32 ± 361.41 $mm^3$ and 390.24 ± 45.45 $mm^3$ on Day 21 after treatment, respectively. Trastuzumab, cetuximab, or sacituzumab treatment all showed only partial antitumor effects compared to the saline control group without statistical significance (p>0.05), and the mean tumor volume was 671.06 ± 92.85 $mm^3$ (TGI = 59.41 %), 363.07 ± 44.38 $mm^3$ (TGI = 15.92 %) and 433.08 ± 126.82 $mm^3$ (TGI = 43.6 %), respectively. However, the immunoconjugate of Example 5 showed a mean tumor volume of 370.14 ± 71.86, 102.20 ± 18.12, and 73.87 ± 12.01 $mm^3$ at the dose of 1 mg/kg, 5 mg/kg, and 10 mg/kg (Groups 3, 4 and 5), respectively; the immunoconjugate of Example 8 showed a mean tumor volume of 24.59 ± 12.8 and 7.56 ± 7.56 $mm^3$ at the dose of 5 mg/kg and 10 mg/kg (Groups 4 and 5), respectively; and the immunoconjugate of Example 9 showed a mean tumor volume of 77.45 ± 1.46 and 74.61 ± 11.55 $mm^3$ at the dose of 5 mg/kg and 7 mg/kg (Groups 5 and 6), respectively, indicating an excellent anti-tumor effect (TGI

= 86.31, 110.23, 112.73 %, p < 0.01, 0.001, 0.001 for the immunoconjugate of Example 5; TGI = 207.18, 216.60 %, p < 0.001, 0.0001 for the immunoconjugate of Example 8; and TGI = 129.70, 130.53 %, p < 0.01, 0.05 for the immunoconjugate of Example 9). These results prove that the immunoconjugate of the present invention has a superior effect compared to trastuzumab, cetuximab, and sacituzumab, which are already known to exhibit excellent antitumor effects by themselves. In addition, since it was confirmed that all of the immunoconjugates of the present invention exhibit a dose-dependent response, they may be administered in a patient-customized regimen/dose to exhibit maximum efficacy and minimum toxicity for each patient.

In summary, the FL118-linker conjugate of the present invention can form an immunoconjugate by binding to an antibody or antigen-binding fragment thereof against a desired antigen. Although the immunoconjugates of Examples 5 and 6 prepared as an embodiment of the present invention comprise the same antibody (trastuzumab) as DS8201, which is a known immunoconjugate, and comprise a drug (payload) that is similar in structure and function, it was confirmed that the immunoconjugates exhibit overwhelmingly superior cytotoxicity to cancer cells in vitro compared to DS8201. The immunoconjugate of Example 8 comprising cetuximab, prepared as an example of the present invention, was also confirmed to exhibit strong cytotoxicity in vitro. The immunoconjugate of Example 9 comprising sacituzumab prepared as an example of the present invention, was also confirmed to exhibit strong cytotoxicity in vitro.

In addition, the test of the anticancer activity of the immunoconjugate of Example 5 and trastuzumab; the immunoconjugate of Example 8 and cetuximab; and the immunoconjugate of Example 9 and sacituzumab in tumor model mice showed that trastuzumab, cetuximab, and sasituzumab also exhibited a measure of anticancer effects, but it was confirmed that the immunoconjugate of the present invention showed significantly better anticancer activity at all the administered doses without a side effect such as weight loss.

Therefore, the immunoconjugate of the present invention can be very usefully applied to the prevention or treatment of cancer, and the FL118-linker conjugate of the present invention can be very usefully applied for the purpose of preparing the immunoconjugate.

From the above description, those skilled in the art to which the present invention belongs will be able to understand that the present invention may be embodied in other specific forms without changing the technical principles or essential characteristics thereof. In this regard, it should be understood that the Examples described above are illustrative in all respects and not restrictive. The scope of the present invention should be construed as including all changes or modifications derived from the meaning and scope of the claims described below and the equivalent concepts thereof rather than the detailed description above.

## Claims

**1.** An immunoconjugate comprising: [an FL118 drug of Formula 1]-[an acid-sensitive linker]-[an antibody or antigen-binding site-containing fragment thereof]; or a pharmaceutically acceptable salt thereof,

wherein at least one FL118 drug of Formula 1 is linked to an antibody or antigen-binding site-containing fragment thereof through an acid-sensitive linker,

wherein after being targeted to cancer cells by an antigen-binding site that targets an antigen of cancer cells, the acid-sensitive linker is degraded in acidic atmosphere around cancer (pH ≤ 7) to free at least a part of the FL118 drug of Formula 1 and the free FL118 drug of Formula 1 penetrates a cell membrane and moves into the cells,

wherein FL118 drug of Formula 1 inhibits the action an efflux pump to enrich the intracellular free FL118 drug of Formula 1, and

optionally, wherein the immunoconjugate in which the FL118 drug of Formula 1 is linked is internalized into the cell to free the FL118 drug of Formula 1 at lysosomes.

[Formula 1]

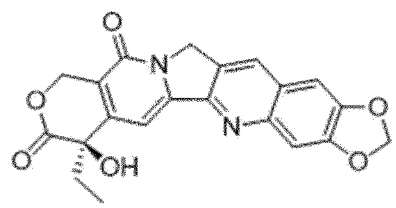

.

2. The immunoconjugate or a pharmaceutically acceptable salt thereof according to claim 1, wherein after being targeted to cancer cells by an antigen-binding site that targets an antigen of cancer cells, the acid-sensitive linker is degraded in acidic atmosphere around cancer (pH $\leq$ 7) to free at least a part of the FL118 drug of Formula 1 and free FL118 drug of Formula 1 penetrates a cell membrane and moves into the cells while penetrating deep into the cancer tissue.

3. The immunoconjugate or a pharmaceutically acceptable salt thereof according to claim 1, wherein, for the free FL118 drug of Formula 1 to be released when the acid-sensitive linker is degraded, the FL118 drug of Formula 1 is linked with the acid-sensitive linker by a carbonate or ester bond.

4. The immunoconjugate or a pharmaceutically acceptable salt thereof according to claim 1, wherein the cells to which the free FL118 drug of Formula 1 moves are targeted cancer cells and/or surrounding cells thereof.

5. The immunoconjugate or a pharmaceutically acceptable salt thereof according to claim 1, wherein the antibody or antigen-binding site thereof binds to a receptor on cell surface.

6. The immunoconjugate or a pharmaceutically acceptable salt thereof according to claim 1, wherein the antibody or antigen-binding site thereof targets antigens selectively distributed on the surface of cancer or cancer cell overexpression antigens, which are also distributed in a small number in normal tissues.

7. The immunoconjugate or a pharmaceutically acceptable salt thereof according to claim 1, wherein the antibody or antigen-binding site thereof targets HER2, FolR, PSMA or Trop-2, which is one of human epidermal growth factor receptor (HER/EGFR/ERBB).

8. The immunoconjugate or a pharmaceutically acceptable salt thereof according to claim 1, wherein the acid-sensitive linker is derived from a compound of Formula 2 below.

[Formula 2]

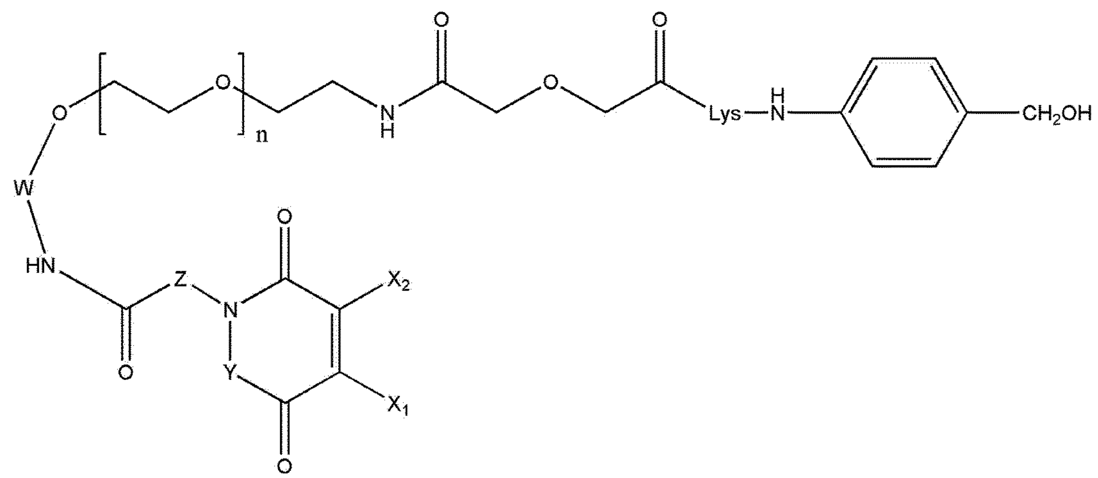

wherein, $X_1$ and $X_2$ are each independently -H or -halogen;
Y is -NH-, -$NR^A$ -, or null;
Z is -$C_1$-$C_4$ alkyl-, -$C_3$-$C_6$ cycloalkyl-, -($C_1$-$C_2$ alkyl)-($C_3$-$C_6$ cycloalkyl)-, -($C_3$-$C_6$ cycloalkyl)-($C_1$-$C_2$ alkyl)-, or -($C_1$-$C_2$ alkyl)-($C_3$-$C_6$ cycloalkyl)-($C_1$-$C_2$ alkyl)-;
W is -$R^B$-, -M- -$R^B$-M-, -M-$R^B$ - or -$R^B$-M-$R^C$-;
$R^A$ to $R^C$ are each independently $C_1$-$C_4$ alkyl;
M is

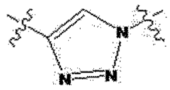

;

and

n is an integer from 5 to 9.

**9.** The immunoconjugate or a pharmaceutically acceptable salt thereof according to claim 8,

wherein in Formula 2 above,

$X_1$ and $X_2$ are each independently -H or -halogen;

Y is $-NR^A-$, or null;

Z is $-C_1-C_4$ alkyl-, $-(C_1-C_2$ alkyl)- $(C_3-C_6$ cycloalkyl)- or - $(C_3-C_6$ cycloalkyl)-$(C_1-C_2$ alkyl)-;

W is $-R^B-$ or $-R^B-M-R^C-$;

$R^A$ to $R^C$ are each independently $C_1-C_4$ alkyl;

M is

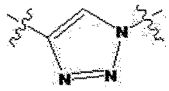

;

and

n is an integer from 5 to 9.

**10.** The immunoconjugate or a pharmaceutically acceptable salt thereof according to claim 8, wherein an alcohol group site of the FL118 drug of Formula 1 and an alcohol group site of the acid-sensitive linker of Formula 2 are linked.

**11.** The immunoconjugate or a pharmaceutically acceptable salt thereof according to claim 1, wherein the [FL118 drug of Formula 1]-[acid-sensitive linker] conjugate is derived from any one selected from the group consisting of compounds represented by Formulas 3 to 5 below.

[Formula 3]

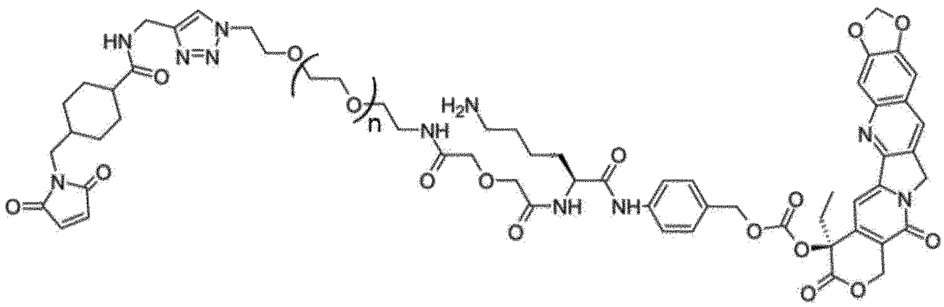

;

[Formula 4]

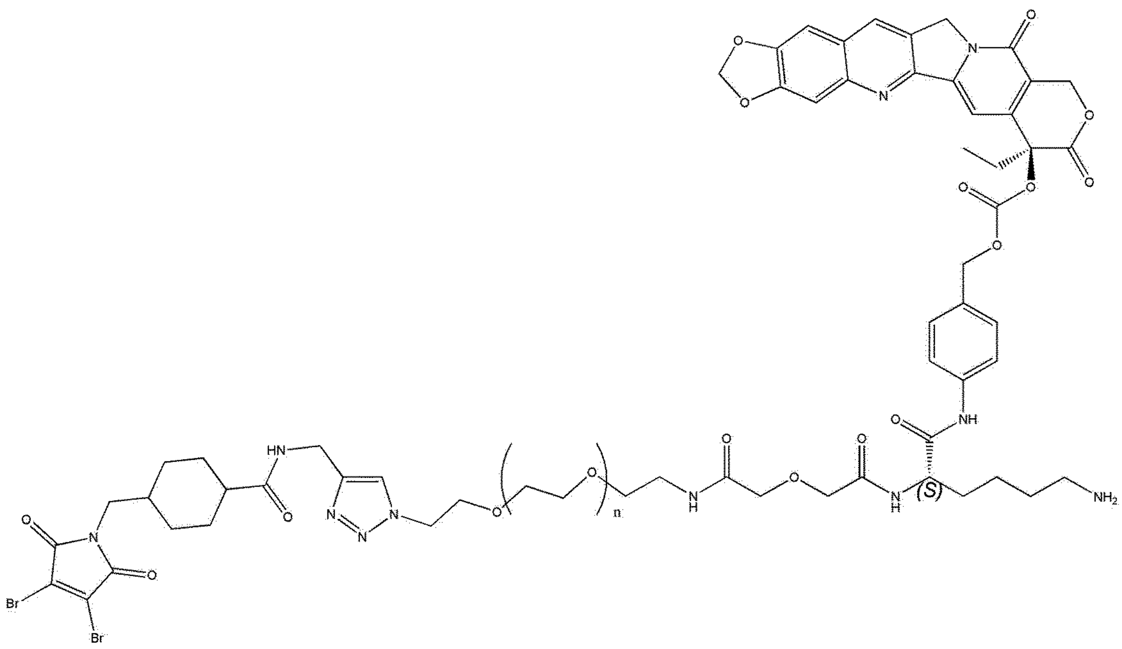

;

and

[Formula 5]

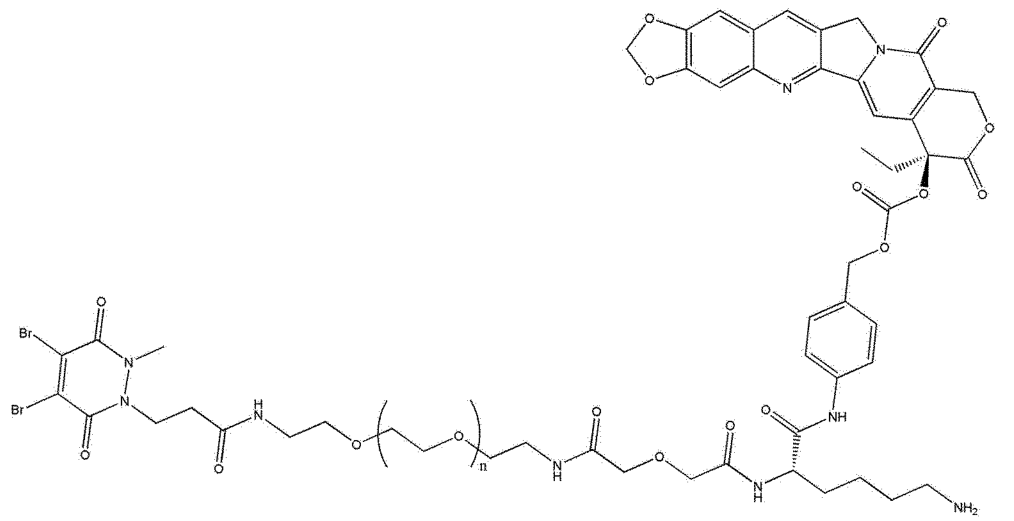

(wherein n is each independently an integer from 5 to 9).

**12.** The immunoconjugate or a pharmaceutically acceptable salt thereof according to claim 1, wherein the [acid-sensitive linker]-[antibody or antigen-binding site-containing fragment thereof] link is formed as a thiol group contained in the antibody or antigen-binding fragment thereof is bonded to a maleimide group or a maleic hydrazide group of the acid-sensitive linker.

**13.** The immunoconjugate or a pharmaceutically acceptable salt thereof according to claim 1, wherein the antibody or antigen-binding site-containing fragment thereof can target a cancel cell having resistance to SN-38 drug of Formula 6 below:

[Formula 6]

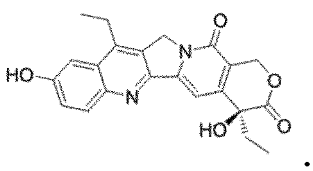

14. The immunoconjugate or a pharmaceutically acceptable salt thereof according to claim 1, wherein the immunoconjugate has an average drug-to-antibody ratio (DAR) of 2 to 12.

15. A pharmaceutical composition for preventing or treating cancer, comprising an immunoconjugate or pharmaceutically acceptable salt thereof according to any one of claims 1-14 as an active ingredient.

16. A drug-linker conjugate or a pharmaceutically acceptable salt thereof, wherein FL118 drug of Formula 1 below is linked to an acid-sensitive linker of Formula 2 below.

[Formula 1]

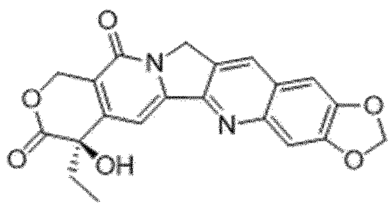

[Formula 2]

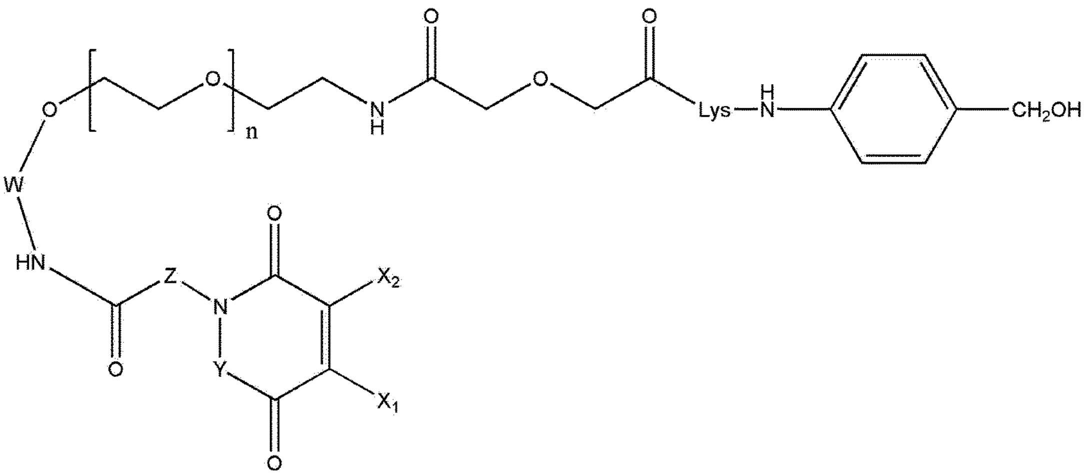

(wherein, $X_1$ and $X_2$ are each independently -H or -halogen;
Y is -NH-, -$NR^A$-, or null;
Z is -$C_1$-$C_4$ alkyl-, -$C_3$-$C_6$ cycloalkyl-, - ($C_1$-$C_2$ alkyl)-($C_3$-$C_6$ cycloalkyl)-, -($C_3$-$C_6$ cycloalkyl)-($C_1$-$C_2$ alkyl)-, or -($C_1$-$C_2$ alkyl)-($C_3$-$C_6$ cycloalkyl)-($C_1$-$C_2$ alkyl)-;
W is -$R^B$-, -M- -$R^B$-M-, -M-$R^B$ - or -$R^B$-M-$R^C$-;
$R^A$ to $R^C$ are each independently $C_1$-$C_4$ alkyl;
M is

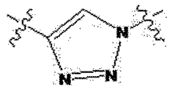;

and
n is an integer from 5 to 9).

**17.** The drug-linker conjugate or a pharmaceutically acceptable salt thereof according to claim 16,

wherein in Formula 2 above,
$X_1$ and $X_2$ are each independently -H or -halogen;
Y is $-NR^A-$, or null;
Z is $-C_1-C_4$ alkyl-, $-(C_1-C_2$ alkyl$)-(C_3-C_6$ cycloalkyl$)-$ or - $(C_3-C_6$ cycloalkyl$)-(C_1-C_2$ alkyl$)-$;
W is $-R^B-$ or $-R^B-M-R^C-$;
$R^A$ to $R^C$ are each independently $C_1-C_4$ alkyl;
M is

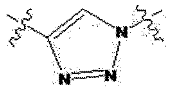;

and
n is an integer from 5 to 9.

**18.** The drug-linker conjugate or a pharmaceutically acceptable salt thereof according to claim 16, wherein for the free FL118 drug of Formula 1 to be released when the acid-sensitive linker is degraded, the FL118 drug of Formula 1 is linked with the acid-sensitive linker by a carbonate or ester bond.

**19.** The drug-linker conjugate or a pharmaceutically acceptable salt thereof according to claim 16, wherein an alcohol group site of the FL118 drug of Formula 1 and an alcohol group site of the acid-sensitive linker of Formula 2 are linked.

**20.** The drug-linker conjugate or a pharmaceutically acceptable salt thereof according to claim 16, wherein the drug-linker conjugate is any one selected from the group consisting of compounds represented by Formulas 3 to 5 below.

[Formula 3]

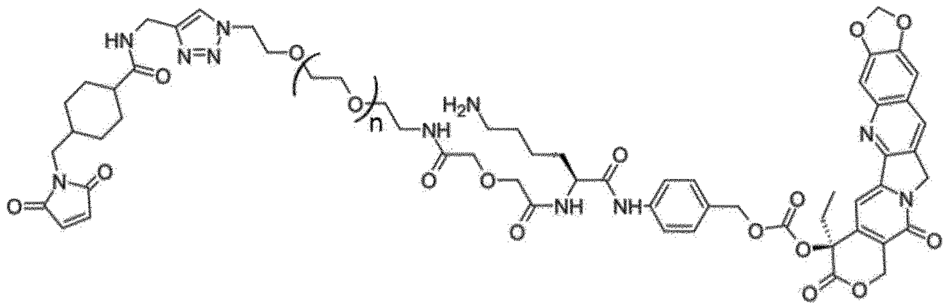;

[Formula 4]

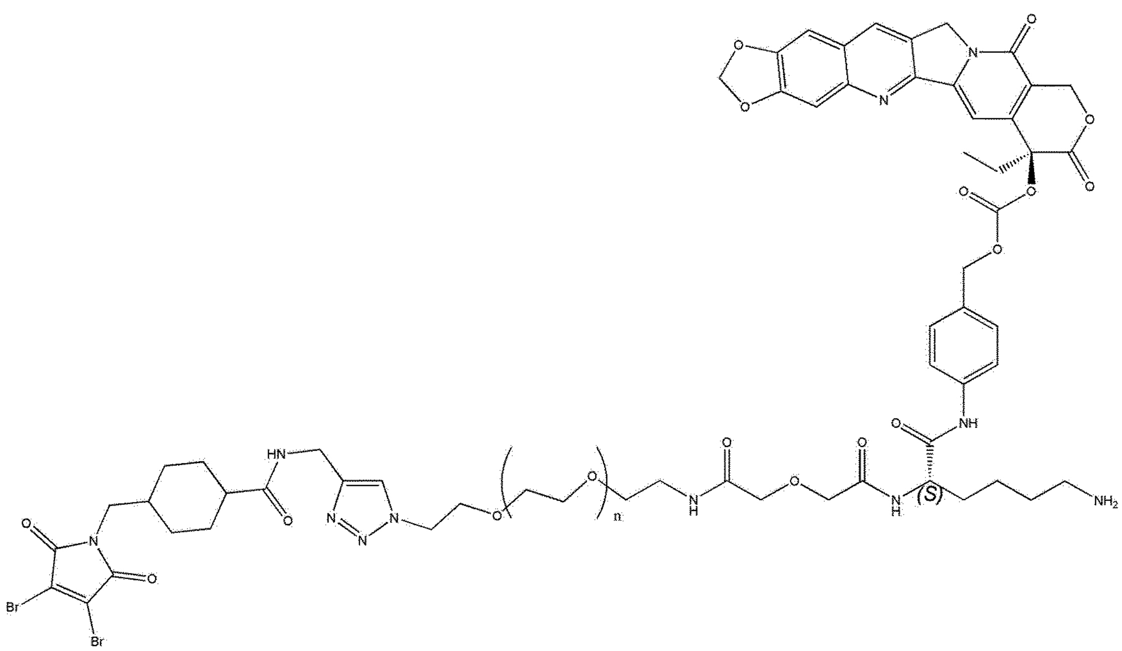

;

and

[Formula 5]

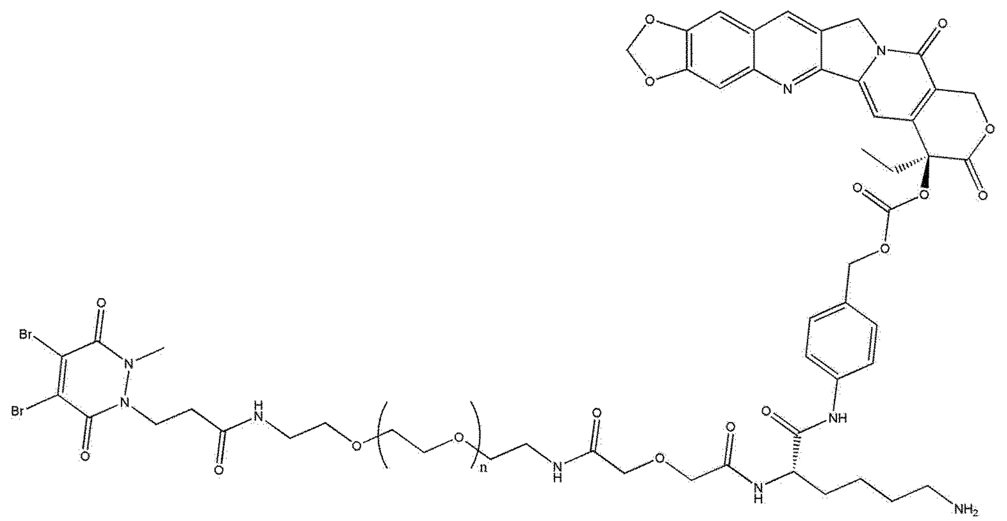

(wherein n is each independently an integer from 5 to 9).

**21.** The drug-linker conjugate or a pharmaceutically acceptable salt thereof according to any one of claims 16-20, wherein the acid-sensitive linker of Formula 2 is degraded in acidic atmosphere (pH ≤ 7) to free the FL118 drug of Formula 1.

**22.** An immunoconjugate or pharmaceutically acceptable salt thereof comprising:

(a) a drug-linker conjugate or a pharmaceutically acceptable salt thereof according to any one of claims 16-20; and
(b) an antibody or antigen-binding site-containing fragment thereof,

wherein at least one of the FL118 drugs of Formula 1 is linked to the antibody or antigen-binding site-containing fragment thereof through the acid-sensitive linker of Formula 2.

**23.** The immunoconjugate or pharmaceutically acceptable salt thereof according to claim 22, wherein the antibody is trastzumab, cetuximab or sacituzumab.

**24.** A method for preparing a carrier-drug conjugate, wherein the drug-linker conjugate or a pharmaceutically acceptable salt thereof according to any one of claims 16-20 is used to link at least one FL118 drug of Formula 1 to a carrier through an acid-sensitive linker of Formula 2.

**25.** The method for preparing a carrier-drug conjugate according to claim 24, wherein the carrier is an antibody, a repebody, and/or an aptamer.

[FIG. 1]

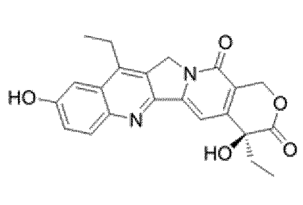
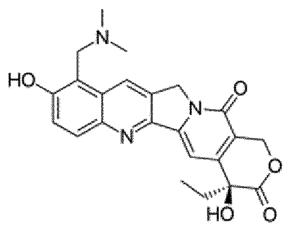
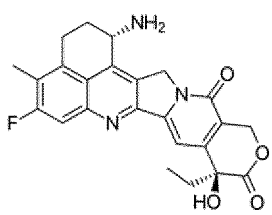

[FIG. 2]

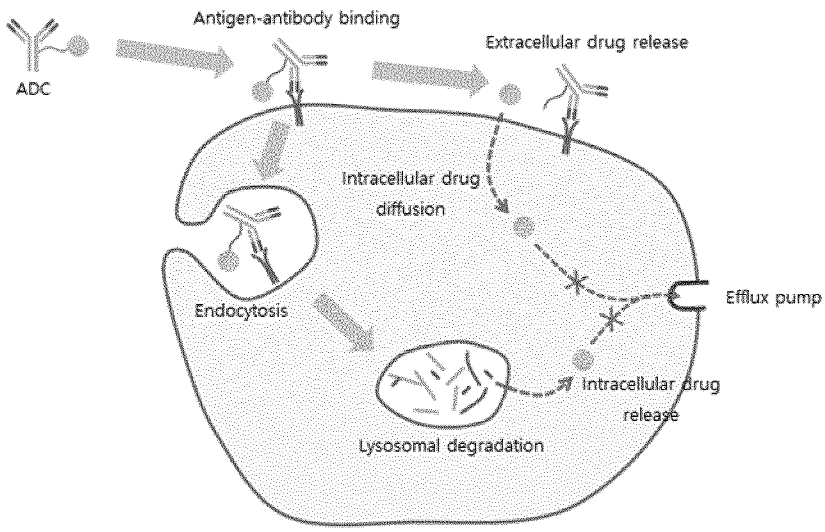
ADC
Antigen-antibody binding
Extracellular drug release
Intracellular drug diffusion
Endocytosis
Efflux pump
Intracellular drug release
Lysosomal degradation

[FIG. 3]

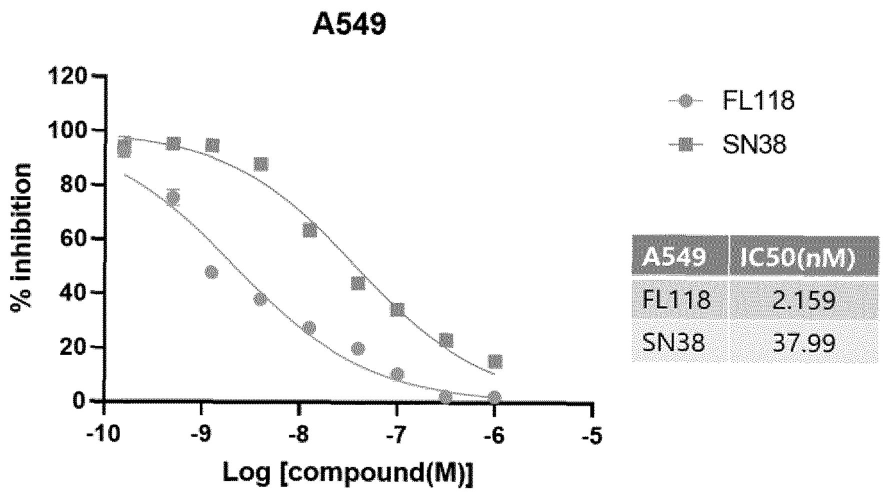


| A549 | IC50(nM) |
|---|---|
| FL118 | 2.159 |
| SN38 | 37.99 |

[FIG. 4]

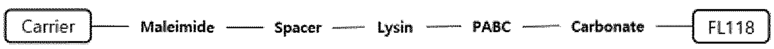


[FIG. 5a]

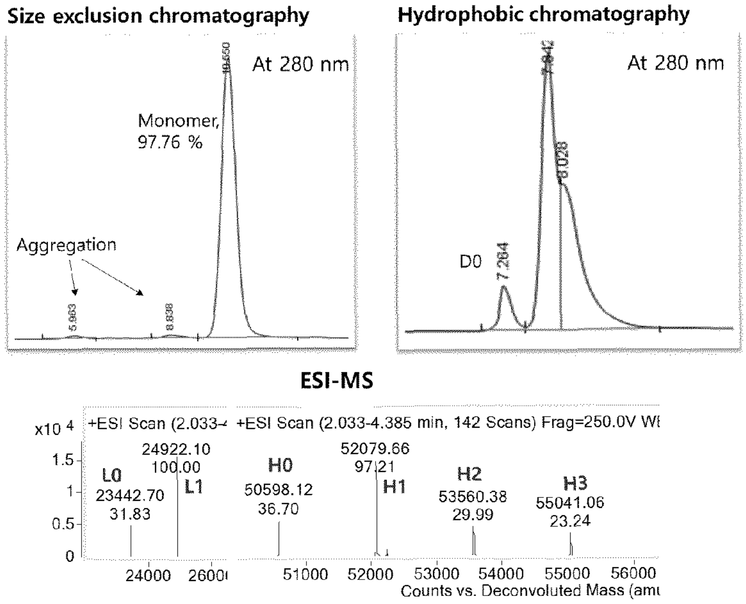


| Drug Distribution | L0 | L1 | L2 | H0 | H1 | H2 | H3 | H4 | MS-DAR |
|---|---|---|---|---|---|---|---|---|---|
| Mass | 23443 | 24922 | 26406 | 50598 | 52080 | 53560 | 55041 | 56526 | 4.02 |
| Peak Area | 25% | 75% | 0% | 20% | 48% | 18% | 14% | 0% | |

[FIG. 5b]

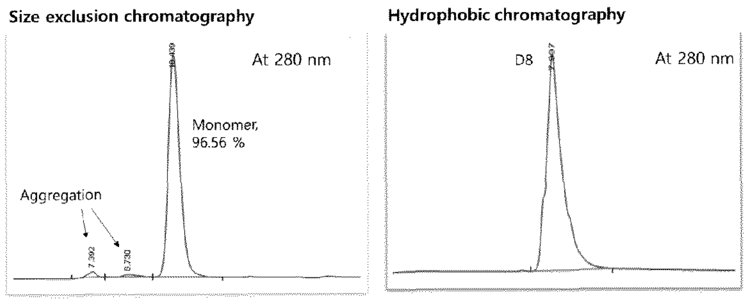

ESI-MS

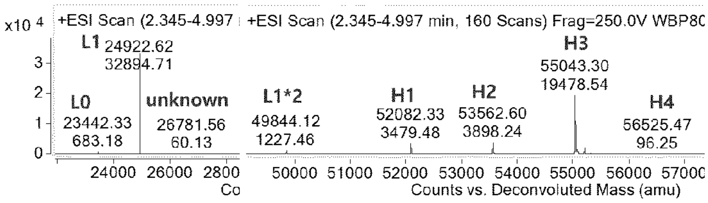


| Drug Distribution | L0 | L1 | L2 | H0 | H1 | H2 | H3 | H4 | MS-DAR |
|---|---|---|---|---|---|---|---|---|---|
| Mass | 23442 | 24923 | 26406 | 50598 | 52082 | 53563 | 55043 | 56525 | 7.14 |
| Peak Area | 2% | 98% | 0% | 0% | 13% | 15% | 71% | 0% | |

[FIG. 6]

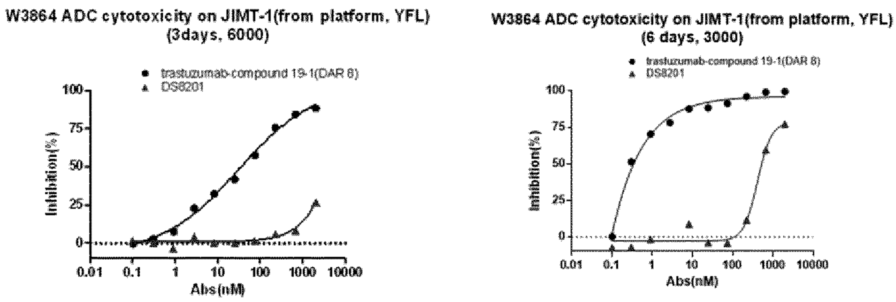


| | JIMT-1 (3 days) | | JIMT-1 (6 days) | |
|---|---|---|---|---|
| Abs | IC50 (nM) | Max Inh% | IC50 (nM) | Max Inh% |
| Trastuzumab-CL2A-FL119 (DAR 8) | 30.72 | 88.6 | 0.27 | 99.5 |
| DS8201 | NA | 27.2 | 412.20 | 77.2 |

[FIG. 7]

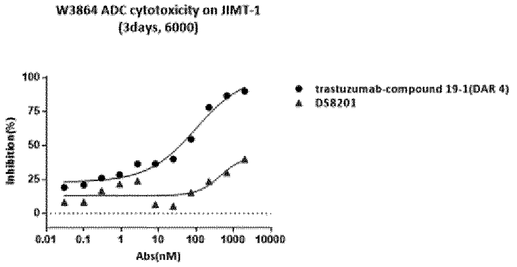


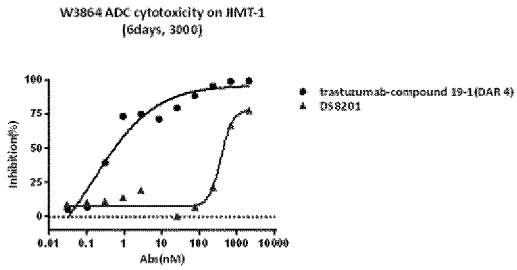


| Abs | JIMT-1-3days | | JIMT-1-6days | |
|---|---|---|---|---|
| | $IC_{50}$ (nM) | Max Cytotoxicity(%) | $IC_{50}$ (nM) | Max Cytotoxicity(%) |
| Trastuzumab-compound 19-1 (DAR 4) | 100.3 | 90.0 | 0.16 | 99.6 |
| DS8201 | 447.4 | 40.0 | 369.4 | 77.9 |

[FIG. 8]

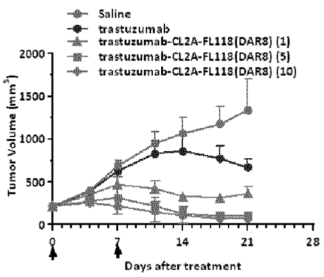


| Group (mg/kg) | TGI (%) |
|---|---|
| saline | na |
| Trastuzumab (10) | 59.41 |
| trastuzumab-CL2A-FL118 (DAR 8) (1) | 86.31 |
| trastuzumab-CL2A-FL118 (DAR 8) (5) | 110.23 |
| trastuzumab-CL2A-FL118 (DAR 8) (10) | 112.73 |

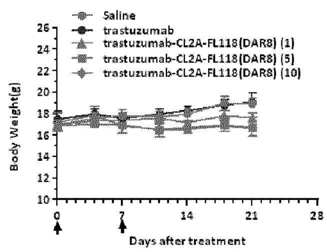


[FIG. 9]

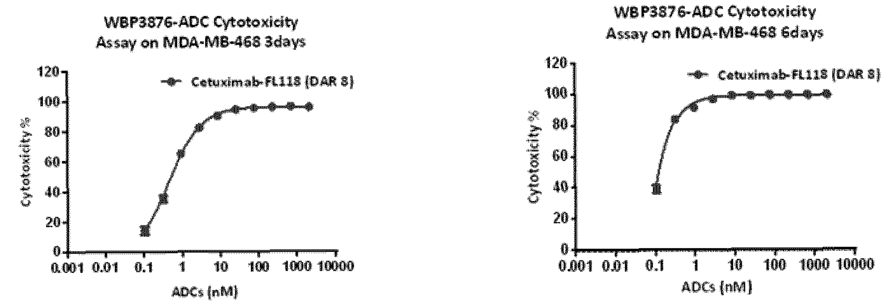


| | MDA-MB-468 (3 days) | | MDA-MB-468 (6 days) | |
|---|---|---|---|---|
| Abs | IC50 (nM) | Max Inh% | IC50 (nM) | Max Inh% |
| Cetuximab-CL2A-FL119 (DAR 8) | 0.4546 | 97 | NA | 100 |

[FIG. 10]

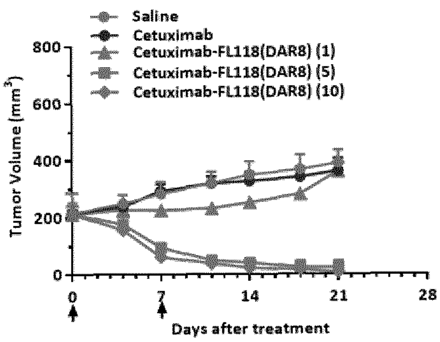


| Group (mg/kg) | TGI (%) |
|---|---|
| saline | NA |
| Cetuximab (10) | 15.92 |
| Cetuximab-FL118 (DAR 8) (1) | 14.71 |
| Cetuximab-FL118 (DAR 8) (5) | 207.18 |
| Cetuximab-FL118 (DAR 8) (10) | 216.60 |

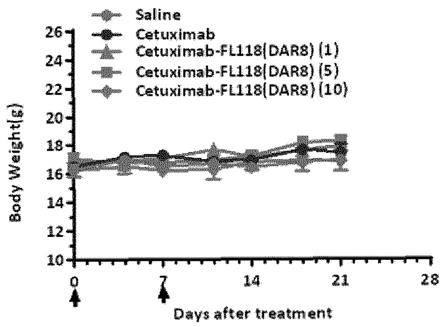


[FIG. 11]

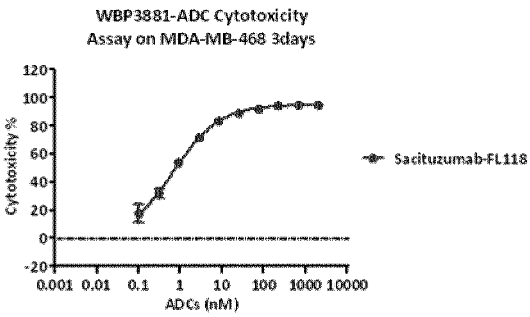


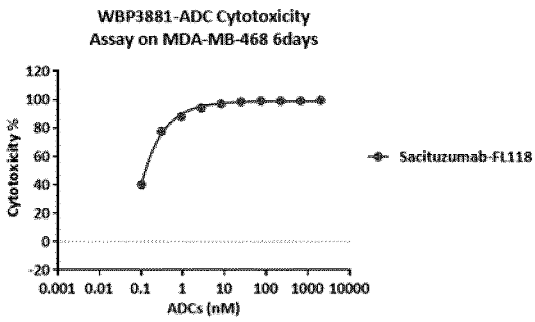


| Abs | MDA-MB-468-3days | | MDA-MB-468-6days | |
|---|---|---|---|---|
| | $IC_{50}$ (nM) | Max Cytotoxicity(%) | $IC_{50}$ (nM) | Max Cytotoxicity(%) |
| Sacituzumab-FL118 | 0.6569 | 95 | NA | 100 |

[FIG. 12]

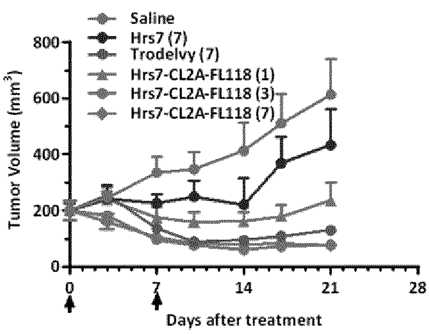


| Group (mg/kg) | TGI (%) |
|---|---|
| saline | NA |
| Hrs7 (7) | 43.6 |
| Trodelvy (7) | 117.32 |
| Hrs7-CL2A-FL118 (1) | 91.85 |
| Hrs7-CL2A-FL118 (3) | 129.70 |
| Hrs7-CL2A-FL118 (7) | 130.53 |

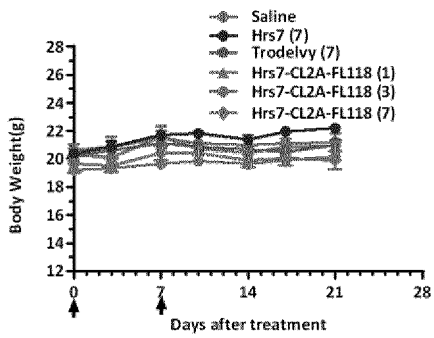

[FIG. 13]

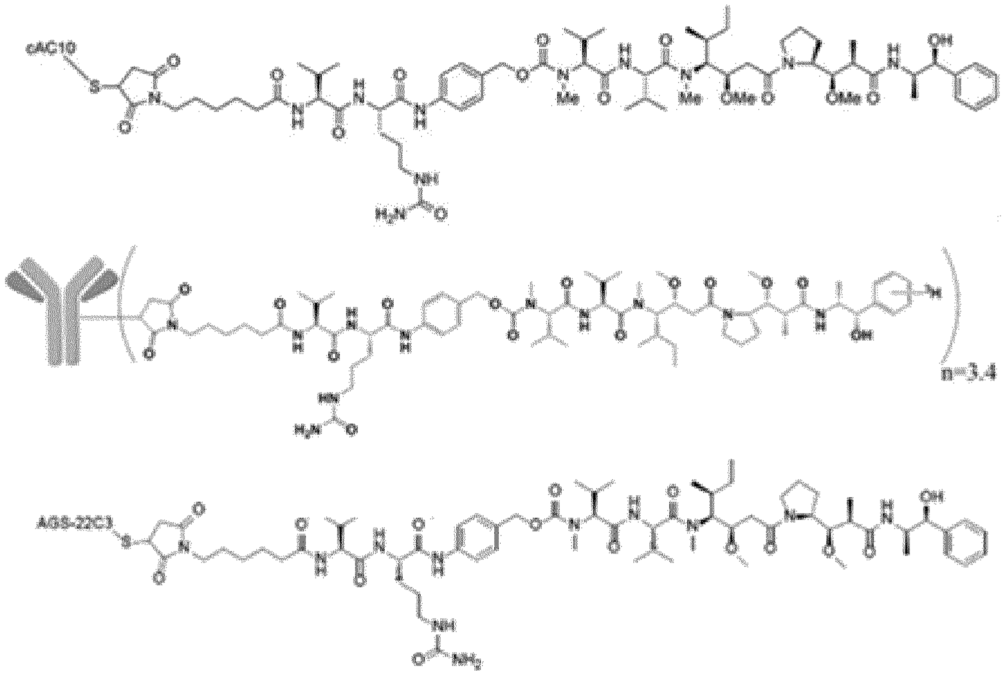

[FIG. 14]

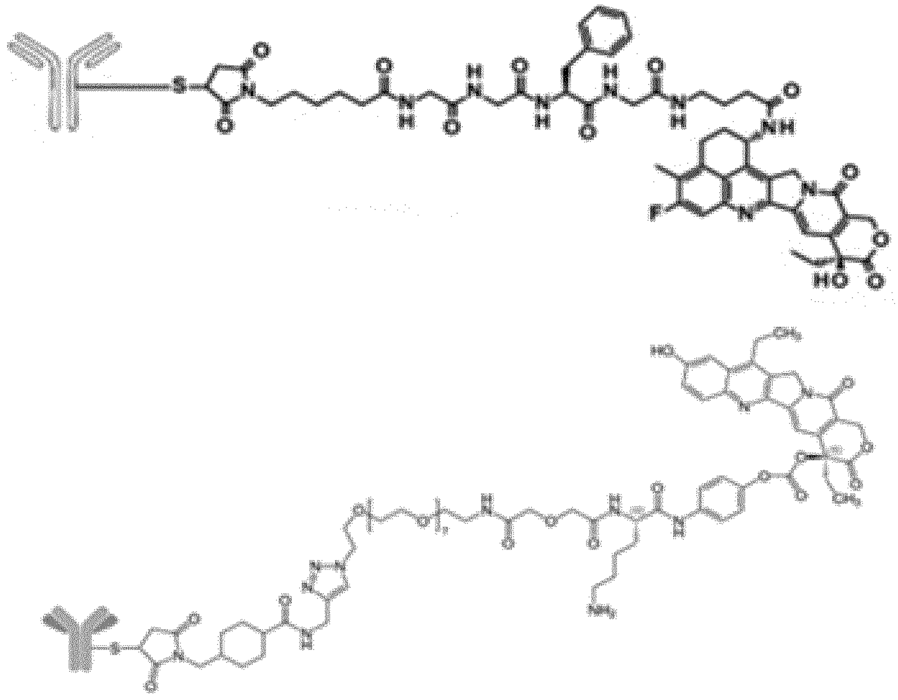

**INTERNATIONAL SEARCH REPORT**

International application No.
**PCT/KR2021/009204**

**A. CLASSIFICATION OF SUBJECT MATTER**

**A61K 47/68**(2017.01)i; **A61K 47/54**(2017.01)i; **A61P 35/00**(2006.01)i; **C07F 7/18**(2006.01)i

According to International Patent Classification (IPC) or to both national classification and IPC

**B. FIELDS SEARCHED**

Minimum documentation searched (classification system followed by classification symbols)

A61K 47/68(2017.01); A61K 31/4745(2006.01); A61K 31/675(2006.01); C07D 491/22(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 산 민감성 링커 (acid-sensitive linker), 항체 (antibody), FL118

**C. DOCUMENTS CONSIDERED TO BE RELEVANT**

| Category* | Citation of document, with indication, where appropriate, of the relevant passages | Relevant to claim No. |
|---|---|---|
| Y | US 2017-0360770 A1 (IMMUNOMEDICS, INC.) 21 December 2017 (2017-12-21)<br>See abstract; claims 1-40; paragraphs [0003], [0009], [0014], [0052] and [0068]; examples 1 and 3; pages 30 and 31; and figure 1. | 1-10,12-19,21-25 |
| A | | 11,20 |
| Y | LING, X. et al. FL118, a novel camptothecin analogue, overcomes irinotecan and topotecan resistance in human tumor xenograft models. American Journal of Translational Research. 2015, vol. 7, no. 10, pp. 1765-1781.<br>See abstract; page 1777; and figure 1. | 1-10,12-19,21-25 |
| A | ZHANG, Y. et al. Self-assembling nanoparticles of dually hydrophobic prodrugs constructed from camptothecin analogue for cancer therapy. European Journal of Medicinal Chemistry. 30 April 2020, vol. 200, 112365, pp. 1-11.<br>See entire document. | 1-25 |
| A | US 2018-0170944 A1 (CANGET BIO TEKPHARMA, LLC) 21 June 2018 (2018-06-21)<br>See entire document. | 1-25 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 October 2021** | **22 October 2021** |
| Name and mailing address of the ISA/KR<br>**Korean Intellectual Property Office**<br>**Government Complex-Daejeon Building 4, 189 Cheongsa-ro, Seo-gu, Daejeon 35208** | Authorized officer |
| Facsimile No. **+82-42-481-8578** | Telephone No. |

Form PCT/ISA/210 (second sheet) (July 2019)

INTERNATIONAL SEARCH REPORT

International application No.
**PCT/KR2021/009204**

**C. DOCUMENTS CONSIDERED TO BE RELEVANT**

| Category* | Citation of document, with indication, where appropriate, of the relevant passages | Relevant to claim No. |
|---|---|---|
| A | WO 2012-058666 A2 (HEALTH RESEARCH, INC.) 03 May 2012 (2012-05-03) See entire document. | 1-25 |

Form PCT/ISA/210 (second sheet) (July 2019)

**INTERNATIONAL SEARCH REPORT**
**Information on patent family members**

International application No.
**PCT/KR2021/009204**

| Patent document cited in search report | Publication date (day/month/year) | Patent family member(s) | Publication date (day/month/year) |
|---|---|---|---|
| US 2017-0360770 A1 | 21 December 2017 | AT 500845 T | 15 March 2011 |
| | | AU 2018-201403 A1 | 15 March 2018 |
| | | AU 2018-201403 B2 | 18 July 2019 |
| | | AU 2019-250192 A1 | 07 November 2019 |
| | | AU 2019-250192 B2 | 01 April 2021 |
| | | AU 4829600 A | 21 November 2000 |
| | | AU 5600500 A | 28 December 2000 |
| | | AU 6761098 A | 20 October 1998 |
| | | AU 728325 B2 | 04 January 2001 |
| | | AU 774044 B2 | 17 June 2004 |
| | | AU 782160 B2 | 07 July 2005 |
| | | BR 0311799 A | 10 May 2005 |
| | | BR 112012004269 A2 | 16 November 2016 |
| | | BR PI0406574 A | 17 January 2006 |
| | | BR PI0607486 A2 | 04 August 2009 |
| | | BR PI0607486 B1 | 16 April 2019 |
| | | BR PI0607486 B8 | 25 May 2021 |
| | | CA 2914438 A1 | 29 January 2015 |
| | | CA 2916671 A1 | 24 July 2008 |
| | | CA 2916671 C | 09 January 2018 |
| | | CA 2920192 A1 | 02 April 2015 |
| | | CA 2948013 A1 | 07 January 2016 |
| | | CA 2948693 A1 | 19 August 2010 |
| | | CA 2948693 C | 04 September 2018 |
| | | CA 2961774 A1 | 14 April 2016 |
| | | CA 3031737 A1 | 15 February 2018 |
| | | CA 3043766 A1 | 30 August 2018 |
| | | CA 3044096 A1 | 07 June 2018 |
| | | CN 107735090 A | 23 February 2018 |
| | | CN 107735090 B | 06 April 2021 |
| | | CN 107735104 A | 23 February 2018 |
| | | CN 107753954 A | 06 March 2018 |
| | | CN 109562172 A | 02 April 2019 |
| | | CN 110075295 A | 02 August 2019 |
| | | CN 110248680 A | 17 September 2019 |
| | | CN 110392580 A | 29 October 2019 |
| | | CN 1649902 A | 03 August 2005 |
| | | CN 1649902 B | 13 April 2011 |
| | | CN 1649903 A | 03 August 2005 |
| | | CN 1675245 A | 28 September 2005 |
| | | CN 1675245 B | 12 January 2011 |
| | | CN 1764478 A | 26 April 2006 |
| | | CN 1764478 B | 21 October 2015 |
| | | CY 1110556 T1 | 29 April 2015 |
| | | DE 05075555 T1 | 08 February 2007 |
| | | DE 69830570 T2 | 03 November 2005 |
| | | DK 0969866 T3 | 03 October 2005 |
| | | DK 1194167 T3 | 16 November 2009 |
| | | DK 2396036 T3 | 16 October 2017 |
| | | DK 3332808 T3 | 14 December 2020 |

Form PCT/ISA/210 (patent family annex) (July 2019)

**INTERNATIONAL SEARCH REPORT**
**Information on patent family members**

International application No.
**PCT/KR2021/009204**

| Patent document cited in search report | Publication date (day/month/year) | Patent family member(s) | Publication date (day/month/year) |
|---|---|---|---|
| | | DK 3483183 T3 | 21 June 2021 |
| | | EP 2674440 A2 | 18 December 2013 |
| | | EP 2674440 A3 | 05 March 2014 |
| | | EP 2674440 B1 | 03 July 2019 |
| | | EP 2675485 A2 | 25 December 2013 |
| | | EP 2675485 A4 | 15 October 2014 |
| | | EP 2704751 A1 | 12 March 2014 |
| | | EP 2704751 A4 | 03 June 2015 |
| | | EP 2704751 B1 | 17 April 2019 |
| | | EP 2764362 A1 | 13 August 2014 |
| | | JP 2016-534092 A | 04 November 2016 |
| | | JP 2017-536340 A | 07 December 2017 |
| | | JP 2018-520148 A | 26 July 2018 |
| | | JP 2020-105187 A | 09 July 2020 |
| | | JP 2020-183429 A | 12 November 2020 |
| | | JP 2020-508292 A | 19 March 2020 |
| | | JP 5214252 B2 | 19 June 2013 |
| | | JP 5231231 B2 | 10 July 2013 |
| | | JP 5314590 B2 | 16 October 2013 |
| | | JP 5335651 B2 | 06 November 2013 |
| | | JP 5388355 B2 | 15 January 2014 |
| | | JP 5435432 B2 | 05 March 2014 |
| | | JP 5682000 B2 | 11 March 2015 |
| | | JP 5699362 B2 | 08 April 2015 |
| | | JP 5740772 B2 | 01 July 2015 |
| | | JP 5779789 B2 | 16 September 2015 |
| | | JP 5789821 B2 | 07 October 2015 |
| | | JP 6024025 B2 | 09 November 2016 |
| | | JP 6114936 B2 | 19 April 2017 |
| | | JP 6205621 B2 | 04 October 2017 |
| | | JP 6366111 B2 | 01 August 2018 |
| | | JP 6427789 B2 | 28 November 2018 |
| | | JP 6678941 B2 | 15 April 2020 |
| | | JP 6741349 B2 | 19 August 2020 |
| | | KR 10-1002443 B1 | 17 December 2010 |
| | | KR 10-1017732 B1 | 28 February 2011 |
| | | KR 10-1053739 B1 | 02 August 2011 |
| | | KR 10-1143035 B1 | 08 May 2012 |
| | | KR 10-1287280 B1 | 23 July 2013 |
| | | KR 10-1410521 B1 | 20 June 2014 |
| | | KR 10-1482295 B1 | 13 January 2015 |
| | | KR 10-1583388 B1 | 07 January 2016 |
| | | KR 10-2002-0020730 A | 15 March 2002 |
| | | KR 10-2004-0089694 A | 21 October 2004 |
| | | KR 10-2004-0089695 A | 21 October 2004 |
| | | KR 10-2005-0010054 A | 26 January 2005 |
| | | KR 10-2005-0104354 A | 02 November 2005 |
| | | KR 10-2008-0055932 A | 19 June 2008 |
| | | KR 10-2008-0097995 A | 06 November 2008 |
| | | KR 10-2010-0085932 A | 29 July 2010 |

Form PCT/ISA/210 (patent family annex) (July 2019)

**INTERNATIONAL SEARCH REPORT**
**Information on patent family members**

International application No.
**PCT/KR2021/009204**

| Patent document cited in search report | Publication date (day/month/year) | Patent family member(s) | Publication date (day/month/year) |
|---|---|---|---|
| | | KR 10-2010-0132484 A | 17 December 2010 |
| | | KR 10-2015-0093159 A | 17 August 2015 |
| | | KR 10-2018-0023915 A | 07 March 2018 |
| | | MX 2010004547 A | 05 July 2010 |
| | | MX 2010006728 A | 05 November 2010 |
| | | MX 2015007343 A | 10 September 2015 |
| | | MX 342894 B | 18 October 2016 |
| | | PL 2396036 T3 | 29 December 2017 |
| | | PL 378012 A1 | 20 February 2006 |
| | | PT 1194167 E | 23 October 2009 |
| | | PT 1450891 E | 31 January 2013 |
| | | RU 2019119320 A3 | 12 January 2021 |
| | | RU 2349343 C2 | 20 March 2009 |
| | | SG 153825 A1 | 29 July 2009 |
| | | TR 201901824 T4 | 21 March 2019 |
| | | TR 201910848 T4 | 21 August 2019 |
| | | US 10377829 B2 | 13 August 2019 |
| | | US 10413539 B2 | 17 September 2019 |
| | | US 10561738 B2 | 18 February 2020 |
| | | US 10653793 B2 | 19 May 2020 |
| | | US 10682347 B2 | 16 June 2020 |
| | | US 10709701 B2 | 14 July 2020 |
| | | US 10744129 B2 | 18 August 2020 |
| | | US 10751420 B2 | 25 August 2020 |
| | | US 10849986 B2 | 01 December 2020 |
| | | US 2002-0041847 A1 | 11 April 2002 |
| | | US 2002-0071807 A1 | 13 June 2002 |
| | | US 2003-0124058 A1 | 03 July 2003 |
| | | US 2003-0133930 A1 | 17 July 2003 |
| | | US 2003-0171637 A1 | 11 September 2003 |
| | | US 2004-0202666 A1 | 14 October 2004 |
| | | US 2004-0219203 A1 | 04 November 2004 |
| | | US 2004-0230087 A1 | 18 November 2004 |
| | | US 2005-0014207 A1 | 20 January 2005 |
| | | US 2005-0191300 A1 | 01 September 2005 |
| | | US 2005-0271671 A1 | 08 December 2005 |
| | | US 2006-0014245 A1 | 19 January 2006 |
| | | US 2006-0228300 A1 | 12 October 2006 |
| | | US 2006-0228357 A1 | 12 October 2006 |
| | | US 2006-0235365 A1 | 19 October 2006 |
| | | US 2006-0264688 A1 | 23 November 2006 |
| | | US 2007-0020265 A1 | 25 January 2007 |
| | | US 2007-0086942 A1 | 19 April 2007 |
| | | US 2007-0087001 A1 | 19 April 2007 |
| | | US 2007-0135674 A1 | 14 June 2007 |
| | | US 2008-0050311 A1 | 28 February 2008 |
| | | US 2008-0253964 A1 | 16 October 2008 |
| | | US 2009-0060862 A1 | 05 March 2009 |
| | | US 2010-0189641 A1 | 29 July 2010 |
| | | US 2010-0266497 A1 | 21 October 2010 |

Form PCT/ISA/210 (patent family annex) (July 2019)

**INTERNATIONAL SEARCH REPORT**
**Information on patent family members**

International application No.
**PCT/KR2021/009204**

| Patent document cited in search report | Publication date (day/month/year) | Patent family member(s) | Publication date (day/month/year) |
|---|---|---|---|
| | | US 2010-0272636 A1 | 28 October 2010 |
| | | US 2010-0284906 A1 | 11 November 2010 |
| | | US 2011-0008251 A1 | 13 January 2011 |
| | | US 2011-0020273 A1 | 27 January 2011 |
| | | US 2011-0300105 A1 | 08 December 2011 |
| | | US 2011-0305631 A1 | 15 December 2011 |
| | | US 2011-0311531 A1 | 22 December 2011 |
| | | US 2011-0318306 A1 | 29 December 2011 |
| | | US 2012-0009149 A1 | 12 January 2012 |
| | | US 2012-0040431 A1 | 16 February 2012 |
| | | US 2012-0076727 A1 | 29 March 2012 |
| | | US 2013-0164816 A1 | 27 June 2013 |
| | | US 2013-0171064 A1 | 04 July 2013 |
| | | US 2013-0171065 A1 | 04 July 2013 |
| | | US 2014-0377177 A1 | 25 December 2014 |
| | | US 2014-0377287 A1 | 25 December 2014 |
| | | US 2015-0010982 A1 | 08 January 2015 |
| | | US 2015-0023870 A1 | 22 January 2015 |
| | | US 2015-0024458 A1 | 22 January 2015 |
| | | US 2015-0038686 A1 | 05 February 2015 |
| | | US 2015-0050715 A1 | 19 February 2015 |
| | | US 2015-0056680 A1 | 26 February 2015 |
| | | US 2015-0374846 A1 | 31 December 2015 |
| | | US 2016-0024212 A1 | 28 January 2016 |
| | | US 2017-0320952 A1 | 09 November 2017 |
| | | US 2017-0334996 A1 | 23 November 2017 |
| | | US 2018-0079816 A1 | 22 March 2018 |
| | | US 2018-0085469 A1 | 29 March 2018 |
| | | US 2018-0327495 A1 | 15 November 2018 |
| | | US 2019-0015405 A1 | 17 January 2019 |
| | | US 2020-0276325 A1 | 03 September 2020 |
| | | US 2020-0316054 A1 | 08 October 2020 |
| | | US 6183744 B1 | 06 February 2001 |
| | | US 6306393 B1 | 23 October 2001 |
| | | US 6761680 B2 | 13 July 2004 |
| | | US 6786858 B2 | 07 September 2004 |
| | | US 6820318 B2 | 23 November 2004 |
| | | US 6997862 B2 | 14 February 2006 |
| | | US 7008368 B2 | 07 March 2006 |
| | | US 7060020 B2 | 13 June 2006 |
| | | US 7074291 B2 | 11 July 2006 |
| | | US 7074403 B1 | 11 July 2006 |
| | | US 7094198 B2 | 22 August 2006 |
| | | US 7211039 B2 | 01 May 2007 |
| | | US 7238785 B2 | 03 July 2007 |
| | | US 7244226 B2 | 17 July 2007 |
| | | US 7252630 B2 | 07 August 2007 |
| | | US 7282567 B2 | 16 October 2007 |
| | | US 7312318 B2 | 25 December 2007 |
| | | US 7407477 B2 | 05 August 2008 |

Form PCT/ISA/210 (patent family annex) (July 2019)

**INTERNATIONAL SEARCH REPORT**
**Information on patent family members**

International application No.
**PCT/KR2021/009204**

| Patent document cited in search report | Publication date (day/month/year) | Patent family member(s) | Publication date (day/month/year) |
|---|---|---|---|
| | | US 7497818 B2 | 03 March 2009 |
| | | US 7517964 B2 | 14 April 2009 |
| | | US 7521056 B2 | 21 April 2009 |
| | | US 7597876 B2 | 06 October 2009 |
| | | US 7612180 B2 | 03 November 2009 |
| | | US 7641901 B2 | 05 January 2010 |
| | | US 7642239 B2 | 05 January 2010 |
| | | US 7666400 B2 | 23 February 2010 |
| | | US 7942803 B2 | 17 May 2011 |
| | | US 7981398 B2 | 19 July 2011 |
| | | US 7993626 B2 | 09 August 2011 |
| | | US 7999083 B2 | 16 August 2011 |
| | | US 8003111 B2 | 23 August 2011 |
| | | US 8343460 B2 | 01 January 2013 |
| | | US 8343496 B2 | 01 January 2013 |
| | | US 8349332 B2 | 08 January 2013 |
| | | US 8361464 B2 | 29 January 2013 |
| | | US 8367037 B2 | 05 February 2013 |
| | | US 8632752 B2 | 21 January 2014 |
| | | US 8652484 B2 | 18 February 2014 |
| | | US 8658773 B2 | 25 February 2014 |
| | | US 9513293 B2 | 06 December 2016 |
| | | US 9522959 B2 | 20 December 2016 |
| | | US 9540435 B2 | 10 January 2017 |
| | | US 9849176 B2 | 26 December 2017 |
| | | US 9855344 B2 | 02 January 2018 |
| | | US 9862770 B2 | 09 January 2018 |
| | | US 9872920 B2 | 23 January 2018 |
| | | US 9931413 B2 | 03 April 2018 |
| | | US 9931417 B2 | 03 April 2018 |
| | | US 9963516 B2 | 08 May 2018 |
| | | US 9999668 B2 | 19 June 2018 |
| | | WO 00-67795 A1 | 16 November 2000 |
| | | WO 00-74718 A1 | 14 December 2000 |
| | | WO 03-039463 A2 | 15 May 2003 |
| | | WO 03-039463 A3 | 31 July 2003 |
| | | WO 2004-110390 A2 | 23 December 2004 |
| | | WO 2004-110390 A3 | 28 April 2005 |
| | | WO 2005-080586 A1 | 01 September 2005 |
| | | WO 2006-094192 A2 | 08 September 2006 |
| | | WO 2006-094192 A3 | 09 November 2006 |
| | | WO 2007-046893 A2 | 26 April 2007 |
| | | WO 2007-046893 A3 | 23 April 2009 |
| | | WO 2007-112193 A2 | 04 October 2007 |
| | | WO 2007-112193 A3 | 20 November 2008 |
| | | WO 2007-134037 A2 | 22 November 2007 |
| | | WO 2007-134037 A3 | 27 November 2008 |
| | | WO 2008-088648 A2 | 24 July 2008 |
| | | WO 2008-088648 A3 | 25 September 2008 |
| | | WO 2008-088658 A2 | 24 July 2008 |

Form PCT/ISA/210 (patent family annex) (July 2019)

**INTERNATIONAL SEARCH REPORT**
**Information on patent family members**

International application No.
**PCT/KR2021/009204**

| Patent document cited in search report | Publication date (day/month/year) | Patent family member(s) | Publication date (day/month/year) |
|---|---|---|---|
| | | WO 2008-088658 A3 | 20 November 2008 |
| | | WO 2009-055653 A1 | 30 April 2009 |
| | | WO 2009-079024 A1 | 25 June 2009 |
| | | WO 2009-100194 A2 | 13 August 2009 |
| | | WO 2009-100194 A3 | 07 January 2010 |
| | | WO 2009-126558 A1 | 15 October 2009 |
| | | WO 2009-135015 A2 | 05 November 2009 |
| | | WO 2009-135015 A3 | 07 January 2010 |
| | | WO 2009-135015 A8 | 08 April 2010 |
| | | WO 2010-017500 A2 | 11 February 2010 |
| | | WO 2010-017500 A3 | 22 April 2010 |
| | | WO 2010-022225 A1 | 25 February 2010 |
| | | WO 2010-022225 A9 | 28 April 2011 |
| | | WO 2010-093395 A1 | 19 August 2010 |
| | | WO 2010-117984 A1 | 14 October 2010 |
| | | WO 2011-025904 A1 | 03 March 2011 |
| | | WO 2012-024223 A2 | 23 February 2012 |
| | | WO 2012-024223 A3 | 05 July 2012 |
| | | WO 2012-061548 A1 | 10 May 2012 |
| | | WO 2012-075361 A2 | 07 June 2012 |
| | | WO 2012-075361 A3 | 26 July 2012 |
| | | WO 2012-082618 A2 | 21 June 2012 |
| | | WO 2012-082618 A3 | 07 September 2012 |
| | | WO 2012-162583 A1 | 29 November 2012 |
| | | WO 2013-052484 A1 | 11 April 2013 |
| | | WO 2013-082254 A1 | 06 June 2013 |
| | | WO 2013-112801 A1 | 01 August 2013 |
| | | WO 2014-092804 A1 | 19 June 2014 |
| | | WO 2014-165506 A1 | 09 October 2014 |
| | | WO 2015-012904 A2 | 29 January 2015 |
| | | WO 2015-012904 A3 | 07 May 2015 |
| | | WO 2015-047510 A1 | 02 April 2015 |
| | | WO 2016-003869 A1 | 07 January 2016 |
| | | WO 2016-057398 A1 | 14 April 2016 |
| | | WO 2016-077061 A2 | 19 May 2016 |
| | | WO 2016-077061 A3 | 18 August 2016 |
| | | WO 2016-118353 A1 | 28 July 2016 |
| | | WO 2016-210108 A1 | 29 December 2016 |
| | | WO 2017-004144 A1 | 05 January 2017 |
| | | WO 2017-034906 A1 | 02 March 2017 |
| | | WO 2017-034906 A8 | 12 October 2017 |
| | | WO 2017-180565 A1 | 19 October 2017 |
| | | WO 2018-031408 A1 | 15 February 2018 |
| | | WO 2018-102212 A1 | 07 June 2018 |
| | | WO 2018-156634 A1 | 30 August 2018 |
| | | WO 98-42378 A1 | 01 October 1998 |
| | | ZA 982438 B | 04 November 1998 |
| US 2018-0170944 A1 | 21 June 2018 | CN 106715443 A | 24 May 2017 |
| | | EP 3122754 A2 | 01 February 2017 |
| | | EP 3122754 A4 | 21 February 2018 |

Form PCT/ISA/210 (patent family annex) (July 2019)

**INTERNATIONAL SEARCH REPORT**
**Information on patent family members**

International application No.
**PCT/KR2021/009204**

| Patent document cited in search report | Publication date (day/month/year) | Patent family member(s) | Publication date (day/month/year) |
|---|---|---|---|
| | | US 10344037 B2 | 09 July 2019 |
| | | US 1084828 B2 | 10 August 2021 |
| | | US 2015-0276812 A1 | 01 October 2015 |
| | | US 2019-0367529 A1 | 05 December 2019 |
| | | US 9709603 B2 | 18 July 2017 |
| | | WO 2015-148415 A2 | 01 October 2015 |
| | | WO 2015-148415 A3 | 10 December 2015 |
| | | WO 2015-153176 A1 | 08 October 2015 |
| WO 2012-058666 A2 | 03 May 2012 | CA 2816418 A1 | 03 May 2012 |
| | | CA 2816418 C | 26 May 2020 |
| | | CN 103442565 A | 11 December 2013 |
| | | CN 103442565 B | 10 August 2016 |
| | | EP 2632264 A2 | 04 September 2013 |
| | | EP 2632264 A4 | 23 April 2014 |
| | | EP 2632264 B1 | 02 October 2019 |
| | | US 2014-0066470 A1 | 06 March 2014 |
| | | US 9422303 B2 | 23 August 2016 |
| | | WO 2012-058666 A3 | 10 January 2013 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Cancer Sci,* 2016, vol. 107, 1039-1046
- *N Engl J Med,* 2020, vol. 382, 610-621